# EUROPEAN PATENT SPECIFICATION

(11) **EP 3 221 300 B1**
(45) Date of publication and mention of the grant of the patent: **20.03.2019**
(21) Application number: 15801263.3
(22) Date of filing: 18.11.2015
(51) Int. Cl.: C07D 277/62, A61K 31/428, A61P 31/04

(54) **ANTIBACTERIAL BENZOTHIAZOLE DERIVATIVES**
ANTIBAKTERIELLE BENZOTHIAZOL-DERIVATE
DÉRIVÉS DE BENZOTHIAZOLE ANTIBACTÉRIENS

(30) Priority: 19.11.2014 WO PCT/EP2014/075009
(43) Date of publication of application: 27.09.2017
(73) Proprietor: Idorsia Pharmaceuticals Ltd, 4123 Allschwil (CH)
(72) Inventor: SCHMITT, Christine, CH-4123 Allschwil (CH); SURIVET, Jean-Philippe, CH-4123 Allschwil (CH); CHAPOUX, Gaëlle, CH-4123 Allschwil (CH); MIRRE, Azely, CH-4123 Allschwil (CH)
(74) Representative: Velker, Jörg
(86) International application number: PCT/IB2015/058919
(87) International publication number: WO 2016/079688

(56) References cited:
- WO-A1-2012/137099
- WO-A2-2011/045703
- LAURA A. MCALLISTER ET AL: "Heterocyclic methylsulfone hydroxamic acid LpxC inhibitors as Gram-negative antibacterial agents", BIOORGANIC & MEDICINAL CHEMISTRY LETTERS, vol. 22, no. 22, 1 November 2012 (2012-11-01), pages 6832-6838, XP055154150, ISSN: 0960-894X, DOI: 10.1016/j.bmcl.2012.09.058

## Description

The present invention concerns antibacterial benzothiazole derivatives, pharmaceutical compositions containing them and uses of these compounds in the manufacture of medicaments for the treatment of bacterial infections. These compounds are useful antimicrobial agents effective against a variety of human and veterinary pathogens, especially Gram-negative aerobic and anaerobic bacteria. The compounds of the present invention can optionally be employed in combination, either sequentially or simultaneously, with one or more therapeutic agents effective against bacterial infections.

The intensive use of antibiotics has exerted a selective evolutionary pressure on microorganisms to produce genetically based resistance mechanisms. Modern medicine and socio-economic behaviour exacerbate the problem of resistance development by creating slow growth situations for pathogenic microbes, e.g. in artificial joints, and by supporting long-term host reservoirs, e.g. in immune-compromised patients.

In hospital settings, an increasing number of strains of *Staphylococcus aureus, Streptococcus pneumoniae, Enterococcus spp., Enterobacteriaceae* such as *Klebsiella pneumoniae, Acinetobacter baumannii* and *Pseudomonas aeruginosa,* major sources of infections, are becoming multi-drug resistant and therefore difficult if not impossible to treat. This is particularly the case for Gram-negative organisms where the situation is getting worrisome since no novel agents have been approved for decades and the development pipeline looks empty.

Therefore, there is an important medical need for new antibacterial compounds addressing Gram-negative resistant bacteria, in particular third generation cephalosporins- and carbapenem- resistant *Klebsiella pneumoniae* and multi-drug-resistant *Pseudomonas aeruginosa* and *Acinetobacter baumannii.* One way to tackle the problem of cross resistance to established classes of antibiotics is to inhibit a new essential target. In this respect, LpxC, which is an enzyme in the biosynthesis of lipopolysaccharides (a major constituent of the outer membrane of Gram-negative bacteria), has received some attention and several patent applications relating to LpxC inhibitors have been published recently.

For example, WO 2011/045703 describes antibacterial compounds of formula (A1) 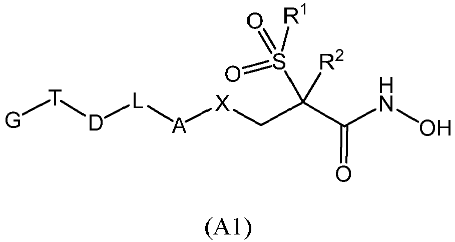 wherein R¹ is (C₁-C₃)alkyl; R² is H or (C₁-C₃)alkyl; X is CH₂, O, NH, S or SO₂; A is an optionally substituted phenyl or a 6-membered heteroaryl group; L is absent or is S, SH, OH, -(CH₂)ₚ-O-(CH₂)ₙ-, -(CH₂)ₚ-O-(CH₂)_{z}-O-(CH₂)ₙ-, -S-(CH₂)_{z}- or -(CH₂)_{z}-S-; D is absent or is an optionally substituted group containing a carbocyclic or heterocyclic component with optionally a (C₁-C₃)alkyl chain appended; T is absent or is -(CH₂)_{z}-, -(CH₂)_{z}-O- or -O-(CH₂)ₚ-C(O)-(CH₂)ₙ-; G is absent or is an optionally substituted carbocyclic or heterocyclic group; and n and p are integers each ranging from 0 to 3 and z is an integer ranging from 1 to 3.

WO 2011/073845 and WO 2012/120397 describe antibacterial compounds with a structural formula similar to formula (A1), whereby the group corresponding to the group A of formula (A1) however respectively represents a pyridin-2-one or a fluoropyridin-2-one residue.

WO 2012/137094 describes antibacterial compounds of formulae (A2) and (A3) 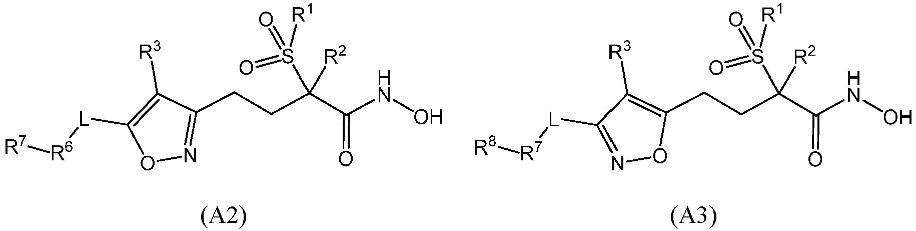 wherein R¹ is (C₁-C₃)alkyl; R² is H or (C₁-C₃)alkyl; R³ is H, (C₁-C₃)alkoxy, (C₁-C₃)alkyl, cyano, (C₁-C₃)haloalkoxy, (C₁-C₃)haloalkyl, halogen or hydroxy; L is a bond, -(CH₂)ₙ-, -(CH₂)ₙO(CH₂)ₚ-, -(CH₂)ₙNR⁴(CH₂)ₚ-, -(CH₂)ₙSO₂NR⁴(CH₂)ₚ-, -(CH₂)ₙCONR⁴(CH₂)ₚ- or -(CH₂)ₙNR⁴CO(CH₂)ₚ-; R⁴ and R⁵ are independently H, (C₁-C₆)alkyl, (C₁-C₆)alkylcarbonyl, (C₃-C₈)cycloalkyl, (C₃-C₈)cycloalkyl(C₁-C₆)alkyl or formyl; n is 0, 1, 2, 3 or 4; p is 0, 1, 2, 3 or 4; R⁶ is (C₁-C₆)alkoxy(C₁-C₆)alkyl, (C₁-C₆)alkoxycarbonyl, (C₁-C₆)alkyl-NR⁴-(C₁-C₆)alkyl, (C₁-C₆)alkylthio(C₁-C₆)alkyl, (C₁-C₆)alkylthiocarbonyl, (C₆-C₁₂)aryl, (C₆-C₁₂)aryloxy, (C₆-C₁₂)arylthio, (C₆₋C₁₂)aryl-NR⁴-, (C₃-C₈)cycloalkyl, (C₃-C₈)cycloalkyloxy, (C₃-C₈)cycloalkylthio, (C₅-C₈)cycloalkyl-NR⁴-, (C₅-C₁₂)heteroaryl, (C₅-C₁₂)heteroaryloxy, (C₅-C₁₂)heteroarylthio, (C₅-C₁₂)heteroaryl-NR⁴-, (C₃-C₁₃)heterocyclyl, (C₃-C₁₃)heterocyclyloxy, (C₃-C₁₃)heterocyclylthio, (C₃-C₁₃)heterocycle-NR⁴-, hydroxy(C₁-C₁₀)alkyl, mercapto(C₁-C₆)alkyl, (NR⁴R⁵)alkyl, or (NR⁴R⁵)carbonyl; and R⁷ is absent or is (C₆-C₁₂)aryl, (C₆-C₁₂)aryl(C₁-C₆)alkyl, (C₃-C₈)cycloalkyl, (C₃-C₈)cycloalkyl(C₁-C₆)alkyl, (C₅-C₁₂)heteroaryl, (C₅-C₁₂)heteroaryl(C₁-C₆)alkyl, (C₃-C₁₃)heterocyclyl or (C₃-C₁₃)heterocyclyl(C₁-C₆)alkyl.

WO 2012/137099 describes antibacterial compounds of formula (A4) 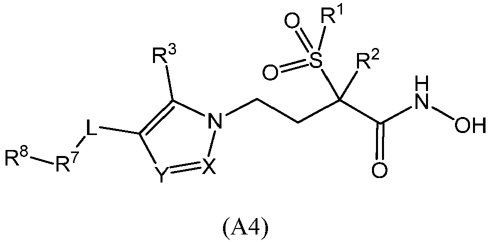 wherein R¹ is (C₁-C₃)alkyl; R² is H or (C₁-C₃)alkyl; R³ is H or (C₁-C₃)alkyl; X is N or CR⁴; Y is N or CR⁴; R⁴ is H or (C₁-C₃)alkyl; L is a bond, (C₂-C₆)alkenylene, (C₁-C₆)alkylene, (C₂-C₆)alkynylene, -(CH₂)ₙO(CH₂)ₚ-, -(CH₂)ₙS(CH₂)ₚ-, -(CH₂)ₙNR⁵(CH₂)ₚ-, -(CH₂)ₙSO₂NR⁵(CH₂)ₚ-, -(CH₂)ₙNR⁵SO₂(CH₂)ₚ-, -(CH₂)ₙCONR⁵(CH₂)ₚ- or -(CH₂)ₙNR⁵CO(CH₂)ₚ-; R⁵ and R⁶ are independently H, (C₁-C₆)alkyl, (C₁-C₆)alkylcarbonyl, (C₃-C₈)cycloalkyl, (C₃-C₈)cycloalkyl(C₁-C₆)alkyl or formyl; n is 0, 1, 2, 3 or 4; p is 0, 1, 2, 3 or 4; R⁷ is (C₂-C₆)alkenyl, (C₁-C₆)alkoxy, (C₁-C₆)alkoxy(C₁-C₆)alkyl, (C₁-C₆)alkoxycarbonyl, (C₁-C₆)alkyl, (C₁-C₆)alkylcarbonyl, (C₁-C₆)alkyl-NR⁵-(C₁-C₆)alkyl, (C₁-C₆)alkylthio, (C₁-C₆)alkylthio(C₁-C₆)alkyl, (C₁-C₆)alkylthiocarbonyl, (C₂-C₆)alkynyl, (C₆-C₁₂)aryl, (C₆-C₁₂)aryloxy, (C₆-C₁₂)arylthio, (C₆₋C₁₂)aryl-NR⁵-, cyano, cyano(C₁-C₆)alkyl, (C₅-C₈)cycloalkenyl, (C₃-C₈)cycloalkyl, (C₃-C₈)cycloalkyloxy, (C₃-C₈)cycloalkylthio, (C₅-C₈)cycloalkyl-NR⁵- (C₅-C₁₂)heteroaryl, (C₅-C₁₂)heteroaryloxy, (C₅-C₁₂)heteroarylthio, (C₅-C₁₂)heteroaryl-NR⁵-, (C₃-C₁₃)heterocyclyl, (C₃-C₁₃)heterocyclyloxy, (C₃-C₁₃)heterocyclylthio, (C₃-C₁₃)heterocyclyl-NR⁵-, hydroxy(C₁-C₁₀)alkyl, mercapto(C₁-C₆)alkyl, (NR⁵R⁶)alkyl, or (NR⁵R⁶)carbonyl; and R⁸ is absent or is (C₆-C₁₂)aryl, (C₆-C₁₂)aryl(C₁-C₆)alkyl, (C₃-C₈)cycloalkyl, (C₃-C₈)cycloalkyl(C₁-C₆)alkyl, (C₅-C₁₂)heteroaryl, (C₅-C₁₂)heteroaryl(C₁-C₆)alkyl, (C₃-C₁₃)heterocyclyl or (C₃-C₁₃)heterocyclyl(C₁-C₆)alkyl.

WO 2013/170165 describes notably antibacterial compounds of formula (A5) 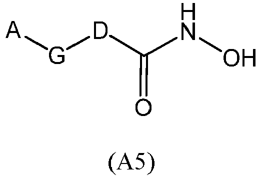 wherein A is a substituted alkyl group, wherein at least one substituent is hydroxy, or A is a substituted cycloalkyl group, wherein at least one substituent is hydroxy or hydroxyalkyl; G is a group comprising at least one carbon-carbon double or triple bond and/or a phenyl ring; D represents a group selected from

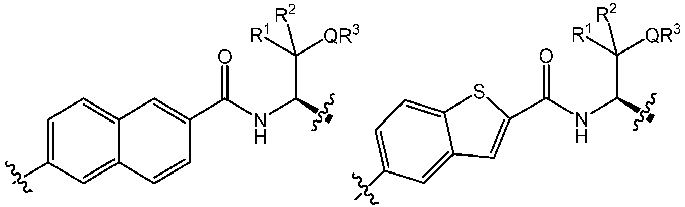

Q is O or NR, wherein R is H or an unsubstituted (C₁-C₃)alkyl; R¹ and R² independently are selected from the group consisting of H and substituted or unsubstituted (C₁-C₃)alkyl, or R¹ and R², together with the carbon atom to which they are attached, form an unsubstituted (C₃-C₄)cycloalkyl group or an unsubstituted 4-6 membered heterocyclic group; and R³ is selected from the group consisting of hydrogen, substituted or unsubstituted (C₁-C₃)alkyl, substituted or unsubstituted cycloalkyl, substituted or unsubstituted cycloalkylalkyl, substituted or unsubstituted aryl, substituted or unsubstituted arylalkyl, substituted or unsubstituted heterocyclyl, substituted or unsubstituted heterocyclylalkyl, substituted or unsubstituted heteroaryl, and substituted or unsubstituted heteroarylalkyl.

In WO 2015/036964, we have reported antibacterial 2*H*-indazole derivatives of general formula (A6) 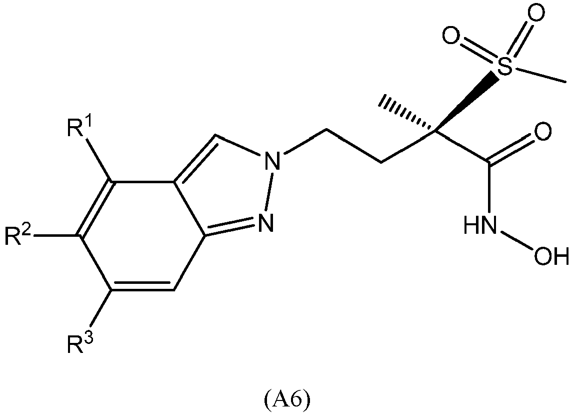 wherein
R¹ is H or halogen; R² is (C₃-C₄)alkynyloxy or the group M; R³ is H or halogen; M is one of the groups M^{A} and M^{B} represented below 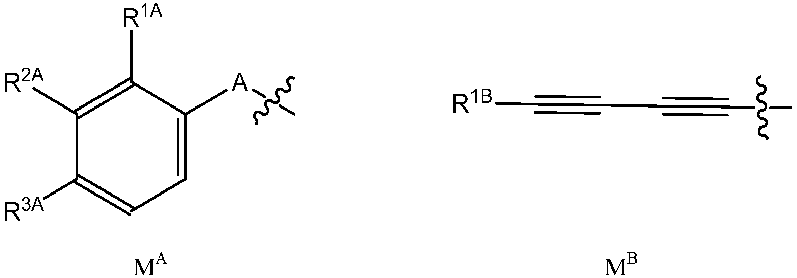 wherein A is a bond, CH₂CH₂, CH=CH or C≡C; R^{1A} is H or halogen; R^{2A} is H, alkoxy or halogen; R^{3A} is H, alkoxy, hydroxyalkoxy, thioalkoxy, trifluoromethoxy, amino, dialkylamino, hydroxyalkyl, 1-hydroxymethyl-cycloprop-1-yl, *trans*-2-hydroxymethyl-cycloprop-1-yl, 1,2-dihydroxyethyl, 3-hydroxyoxetan-3-yl, 3-(hydroxyalkyl)oxetan-3-yl, 3-aminooxetan-3-yl, 3-(dialkylamino)oxetan-3-yl, 3-hydroxythietan-3-yl, morpholin-4-ylalkoxy, morpholin-4-ylalkyl, oxazol-2-yl or [1,2,3]triazol-2-yl; and R^{1B} is 3-hydroxyoxetan-3-yl, 3-hydroxythietan-3-yl, hydroxyalkyl, aminoalkyl, *trans*-2-hydroxymethyl-cycloprop-1-yl or 4-hydroxytetrahydro-2*H*-pyran-4-yl.

In WO 2015/091741, we have reported antibacterial 1*H*-indazole derivatives of general formula (A7) 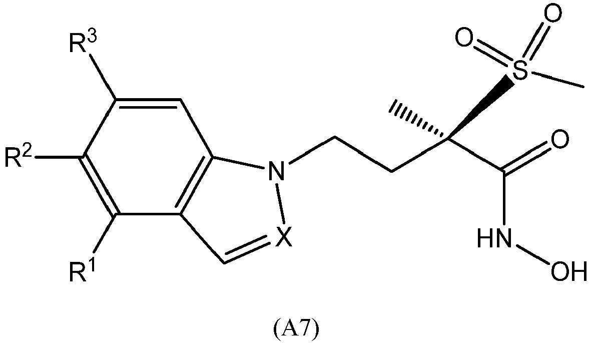 wherein X is N or CH; R¹ is H or halogen; R² is (C₃-C₄)alkynyloxy or the group M; R³ is H or halogen; M is one of the groups M^{A} and M^{B} represented below 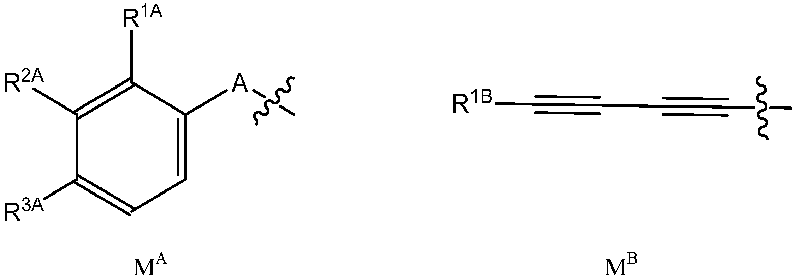 wherein A is a bond, CH₂CH₂, CH=CH or C≡C; R^{1A} is H or halogen; R^{2A} is H, (C₁-C₃)alkoxy or halogen; R^{3A} is H, (C₁-C₃)alkoxy, hydroxy(C₁-C₄)alkoxy, (C₁-C₃)thioalkoxy, trifluoromethoxy, amino, hydroxy(C₁-C₄)alkyl, 2-hydroxyacetamido, 1-hydroxymethyl-cycloprop-1-yl, *trans*-2-hydroxymethyl-cycloprop-1-yl, 1,2-dihydroxyethyl, 3 -hydroxyoxetan-3 -yl, 3-(hydroxy(C₁-C₃)alkyl)oxetan-3-yl, 3-aminooxetan-3-yl, 3-hydroxythietan-3-yl, morpholin-4-yl(C₂-C₃)alkoxy, morpholin-4-yl-(C₁-C₂)alkyl, oxazol-2-yl or [1,2,3]triazol-2-yl; and R^{1B} is 3-hydroxyoxetan-3-yl, 3-hydroxythietan-3-yl, hydroxy(C₁-C₃)alkyl, amino(C₁-C₃)alkyl, 1-hydroxymethyl-cycloprop-1-yl or *trans*-2-hydroxymethyl-cycloprop-1-yl.

In a further previous, yet unpublished patent application, we have reported antibacterial 1,2-dihydro-3*H*-pyrrolo[1,2-*c*]imidazol-3-one derivatives of general formula (A8) 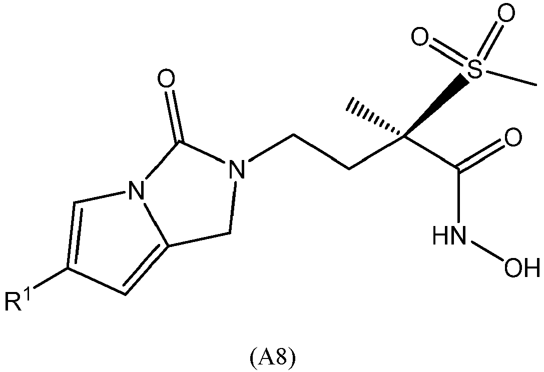 wherein R¹ is the group M; M is one of the groups M^{A} and M^{B} represented below 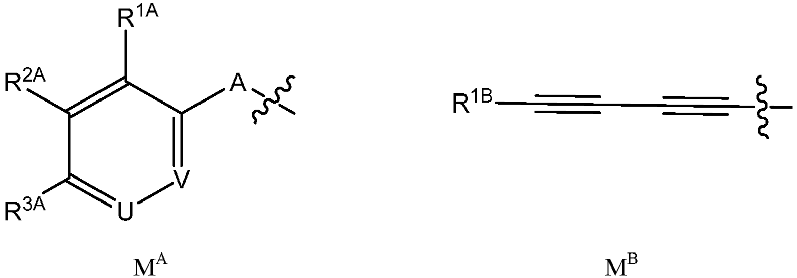 wherein A is a bond, CH=CH or C=C; U is N or CH; V is N or CH; R^{1A} is H or halogen; R^{2A} is H, (C₁-C₃)alkoxy or halogen; R^{3A} is H, (C₁-C₃)alkoxy, hydroxy(C₂-C₄)alkoxy, (C₁-C₃)alkoxy(C₁-C₃)alkoxy, (C₁-C₃)thioalkoxy, trifluoromethoxy, amino, hydroxy(C₁-C₄)alkyl, (C₁-C₃)alkoxy(C₁-C₄)alkyl, 3-hydroxy-3-methylbut-1-yn-1-yl, 2-hydroxyacetamido, (carbamoyloxy)methyl, 1-hydroxymethyl-cycloprop-1-yl, 1-aminomethyl-cycloprop-1-yl, 1-(carbamoyloxy)methyl-cycloprop-1-yl, 1-(morpholin-4-yl)methylcycloprop-1-yl, *trans*-2-hydroxymethyl-cycloprop-1-yl, 1,2-dihydroxyethyl, 3 -hydroxyoxetan-3 -yl, 3-(hydroxy(C₁-C₃)alkyl)oxetan-3-yl, 3-aminooxetan-3-yl, 3-hydroxythietan-3-yl, morpholin-4-yl(C₂-C₃)alkoxy, [4-*N*-(C₁-C₃)alkylpiperazin-1-yl](C₁-C₃)alkyl, morpholin-4-yl-(C₁-C₂)alkyl, [1,2,3]triazol-2-yl or 3-[hydroxy(C₂-C₃)alkyl]-2-oxo-imidazolidin-1-yl; and R^{1B} is 3-hydroxyoxetan-3-yl, 3-hydroxythietan-3-yl, 3-(hydroxy(C₁-C₃)alkyl)oxetan-3-yl, hydroxy(C₁-C₃)alkyl, 1,2-dihydroxyethyl, amino(C₁-C₃)alkyl, 1-hydroxymethyl-cycloprop-1-yl, *trans*-2-hydroxymethyl-cycloprop-1-yl, *trans*-(*cis*-3,4-dihydroxy)-cyclopent-1-yl or 3-hydroxymethylbicyclo[1,1,1]pentan-1-yl.

In a further previous, yet unpublished patent application, we have reported antibacterial quinazoline-4(3*H*)-one derivatives of general formula (A9) 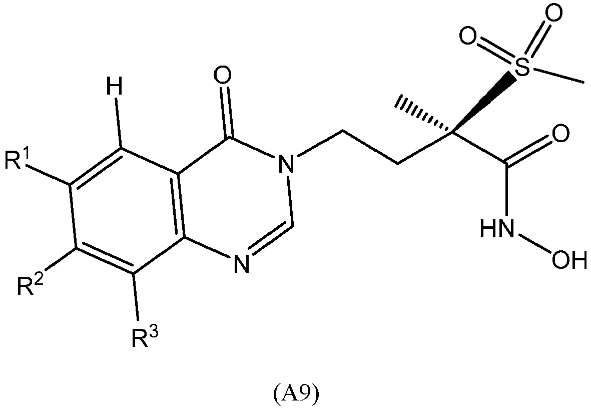 wherein R¹ is H or halogen; R² is the group M; R³ is H or halogen; M is one of the groups M^{A} and M^{B} represented below 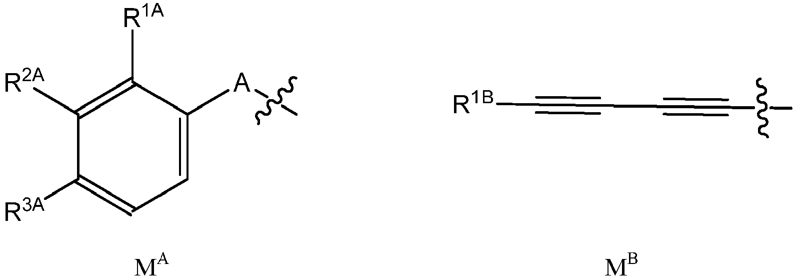 wherein A represents a bond or C≡C; R^{1A} is H or halogen; R^{2A} is H, (C₁-C₃)alkoxy or halogen; R^{3A} is H, (C₁-C₃)alkoxy, hydroxy(C₂-C₄)alkoxy, hydroxy(C₁-C₄)alkyl, 1,2-dihydroxyethyl, di(C₁-C₃)alkylamino, 1-hydroxymethyl-cycloprop-1-yl, 1-((dimethylglycyl)oxy)methyl-cycloprop-1-yl, 3-hydroxyoxetan-3-yl, morpholin-4-yl-(C₁-C₂)alkyl or morpholin-4-yl(C₂-C₃)alkoxy; and R^{1B} is hydroxy(C₁-C₃)alkyl, amino(C₁-C₃)alkyl, 1,2-dihydroxyprop-3-yl, 1-amino-cycloprop-1-yl, 1-hydroxymethyl-cycloprop-1-yl, *trans*-2-hydroxymethyl-cycloprop-1-yl, *trans*-2-aminomethyl-cycloprop-1-yl, *trans*-2-hydroxymethyl-1-methyl-cycloprop-1-yl, *trans*-2-hydroxymethyl-2-methyl-cycloprop-1-yl, 1-(1,2-dihydroxyethyl)-cycloprop-1-yl, *trans*-2-(1,2-dihydroxyethyl)-cycloprop-1-yl, 3-hydroxyoxetan-3-yl, 3-(hydroxy(C₁-C₃)alkyl)oxetan-3-yl, 3 -hydroxythietan-3 -yl, *trans*-(*cis*-3,4-dihydroxy)-cyclopent-1-yl, 3-(2-aminoacetamido)cyclopentyl or 3-hydroxymethylbicyclo[1,1,1]pentan-1-yl.

In WO 2011/073845, WO 2012/120397 or WO 2013/170165, further LpxC inhibitors are disclosed, among others the compounds of general formula (A10) 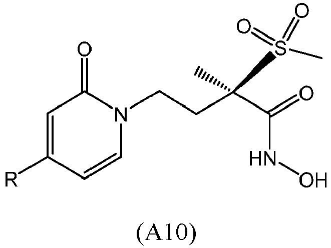 wherein R can notably be phenylethynyl or styryl.

Besides, in Montgomery et al., J. Med. Chem. (2012), 55(4), 1662-1670, yet further LpxC inhibitors are disclosed, among others the compound of formula (A11)

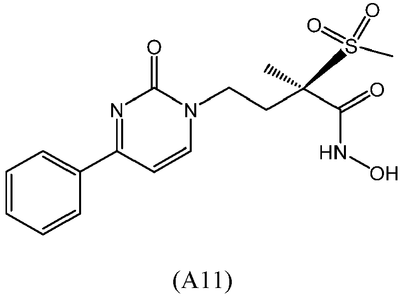

The instant invention provides new antibacterial benzothiazole derivatives, namely the compounds of formula I described herein.

Various embodiments of the invention are presented hereafter:
1) In a first embodiment, the invention relates to compounds of formula I 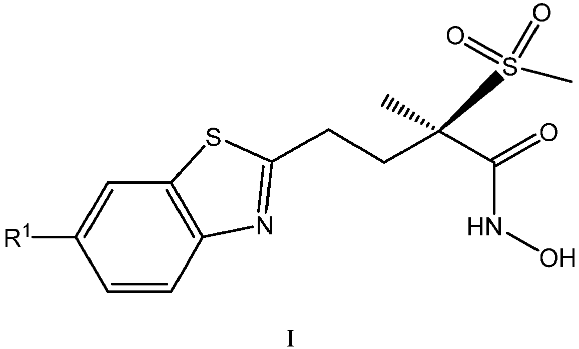 wherein
   R¹ is the group M, whereby M is one of the groups M^{A} and M^{B} represented below 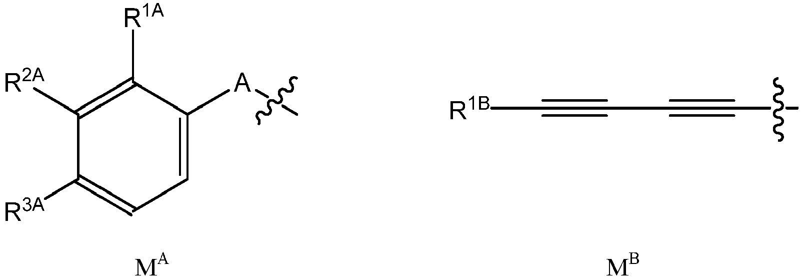
   wherein A represents a bond or C=C;
   R^{1A} is H or halogen;
   R^{2A} is H or halogen, preferably H; and
   R^{3A} is H, (C₁-C₃)alkoxy, hydroxy(C₂-C₄)alkoxy, hydroxy(C₁-C₄)alkyl, dihydroxy(C₂-C₄)alkyl, 2-hydroxyacetamido, 1-hydroxymethyl-cycloprop-1-yl, *trans*-2-hydroxymethyl-cycloprop-1-yl, 3-hydroxyoxetan-3-yl, 3-(hydroxy(C₁-C₃)alkyl)oxetan-3-yl, 3-aminooxetan-3-yl or 1-aminocycloprop-1-yl;
   and wherein R^{1B} is hydroxy(C₁-C₄)alkyl (such as especially hydroxymethyl or 1-hydroxy-1-methyl-ethyl), dihydroxy(C₂-C₄)alkyl (such as especially (*S*)-1,2-dihydroxy-ethyl), amino(C₁-C₄)alkyl (such as especially 1-amino-1-methyl-ethyl), di(C₁-C₄)alkylamino(C₁-C₃)alkyl (such as especially dimethylaminomethyl), 1-amino-cycloprop-1-yl, 1-hydroxymethyl-cycloprop-1-yl, *trans*-2-hydroxymethyl-cycloprop-1-yl, *trans*-2-aminomethyl-cycloprop-1-yl, *trans*-2-hydroxymethyl-1-methyl-cycloprop-1-yl, *trans*-2-hydroxymethyl-2-methyl-cycloprop-1-yl, *cis*-1-fluoro-2-(hydroxymethyl)cycloprop-1-yl, *cis*-2-fluoro-2-(hydroxymethyl)cycloprop-1-yl, 2-(1,2-dihydroxyethyl)-cycloprop-1-yl, 1-(hydroxymethyl)-cyclobutan-1-yl, *cis*-3-(hydroxymethyl)-1-hydroxy-cyclobutan-1-yl, 3-hydroxyoxetan-3-yl, 3-hydroxyoxetan-3-yl-(C₁-C₃)alkyl (such as especially 3-hydroxyoxetan-3-yl-methyl), 3-aminooxetan-3-yl, 3-hydroxymethyl-oxetan-3-yl, *trans*-(*cis*-3,4-dihydroxy)-cyclopent-1-yl, 3-hydroxymethylbicyclo[1,1,1]pentan-1-yl, 4-hydroxytetrahydro-2*H*-pyran-4-yl, (*3R*,*6S*)-3-aminotetrahydro-2*H*-pyran-6-yl, piperidin-4-yl, 1-(2-hydroxyacetyl)piperidin-4-yl, 3-hydroxythietan-3-yl, 1-(2-hydroxyacetyl)azetidin-3-yl or 1-glycylazetidin-3-yl;
   and to salts (in particular pharmaceutically acceptable salts) of such compounds of formula I.

The following paragraphs provide definitions of the various chemical moieties for the compounds according to the invention and are intended to apply uniformly throughout the specification and claims, unless an otherwise expressly set out definition provides a broader or narrower definition:
❖ The term "halogen" refers to fluorine, chlorine, bromine or iodine, and preferably to fluorine or chlorine, and most preferably to fluorine.
❖ The term "alkyl", used alone or in combination, refers to a straight or branched chain alkyl group containing from one to four carbon atoms. The term "(Cₓ-C_{y})alkyl" (x and y each being an integer) refers to a straight or branched chain alkyl group containing x to y carbon atoms. For example, a (C₁-C₃)alkyl group contains from one to three carbon atoms.
❖ The term "hydroxyalkyl", used alone or in combination, refers to an alkyl group as defined before wherein one hydrogen atom has been replaced by a hydroxy group. The term "hydroxy(Cₓ-C_{y})alkyl" (x and y each being an integer) refers to a hydroxyalkyl group as defined which contains x to y carbon atoms. For example, a hydroxy(C₁-C₄)alkyl group is a hydroxyalkyl group as defined before which contains from one to four carbon atoms.
❖ The term "dihydroxy(C₂-C₄)alkyl", used alone or in combination, refers to an alkyl group containing from two to four carbon atoms wherein two hydrogen atoms on two different carbon atoms have each been replaced by a hydroxy group.
❖ The term "aminoalkyl", used alone or in combination, refers to an alkyl group as defined before wherein one hydrogen atom has been replaced by an amino group. The term "amino(Cₓ-C_{y})alkyl" (x and y each being an integer) refers to an aminoalkyl group as defined which contains x to y carbon atoms. For example, an amino(C₁-C₄)alkyl group is an aminoalkyl group as defined before which contains from one to four carbon atoms.
❖ The term "dialkylamino", used alone or in combination, refers to an amino group wherein each hydrogen atom has been replaced by an alkyl group as defined before, whereby the alkyl groups may be the same or different. The term "di(Cₓ-C_{y})alkylamino" (x and y each being an integer) refers to a dialkylamino group as defined before wherein each alkyl group independently contains x to y carbon atoms. For example, a di(C₁-C₄)alkylamino group is a dialkylamino group as defined before wherein each alkyl group independently contains from one to four carbon atoms.
❖ The term "alkoxy", used alone or in combination, refers to a straight or branched chain alkoxy group containing from one to four carbon atoms. The term "(Cₓ-C_{y})alkoxy" (x and y each being an integer) refers to an alkoxy group as defined before containing x to y carbon atoms. For example, a (C₁-C₃)alkoxy group contains from one to three carbon atoms.
❖ The term "hydroxyalkoxy", used alone or in combination, refers to a straight or branched chain alkoxy group containing from two to four carbon atoms wherein one of the carbon atoms bears a hydroxy group. The term "hydroxy(Cₓ-C_{y})alkoxy" (x and y each being an integer) refers to a hydroxyalkoxy group as defined before containing x to y carbon atoms. For example, a hydroxy(C₂-C₄)alkoxy group contains from two to four carbon atoms.
❖ The term "3-(hydroxy(C₁-C₃)alkyl)oxetan-3-yl" refers to an oxetan-3-yl group wherein the hydrogen on the carbon at position 3 of the oxetane ring has been replaced by a hydroxy(C₁-C₃)alkyl group as defined before.
❖ The term "quinolone-resistant", when used in this text, refers to a bacterial strain against which ciprofloxacin has a Minimal Inhibitory Concentration of at least 16 mg/L (said Minimal Inhibitory Concentration being measured with the standard method described in *"*Methods for Dilution Antimicrobial Susceptibility Tests for Bacteria that Grow Aerobically", Approved standard, 7th ed., Clinical and Laboratory Standards Institute (CLSI) Document M7-A7, Wayne, PA, USA (2006)).
❖ The term "carbapenem-resistant", when used in this text, refers to a bacterial strain against which imipenem has a Minimal Inhibitory Concentration of at least 16 mg/L (said Minimal Inhibitory Concentration being measured with the standard method described in "Methods for Dilution Antimicrobial Susceptibility Tests for Bacteria that Grow Aerobically", Approved standard, 7th ed., Clinical and Laboratory Standards Institute (CLSI) Document M7-A7, Wayne, PA, USA (2006)).
❖ The term "multi-drug resistant", when used in this text, refers to a bacterial strain against which at least three antibiotic compounds selected from three distinct antibiotic categories have Minimal Inhibitory Concentrations (MICs) over their respective clinical breakpoints, whereby said three distinct antibiotic categories are chosen among penicillins, combinations of penicillins with beta-lactamase inhibitors, cephalosporins, carbapenems, monobactams, fluoro-quinolones, aminoglycosides, phosphonic acids, tetracyclins and polymixins. Clinical breakpoints are defined according to the latest available list published by Clinical and Laboratory Standards Institute (Wayne, PA, USA). Accordingly, clinical breakpoints are the levels of MIC at which, at a given time, a bacterium is deemed either susceptible or resistant to treatment by the corresponding antibiotic or antibiotic combination.

Any reference hereinbefore or hereinafter to a compound of formula I is to be understood as referring also to salts, especially pharmaceutically acceptable salts, of a compound of formula I, as appropriate and expedient.

The term "pharmaceutically acceptable salts" refers to salts that retain the desired biological activity of the subject compound and exhibit minimal undesired toxicological effects. Such salts include inorganic or organic acid and/or base addition salts depending on the presence of basic and/or acidic groups in the subject compound. For reference see for example 'Handbook of Pharmaceutical Salts. Properties, Selection and Use.', P. Heinrich Stahl, Camille G. Wermuth (Eds.), Wiley-VCH (2008) and 'Pharmaceutical Salts and Co-crystals', Johan Wouters and Luc Quéré (Eds.), RSC Publishing (2012).

For the avoidance of any doubt, if in this text a radical contains the designation "*cis*" and/or *"trans"* said designations refer to the configuration of the radical when attached to the rest of the molecule. For example, the R^{1B} radical *trans*-2-hydroxymethyl-1-methyl-cycloprop-1-yl refers to the following relative configuration: 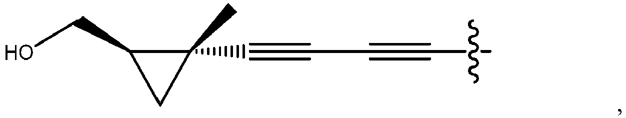 and the R^{1B} radical *cis*-3-(hydroxymethyl)-1-hydroxy-cyclobutan-1-yl refers to the following relative configuration:

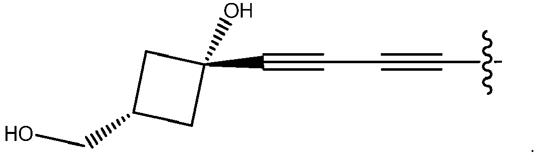

In this text, a bond interrupted by a wavy line shows a point of attachment of the radical drawn to the rest of the molecule. For example, the radical drawn below 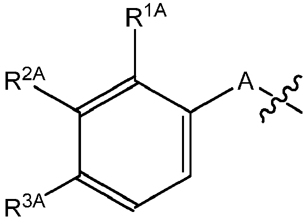 wherein A represents a bond, and each of R^{1A}, R^{2A} and R^{3A} represents H is the phenyl group.

Besides, the term "room temperature" as used herein refers to a temperature of 25°C. Unless used regarding temperatures, the term "about" placed before a numerical value "X" refers in the current application to an interval extending from X minus 10% of X to X plus 10% of X, and preferably to an interval extending from X minus 5% of X to X plus 5% of X. In the particular case of temperatures, the term "about" placed before a temperature "Y" refers in the current application to an interval extending from the temperature Y minus 10°C to Y plus 10°C, and preferably to an interval extending from Y minus 5°C to Y plus 5°C.
2) Another embodiment of the invention relates to compounds of formula I according to embodiment 1), wherein R¹ is the group M^{A}.
3) Another embodiment of the invention relates to compounds of formula I according to embodiment 2), wherein A represents a bond or C=C;
   R^{1A} is H or halogen;
   R^{2A} is H; and
   R^{3A} is (C₁-C₃)alkoxy, hydroxy(C₁-C₄)alkyl, 1-hydroxymethyl-cycloprop-1-yl, 3-hydroxyoxetan-3-yl, or 3-aminooxetan-3-yl.
4) Another embodiment of the invention relates to compounds of formula I according to embodiment 2), wherein A represents a bond.
5) Another embodiment of the invention relates to compounds of formula I according to embodiment 4), wherein
   R^{1A} is H or halogen (such as especially fluoro);
   R^{2A} is H; and
   R^{3A} is (C₁-C₃)alkoxy (such as especially methoxy).
6) Another embodiment of the invention relates to compounds of formula I according to embodiment 2), wherein A represents C≡C.
7) Another embodiment of the invention relates to compounds of formula I according to embodiment 6), wherein
   R^{1A} and R^{2A} are both H; and
   R^{3A} is hydroxy(C₁-C₄)alkyl (such as especially hydroxymethyl), 1-hydroxymethyl-cycloprop-1-yl, 3-hydroxyoxetan-3-yl, or 3-aminooxetan-3-yl.
8) Another embodiment of the invention relates to compounds of formula I according to embodiment 1), wherein R¹ is the group M^{B}.
9) Another embodiment of the invention relates to compounds of formula I according to embodiment 8), wherein R^{1B} is hydroxy(C₁-C₄)alkyl (such as especially hydroxymethyl or 1-hydroxy-1-methyl-ethyl), dihydroxy(C₂-C₄)alkyl (such as especially (*S*)-1,2-dihydroxyethyl), amino(C₁-C₄)alkyl (such as especially 1-amino-1-methyl-ethyl), di(C₁-C₄)alkylamino(C₁-C₃)alkyl (such as especially dimethylaminomethyl), 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 1-(hydroxymethyl)-cyclobutan-1-yl, *cis*-3 -(hydroxymethyl)-1-hydroxy-cyclobutan-1-yl, 3-hydroxyoxetan-3-yl, 3-hydroxyoxetan-3-yl-(C₁-C₃)alkyl (such as especially 3-hydroxyoxetan-3-yl-methyl), 3-aminooxetan-3-yl, 3-hydroxymethyl-oxetan-3-yl, *trans*-(*cis*-3,4-dihydroxy)-cyclopent-1-yl, 4-hydroxytetrahydro-2*H*-pyran-4-yl, (3*R*,6*S*)-3-aminotetrahydro-2*H*-pyran-6-yl, piperidin-4-yl, or 1-(2-hydroxyacetyl)piperidin-4-yl.
10) Another embodiment of the invention relates to compounds of formula I according to embodiment 9), wherein R^{1B} is hydroxy(C₁-C₄)alkyl (such as especially hydroxymethyl or 1-hydroxy-1-methyl-ethyl), dihydroxy(C₂-C₄)alkyl (such as especially (*S*)-1,2-dihydroxyethyl), amino(C₁-C₄)alkyl (such as especially 1-amino-1-methyl-ethyl), 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 3-hydroxyoxetan-3-yl, 3-hydroxyoxetan-3-yl-(C₁-C₃)alkyl (such as especially 3-hydroxyoxetan-3-yl-methyl), 3-hydroxymethyl-oxetan-3-yl, or *trans*-(*cis*-3,4-dihydroxy)-cyclopent-1-yl.
11) Another embodiment of the invention relates to compounds of formula I according to embodiment 1), wherein R¹ is the group M^{A}, A represents a bond,
   R^{1A} is halogen (such as especially fluoro),
   R^{2A} is H, and
   R^{3A} is (C₁-C₃)alkoxy (such as especially methoxy);
   or R¹ is the group M^{A}, A represents C≡C,
   R^{1A} and R^{2A} are both H, and
   R^{3A} is 1-hydroxymethyl-cycloprop-1-yl;
   or R¹ is the group M^{B} and R^{1B} is di(C₁-C₄)alkylamino(C₁-C₃)alkyl (such as especially dimethylaminomethyl), 1-(hydroxymethyl)-cyclobutan-1-yl, *cis*-3 -(hydroxymethyl)-1-hydroxy-cyclobutan-1-yl, 3-aminooxetan-3-yl, 4-hydroxytetrahydro-2*H*-pyran-4-yl, (3*R*,6*S*)-3-aminotetrahydro-2*H*-pyran-6-yl, piperidin-4-yl, or 1-(2-hydroxyacetyl)piperidin-4-yl.
12) Another embodiment of the invention relates to compounds of formula I according to embodiment 1), wherein
   A represents a bond or C=C;
   R^{1A} is H or halogen;
   R^{2A} is H; and
   R^{3A} is (C₁-C₃)alkoxy, hydroxy(C₁-C₄)alkyl, 1-hydroxymethyl-cycloprop-1-yl, 3-hydroxyoxetan-3-yl, or 3-aminooxetan-3-yl;
   and wherein R^{1B} is hydroxy(C₁-C₄)alkyl (such as especially hydroxymethyl or 1-hydroxy-1-methyl-ethyl), dihydroxy(C₂-C₄)alkyl (such as especially (*S*)-1,2-dihydroxy-ethyl), amino(C₁-C₄)alkyl (such as especially 1-amino-1-methyl-ethyl), di(C₁-C₄)alkylamino(C₁-C₃)alkyl (such as especially dimethylaminomethyl), 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 1-(hydroxymethyl)-cyclobutan-1-yl, *cis*-3 -(hydroxymethyl)-1-hydroxy-cyclobutan-1-yl, 3-hydroxyoxetan-3-yl, 3-hydroxyoxetan-3-yl-(C₁-C₃)alkyl (such as especially 3-hydroxyoxetan-3-yl-methyl), 3-aminooxetan-3-yl, 3-hydroxymethyl-oxetan-3-yl, *trans*-(*cis*-3,4-dihydroxy)-cyclopent-1-yl, 4-hydroxytetrahydro-2*H*-pyran-4-yl, (*3R*,*6S*)-3-aminotetrahydro-2*H*-pyran-6-yl, piperidin-4-yl, or 1-(2-hydroxyacetyl)piperidin-4-yl.
13) Another embodiment of this invention relates to compounds of formula I as defined in one of embodiments 1) to 12) as well as to isotopically labelled, especially ²H (deuterium) labelled compounds of formula I as defined in one of embodiments 1) to 12), which compounds are identical to the compounds of formula I as defined in one of embodiments 1) to 12) except that one or more atoms has or have each been replaced by an atom having the same atomic number but an atomic mass different from the atomic mass usually found in nature. Isotopically labelled, especially ²H (deuterium) labelled compounds of formula I and salts (in particular pharmaceutically acceptable salts) thereof are thus within the scope of the present invention. Substitution of hydrogen with the heavier isotope ²H (deuterium) may lead to greater metabolic stability, resulting e.g. in an increased *in-vivo* half-life, reduced dosage requirements, or an improved safety profile. In one variant of the invention, the compounds of formula I are not isotopically labelled, or they are labelled only with one or more deuterium atoms. Isotopically labelled compounds of formula I may be prepared in analogy to the methods described hereinafter, but using the appropriate isotopic variation of suitable reagents or starting materials.
14) Another embodiment of the invention relates to a compound of formula I according to embodiment 1) selected from the group consisting of:
   (*R*)-*N*-hydroxy-4-(6-((3-hydroxyoxetan-3-yl)buta-1,3-diyn-1-yl)benzo[*d*]thiazol-2-yl)-2-methyl-2-(methylsulfonyl)butanamide,
   (*R*)-4-(6-(2-fluoro-4-methoxyphenyl)benzo[*d*]thiazol-2-yl)-*N*-hydroxy-2-methyl-2-(methylsulfonyl)butanamide,
   (R)-*N*-hydroxy-4-(6-((4-(3-hydroxyoxetan-3-yl)phenyl)ethynyl)benzo[*d*]thiazol-2-yl)-2-methyl-2-(methylsulfonyl)butanamide,
   (*R*)-*N*-hydroxy-4-(6-(5-hydroxy-5-methylhexa-1,3-diyn-1-yl)benzo[*d*]thiazol-2-yl)-2-methyl-2-(methylsulfonyl)butanamide,
   (*R*)-4-(6-((*S*)-5,6-dihydroxyhexa-1,3-diyn-1-yl)benzo[*d*]thiazol-2-yl)-*N*-hydroxy-2-methyl-2-(methylsulfonyl)butanamide,
   (*R*)-4-(6-(5-amino-5-methylhexa-1,3-diyn-1-yl)benzo[*d*]thiazol-2-yl)-*N-*hydroxy-2-methyl-2-(methylsulfonyl)butanamide,
   (*R*)-*N*-hydroxy-4-(6-(((*1S*,*2S*)-2-(hydroxymethyl)cyclopropyl)buta-1,3-diyn-1-yl)benzo[*d*]thiazol-2-yl)-2-methyl-2-(methylsulfonyl)butanamide,
   (*R*)-*N*-hydroxy-4-(6-((1-(hydroxymethyl)cyclopropyl)buta-1,3-diyn-1-yl)benzo[*d*]thiazol-2-yl)-2-methyl-2-(methylsulfonyl)butanamide,
   (*R*)-4-(6-((3-aminooxetan-3-yl)buta-1,3-diyn-1-yl)benzo[*d*]thiazol-2-yl)-*N*-hydroxy-2-methyl-2-(methylsulfonyl)butanamide,
   (*R*)-4-(6-((1-aminocyclopropyl)buta-1,3-diyn-1-yl)benzo[*d*]thiazol-2-yl)-*N*-hydroxy-2-methyl-2-(methylsulfonyl)butanamide,
   (*R*)-*N*-hydroxy-4-(6-((4-(1-(hydroxymethyl)cyclopropyl)phenyl)ethynyl)benzo[*d*]thiazol-2-yl)-2-methyl-2-(methylsulfonyl)butanamide,
   (*R*)-*N*-hydroxy-4-(6-((1-(hydroxymethyl)cyclobutyl)buta-1,3-diyn-1-yl)benzo[*d*]thiazol-2-yl)-2-methyl-2-(methylsulfonyl)butanamide,
   (*R*)-4-(6-(((*1s*,*3R*,*4S*)-3,4-dihydroxycyclopentyl)buta-1,3-diyn-1-yl)benzo[*d*]thiazol-2-yl)-*N*-hydroxy-2-methyl-2-(methylsulfonyl)butanamide,
   (*R*)-*N*-hydroxy-4-(6-(5-(3-hydroxyoxetan-3-yl)penta-1,3-diyn-1-yl)benzo[*d*]thiazol-2-yl)-2-methyl-2-(methylsulfonyl)butanamide,
   (*R*)-N-hydroxy-4-(6-(((*1R*,*2R*)-2-(hydroxymethyl)-1-methylcyclopropyl)buta-1,3-diyn-1-yl)benzo[*d*]thiazol-2-yl)-2-methyl-2-(methylsulfonyl)butanamide, (*R*)-4-(6-(((*2S*,*5R*)-5-aminotetrahydro-2*H*-pyran-2-yl)buta-1,3-diyn-1-yl)benzo[*d*]thiazol-2-yl)-N-hydroxy-2-methyl-2-(methylsulfonyl)butanamide,
   (*R*)-4-(6-(5-(dimethylamino)penta-1,3-diyn-1-yl)benzo[*d*]thiazol-2-yl)-*N*-hydroxy-2-methyl-2-(methylsulfonyl)butanamide,
   (*R*)-*N*-hydroxy-4-(6-((4-hydroxytetrahydro-2*H*-pyran-4-yl)buta-1,3-diyn-1-yl)benzo[*d*]thiazol-2-yl)-2-methyl-2-(methylsulfonyl)butanamide,
   (*R*)-*N*-hydroxy-2-methyl-2-(methylsulfonyl)-4-(6-(piperidin-4-ylbuta-1,3-diyn-1-yl)benzo[*d*]thiazol-2-yl)butanamide,
   (*R*)-*N*-hydroxy-4-(6-((3-(hydroxymethyl)oxetan-3-yl)buta-1,3-diyn-1-yl)benzo[*d*]thiazol-2-yl)-2-methyl-2-(methylsulfonyl)butanamide,
   (*R*)-*N*-hydroxy-4-(6-((*cis*-1-hydroxy-3-(hydroxymethyl)cyclobutyl)buta-1,3-diyn-1-yl)benzo[*d*]thiazol-2-yl)-2-methyl-2-(methylsulfonyl)butanamide,
   (*R*)-*N*-hydroxy-4-(6-((1-(2-hydroxyacetyl)piperidin-4-yl)buta-1,3-diyn-1-yl)benzo[*d*]thiazol-2-yl)-2-methyl-2-(methylsulfonyl)butanamide,
   (*R*)-4-(6-(((1*R**,2*R**)-1-fluoro-2-(hydroxymethyl)cyclopropyl)buta-1,3-diyn-1-yl)benzo[*d*]thiazol-2-yl)-*N*-hydroxy-2-methyl-2-(methylsulfonyl)butanamide, and
   (*R*)-4-(6-(((*1R**,*2R**)-2-fluoro-2-(hydroxymethyl)cyclopropyl)buta-1,3-diyn-1-yl)benzo[*d*]thiazol-2-yl)-*N*-hydroxy-2-methyl-2-(methylsulfonyl)butanamide,
   and to salts (in particular the pharmaceutically acceptable salts) of such compounds.
15) Another embodiment of the invention relates to a compound of formula I according to embodiment 1) selected from the group consisting of:
   (*R*)-*N*-hydroxy-4-(6-(4-methoxyphenyl)benzo[*d*]thiazol-2-yl)-2-methyl-2-(methylsulfonyl)butanamide,
   (*R*)-*N*-hydroxy-4-(6-((4-(hydroxymethyl)phenyl)ethynyl)benzo[*d*]thiazol-2-yl)-2-methyl-2-(methylsulfonyl)butanamide,
   (*R*)-4-(6-((4-(3-aminooxetan-3-yl)phenyl)ethynyl)-benzo[*d*]thiazol-2-yl)-*N*-hydroxy-2-methyl-2-(methylsulfonyl)butanamide, and
   (*R*)-*N*-hydroxy-4-(6-(5-hydroxypenta-1,3-diyn-1-yl)benzo[*d*]thiazol-2-yl)-2-methyl-2-(methylsulfonyl)butanamide,
   and to salts (in particular the pharmaceutically acceptable salts) of such compounds.
16) Yet another embodiment of the invention relates to a compound of formula I according to embodiment 1) selected from the group consisting of:
   (*R*)-*N*-hydroxy-4-(6-((1-(2-hydroxyacetyl)azetidin-3-yl)buta-1,3-diyn-1-yl)benzo[*d*]thiazol-2-yl)-2-methyl-2-(methylsulfonyl)butanamide,
   (*R*)-*N*-hydroxy-4-(6-(((*1R*,*2S*)-2-(hydroxymethyl)-2-methylcyclopropyl)buta-1,3-diyn-1-yl)benzo[*d*]thiazol-2-yl)-2-methyl-2-(methylsulfonyl)butanamide, and
   (*R*)-4-(6-(((*1R*,*2R*)-2-fluoro-2-(hydroxymethyl)cyclopropyl)buta-1,3-diyn-1-yl)benzo[d]thiazol-2-yl)-*N*-hydroxy-2-methyl-2-(methylsulfonyl)butanamide,
   and to salts (in particular the pharmaceutically acceptable salts) of such compounds.
17) The invention further relates to the compounds of formula I which are selected from the group consisting of the compounds listed in embodiment 14), the compounds listed in embodiment 15) and the compounds listed in embodiment 16) (and notably from the group consisting of the compounds listed in embodiment 14) and the compounds listed in embodiment 15)). In particular, it also relates to the groups of compounds of formula I selected from the group consisting of the compounds listed in embodiment 14), the compounds listed in embodiment 15) and the compounds listed in embodiment 16), which groups of compounds furthermore correspond to one of embodiments 1) to 12), as well as to the salts (in particular the pharmaceutically acceptable salts) of such compounds (and notably the groups of compounds of formula I selected from the group consisting of the compounds listed in embodiment 14) and the compounds listed in embodiment 15), which groups of compounds furthermore correspond to one of embodiments 1) to 12), as well as to the salts (in particular the pharmaceutically acceptable salts) of such compounds). The invention moreover relates to any individual compound of formula I selected from the group consisting of the compounds listed in embodiment 14), the compounds listed in embodiment 15) and the compounds listed in embodiment 16), and to the salts (in particular the pharmaceutically acceptable salts) of such individual compound.

The compounds of formula I according to this invention, i.e. according to one of embodiments 1) to 17) above, exhibit antibacterial activity, especially against Gram-negative organisms and are therefore suitable to treat bacterial infections in mammals, especially humans. Said compounds may also be used for veterinary applications, such as treating infections in livestock and companion animals. They may further constitute substances for preserving inorganic and organic materials in particular all types of organic materials for example polymers, lubricants, paints, fibres, leather, paper and wood.

They may therefore be used for the treatment or prevention of infectious disorders caused by fermentative or non-fermentative gram negative bacteria, especially those caused by susceptible and multi-drug resistant Gram-negative bacteria. Examples of such Gram-negative bacteria include *Acinetobacter spp.* such as *Acinetobacter baumannii* or *Acinetobacter haemolyticus, Actinobacillus actinomycetemcomitans, Achromobacter spp.* such as *Achromobacter xylosoxidans* or *Achromobacter faecalis, Aeromonas spp.* such as *Aeromonas hydrophila, Bacteroides spp.* such as *Bacteroides fragilis, Bacteroides theataioatamicron, Bacteroides distasonis, Bacteroides ovatus* or *Bacteroides vulgatus, Bartonella hensenae, Bordetella spp.* such as *Bordetella pertussis, Borrelia spp.* such as *Borrelia Burgdorferi, Brucella spp.* such as *Brucella melitensis, Burkholderia spp.* such as *Burkholderia cepacia, Burkholderia pseudomallei* or *Burkholderia mallei, Campylobacter spp.* such as *Campylobacter jejuni, Campylobacter fetus* or *Campylobacter coli, Cedecea, Chlamydia spp.* such as *Chlamydia pneumoniae, Chlamydia trachomatis, Citrobacter spp.* such as *Citrobacter diversus (koseri) or Citrobacter freundii, Coxiella burnetii, Edwardsiella spp.* such as *Edwarsiella tarda, Ehrlichia chafeensis, Eikenella corrodens, Enterobacter spp.* such as *Enterobacter cloacae, Enterobacter aerogenes, Enterobacter agglomerans, Escherichia coli, Francisella tularensis, Fusobacterium spp., Haemophilus spp.* such as *Haemophilus influenzae* (beta-lactamase positive and negative) or *Haemophilus ducreyi, Helicobacter pylori, Kingella kingae, Klebsiella spp.* such as *Klebsiella oxytoca, Klebsiella pneumoniae* (including those encoding extended-spectrum beta-lactamases (hereinafter "ESBLs"), carbapenemases (KPCs), cefotaximase-Munich (CTX-M), metallo-beta-lactamases, and AmpC-type beta-lactamases that confer resistance to currently available cephalosporins, cephamycins, carbapenems, beta-lactams, and beta-lactam/beta-lactamase inhibitor combinations), *Klebsiella rhinoscleromatis* or *Klebsiella ozaenae, Legionella pneumophila, Mannheimia haemolyticus, Moraxella catarrhalis* (beta-lactamase positive and negative), *Morganella morganii, Neisseria spp.* such as *Neisseria gonorrhoeae* or *Neisseria meningitidis, Pasteurella spp.* such as *Pasteurella multocida, Plesiomonas shigelloides, Porphyromonas spp.* such as *Porphyromonas asaccharolytica, Prevotella spp.* such as *Prevotella corporis, Prevotella intermedia* or *Prevotella endodontalis, Proteus spp.* such as *Proteus mirabilis, Proteus vulgaris, Proteus penneri* or *Proteus myxofaciens, Porphyromonas asaccharolytica, Plesiomonas shigelloides, Providencia spp.* such as *Providencia stuartii, Providencia rettgeri* or *Providencia alcalifaciens, Pseudomonas spp.* such as *Pseudomonas aeruginosa* (including ceftazidime-, cefpirome- and cefepime-resistant *P. aeruginosa,* carbapenem-resistant *P. aeruginosa* or quinolone-resistant *P. aeruginosa*) or *Pseudomonas fluorescens, Ricketsia prowazekii, Salmonella spp.* such as *Salmonella typhi* or *Salmonella paratyphi, Serratia marcescens, Shigella spp.* such as *Shigella flexneri, Shigella boydii, Shigella sonnei* or *Shigella dysenteriae, Streptobacillus moniliformis, Stenotrophomonas maltophilia, Treponema spp., Vibrio spp.* such as *Vibrio cholerae, Vibrio parahaemolyticus, Vibrio vulnificus, Vibrio alginolyticus, Yersinia spp.* such as *Yersinia enterocolitica, Yersinia pestis* or *Yersinia pseudotuberculosis.*

The compounds of formula I according to this invention are thus useful for treating a variety of infections caused by fermentative or non-fermentative Gram-negative bacteria, especially infections such as: nosocomial pneumonia (related to infection by *Legionella pneumophila, Haemophilus influenzae,* or *Chlamydia pneumonia*); urinary tract infections; systemic infections (bacteraemia and sepsis); skin and soft tissue infections (including burn patients); surgical infections; intraabdominal infections; lung infections (including those in patients with cystic fibrosis); *Helicobacter pylori* (and relief of associated gastric complications such as peptic ulcer disease, gastric carcinogenesis, etc.); endocarditis; diabetic foot infections; osteomyelitis; otitis media, sinusitus, bronchitis, tonsillitis, and mastoiditis related to infection by *Haemophilus influenzae* or *Moraxella catarrhalis;* pharynigitis, rheumatic fever, and glomerulonephritis related to infection by *Actinobacillus haemolyticum;* sexually transmitted diseases related to infection by *Chlamydia trachormatis, Haemophilus ducreyi, Treponema pallidum, Ureaplasma urealyticum,* or *Neisseria gonorrheae;* systemic febrile syndromes related to infection by *Borrelia recurrentis*; Lyme disease related to infection by *Borrelia burgdorferi;* conjunctivitis, keratitis, and dacrocystitis related to infection by *Chlamydia trachomatis, Neisseria gonorrhoeae* or *H. influenzae;* gastroenteritis related to infection by *Campylobacter jejuni;* persistent cough related to infection by *Bordetella pertussis* and gas gangrene related to infection by *Bacteroides spp.* Other bacterial infections and disorders related to such infections that may be treated or prevented in accord with the method of the present invention are referred to in J. P. Sanford et al., "The Sanford Guide to Antimicrobial Therapy", 26th Edition, (Antimicrobial Therapy, Inc., 1996).

The preceding lists of infections and pathogens are to be interpreted merely as examples and in no way as limiting.

The compounds of formula I according to this invention, or the pharmaceutically acceptable salts thereof, may therefore be used for the preparation of a medicament, and are suitable, for the prevention or treatment of a bacterial infection, in particular for the prevention or treatment of a bacterial infection caused by Gram-negative bacteria, especially by multi-drug resistant Gram-negative bacteria.

The compounds of formula I according to this invention, or the pharmaceutically acceptable salts thereof, may thus especially be used for the preparation of a medicament, and are suitable, for the prevention or treatment of a bacterial infection caused by Gram-negative bacteria selected from the group consisting of *Burkholderia spp.* (e.g. *Burkholderia cepacia*)*, Citrobacter spp., Enterobacter aerogenes, Enterobacter cloacae, Escherichia coli, Klebsiella oxytoca, Klebsiella pneumoniae, Serratia marcescens, Stenotrophomonas maltophilia* and *Pseudomonas aeruginosa* (notably for the prevention or treatment of a bacterial infection caused by *Escherichia coli* bacteria, *Klebsiella pneumoniae* bacteria or *Pseudomonas aeruginosa* bacteria, and in particular for the prevention or treatment of a bacterial infection mediated by quinolone-resistant, carbapenem-resistant or multi-drug resistant *Klebsiella pneumoniae* bacteria).

The compounds of formula I according to this invention, or the pharmaceutically acceptable salts thereof, may more especially be used for the preparation of a medicament, and are suitable, for the prevention or treatment of a bacterial infection caused by Gram-negative bacteria selected from the group consisting of *Citrobacter spp., Enterobacter aerogenes, Enterobacter cloacae, Escherichia coli, Klebsiella oxytoca, Klebsiella pneumoniae, Serratia marcescens, Stenotrophomonas maltophilia* and *Pseudomonas aeruginosa* bacteria (notably of a bacterial infection caused by Gram-negative bacteria selected from the group consisting of *Klebsiella pneumoniae* and *Pseudomonas aeruginosa* bacteria, and in particular of a bacterial infection caused by *Pseudomonas aeruginosa* bacteria).

The compounds of formula I according to this invention, or the pharmaceutically acceptable salts thereof, may thus especially be used for the preparation of a medicament, and are suitable, for the prevention or treatment of a bacterial infection selected from urinary tract infections, systemic infections (such as bacteraemia and sepsis), skin and soft tissue infections (including burn patients), surgical infections; intraabdominal infections and lung infections (including those in patients with cystic fibrosis).

The compounds of formula I according to this invention, or the pharmaceutically acceptable salts thereof, may more especially be used for the preparation of a medicament, and are suitable, for the prevention or treatment of a bacterial infection selected from urinary tract infections, intraabdominal infections and lung infections (including those in patients with cystic fibrosis), and in particular for the prevention or treatment of a bacterial infection selected from urinary tract infections and intraabdominal infections.

Besides, the compounds of formula I according to this invention display intrinsic antibacterial properties and have the ability to improve permeability of the outer membrane of Gram-negative bacteria to other antibacterial agents. Their use in combination with another antibacterial agent might offer some further advantages such as lowered side-effects of drugs due to lower doses used or shorter time of treatment, more rapid cure of infection shortening hospital stays, increasing spectrum of pathogens controlled, and decreasing incidence of development of resistance to antibiotics. The antibacterial agent for use in combination with a compound of formula I according to this invention will be selected from the group consisting of a penicillin antibiotic (such as ampicillin, piperacillin, penicillin G, amoxicillin, or ticarcillin), a cephalosporin antibiotic (such as ceftriaxone, cefatazidime, cefepime, cefotaxime) a carbapenem antibiotic (such as imipenem, or meropenem), a monobactam antibiotic (such as aztreonam or carumonam), a fluoroquinolone antibiotic (such as ciprofloxacin, moxifloxacin or levofloxacin), a macrolide antibiotic (such as erythromycin or azithromycin), an aminoglycoside antibiotic (such as amikacin, gentamycin or tobramycin), a glycopeptide antibiotic (such as vancomycin or teicoplanin), a tetracycline antibiotic (such as tetracycline, oxytetracycline, doxycycline, minocycline or tigecycline), and linezolid, clindamycin, telavancin, daptomycin, novobiocin, rifampicin and polymyxin. Preferably, the antibacterial agent for use in combination with a compound of formula I according to this invention will be selected from the group consisting of vancomycin, tigecycline and rifampicin.

The compounds of formula I according to this invention, or the pharmaceutically acceptable salt thereof, may moreover be used for the preparation of a medicament, and are suitable, for the prevention or treatment (and especially the treatment) of infections caused by biothreat Gram negative bacterial pathogens as listed by the US Center for Disease Control (the list of such biothreat bacterial pathogens can be found at the web page http://www.selectagents.gov/Select%20Agents%20and%20Toxins%20List.html), and in particular by Gram negative pathogens selected from the group consisting of *Yersinia pestis, Francisella tularensis* (tularemia), *Burkholderia pseudomallei* and *Burkholderia mallei.*

One aspect of this invention therefore relates to the use of a compound of formula I according to one of embodiments 1) to 17), or of a pharmaceutically acceptable salt thereof, for the manufacture of a medicament for the prevention or treatment of a bacterial infection (in particular one of the previously mentioned infections caused by Gram-negative bacteria, especially by multi-drug resistant Gram-negative bacteria). Another aspect of this invention relates to a compound of formula I according to one of embodiments 1) to 17), or a pharmaceutically acceptable salt thereof, for the prevention or treatment of a bacterial infection (in particular for the prevention or treatment of one of the previously mentioned infections caused by Gram-negative bacteria, especially by multi-drug resistant Gram-negative bacteria). Yet another aspect of this invention relates to a compound of formula I according to one of embodiments 1) to 17), or a pharmaceutically acceptable salt thereof, as a medicament. Yet a further aspect of this invention relates to a pharmaceutical composition containing, as active principle, a compound of formula I according to one of embodiments 1) to 17), or a pharmaceutically acceptable salt thereof, and at least one therapeutically inert excipient.

As well as in humans, bacterial infections can also be treated using compounds of formula I (or pharmaceutically acceptable salts thereof) in other species like pigs, ruminants, horses, dogs, cats and poultry.

The present invention also relates to pharmacologically acceptable salts and to compositions and formulations of compounds of formula I.

A pharmaceutical composition according to the present invention contains at least one compound of formula I (or a pharmaceutically acceptable salt thereof) as the active agent and optionally carriers and/or diluents and/or adjuvants, and may also contain additional known antibiotics.

The compounds of formula I and their pharmaceutically acceptable salts can be used as medicaments, e.g. in the form of pharmaceutical compositions for enteral or parenteral administration.

The production of the pharmaceutical compositions can be effected in a manner which will be familiar to any person skilled in the art (see for example Remington, The Science and Practice of Pharmacy, 21st Edition (2005), Part 5, "Pharmaceutical Manufacturing" [published by Lippincott Williams & Wilkins]) by bringing the described compounds of formula I or their pharmaceutically acceptable salts, optionally in combination with other therapeutically valuable substances, into a galenical administration form together with suitable, non-toxic, inert, therapeutically compatible solid or liquid carrier materials and, if desired, usual pharmaceutical adjuvants.

Moreover, the compounds of formula I according to this invention may also be used for cleaning purposes, e.g. to remove pathogenic microbes and bacteria from surgical instruments, catheters and artificial implants or to make a room or an area aseptic. For such purposes, the compounds of formula I could be contained in a solution or in a spray formulation.

This invention, thus, relates to the compounds of formula I as defined in embodiment 1), or further limited under consideration of their respective dependencies by the characteristics of any one of embodiments 2) to 17), and to pharmaceutically acceptable salts thereof. It relates furthermore to the use of such compounds as medicaments, especially for the prevention or treatment of a bacterial infection, in particular for the prevention or treatment of a bacterial infection caused by Gram-negative bacteria (notably for the prevention or treatment of a bacterial infection caused by *Escherichia coli* bacteria, *Klebsiella pneumoniae* bacteria or *Pseudomonas aeruginosa* bacteria, and in particular for the prevention or treatment of a bacterial infection caused by *Klebsiella pneumoniae* quinolone-resistant, carbapenem-resistant or multi-drug resistant bacteria). The following embodiments relating to compounds of formula I according to embodiment 1) are thus possible and intended and herewith specifically disclosed in individualised form:
1, 2+1, 3+2+1, 4+2+1, 5+4+2+1, 6+2+1, 7+6+2+1, 8+1, 9+8+1, 10+9+8+1, 11+1, 12+1, 13+1, 13+2+1, 13+3+2+1, 13+4+2+1, 13+5+4+2+1, 13+6+2+1, 13+7+6+2+1, 13+8+1, 13+9+8+1, 13+10+9+8+1, 13+11+1, and 13+12+1.

In the list above, the numbers refer to the embodiments according to their numbering provided hereinabove whereas "+" indicates the dependency from another embodiment. The different individualised embodiments are separated by commas. In other words, "4+2+1" for example refers to embodiment 4) depending on embodiment 2), depending on embodiment 1), i.e. embodiment "4+2+1" corresponds to embodiment 1) further limited by the features of embodiments 2) and 4).

The compounds of formula I can be manufactured in accordance with the present invention using the procedures described hereafter.

### PREPARATION OF THE COMPOUNDS OF FORMULA I

### Abbreviations:

The following abbreviations are used throughout the specification and the examples:
- Ac: acetyl
- AcOH: acetic acid
- aq.: aqueous
- Boc: *tert*-butyloxycarbonyl
- CC: column chromatography over silica gel
- Cipro: ciprofloxacin
- Cy: cyclohexyl
- DAD: diode array detection
- dba: dibenzylideneacetone
- DCC: dicyclohexylcarbodiimide
- DCM: dichloromethane
- DEA: diethylamine
- DIBAH: diisobutylaluminium hydride
- DIPEA: diisopropylethylamine
- DME: 1,2-dimethoxyethane
- DMF: *N*,*N*-dimethylformamide
- DMSO: dimethylsulfoxide
- EA: ethyl acetate
- EDC: *N*-(3-dimethylaminopropyl)-*N'*-ethylcarbodiimide hydrochloride
- ELSD: evaporative light scattering detector
- ESI: electron spray ionisation
- Et: ethyl
- Et₂O: diethyl ether
- EtOH: ethanol
- h: hour(s)
- HATU: *O*-(7-azabenzotriazol-1-yl)-*N*,*N*,*N'*,*N'*-tetramethyluronium hexafluorophosphate
- Hept: heptane
- Hex: hexane
- HOBT: hydroxybenzotriazole
- HPLC: high performance liquid chromatography
- IT: internal temperature
- LC-MS: liquid chromatrography - mass spectrometry
- Me: methyl
- MeCN: acetonitrile
- MeOH: methanol
- min: minute(s)
- MS: mass spectroscopy
- Ms: methyl sulfonyl (mesyl)
- NBS: *N*-bromosuccinimide
- *n*-BuLi: *n*-butyl lithium
- NMR: Nuclear Magnetic Resonance
- org.: organic
- Pd/C: palladium on carbon
- PE: petroleum ether
- PEPPSI™-IPr: [1,3-bis(2,6-diisopropylphenyl)imidazol-2-ylidene](3-chloropyridyl)palladium(II) dichloride
- Ph: phenyl
- prep-HPLC: preparative HPLC
- Pyr: pyridine
- quant.: quantitative yield
- Q-phos: 1,2,3,4,5-pentaphenyl-1'-(di-*tert*-butylphosphino)ferrocene
- rt: room temperature
- sat.: saturated
- SK-CC01-A: 2'-(dimethylamino)-2-biphenylyl-palladium(II) chloride dinorbornylphosphine complex
- S-Phos: 2-dicyclohexylphosphino-2',6'-dimethoxybiphenyl
- TBAF: tetra-*n*-butylammonium fluoride
- TBDPS: *tert*-butyldiphenylsilyl
- TBME: *tert*-butlymethylether
- tBu: *tert*-butyl
- TEA: triethylamine
- Tf: trifluoromethylsulfonyl (triflyl)
- TFA: trifluoroacetic acid
- THF: tetrahydrofuran
- THP: tetrahydropyranyl
- THPO-NH₂: *O*-(tetrahydropyran-2-yl)hydroxylamine
- TMSE: 2-(trimethylsilyl)ethyl
- t_{R}: retention time
- Ts: *para*-toluenesulfonyl

### General reaction techniques:

### General reaction technique 1 (hydroxamic acid protecting group removal):

The protecting groups R of the hydroxamic acid ester derivatives (CONHOR) are removed as follows:
- When R is THP, (2-methylpropoxy)ethyl, methoxymethyl, tBu, COOtBu or COtBu: by acidic treatment with e.g. TFA or HCl in an org. solvent such as DCM, dioxane, Et₂O or MeOH between 0°C and rt or by treatment with pyridinium *para*-toluenesulfonate in EtOH between rt and 80°C;
- When R is trityl: by treatment with diluted acid such as citric acid or HCl in an org. solvent such as MeOH or DCM;
- When R is benzyl: by hydrogenolysis using general reaction technique 5;
- When R is TMSE: by using fluoride anion sources such as BF₃.etherate complex in MeCN at 0°C, TBAF in THF between 0°C and +40°C or HF in MeCN or water between 0°C and +40°C, or using acidic conditions such as AcOH in THF/MeOH or HCl in MeOH;
- When R is allyl: by treatment with Pd(PPh₃)₄ in a solvent such as MeOH in presence of K₂CO₃ or a scavenger such as dimedone, morpholine or tributyltin hydride;
- When R is COMe: by treatment with diluted NaOH or Na₂CO₃ in a solvent such as MeOH.

Further general methods to remove hydroxamic acid protecting groups have been described in T.W. Greene & P.G.M. Wuts, Protecting Groups in Organic Synthesis, 3rd Ed (1999), 23-147 (Publisher: John Wiley and Sons, Inc., New York, N.Y.).

### General reaction technique 2 (amide coupling):

The carboxylic acid is reacted with the hydroxylamine derivative in the presence of an activating agent such as DCC, EDC, HOBT, *n*-propylphosphonic cyclic anhydride, HATU or di-(*N*-succinimidyl)-carbonate, in a dry aprotic solvent such as DCM, MeCN or DMF between -20°C and 60°C (see G. Benz in Comprehensive Organic Synthesis, B.M. Trost, I. Fleming, Eds; Pergamon Press: New York (1991), vol. 6, p. 381). Alternatively, the carboxylic acid can be activated by conversion into its corresponding acid chloride by reaction with oxalyl chloride or thionyl chloride neat or in a solvent like DCM between -20° and 60°C. Further activating agents can be found in R. C. Larock, Comprehensive Organic Transformations. A guide to Functional Group Preparations, 2nd Edition (1999), section nitriles, carboxylic acids and derivatives, p. 1941-1949 (Wiley-VC; New York, Chichester, Weinheim, Brisbane, Singapore, Toronto).

### General reaction technique 3 (Suzuki coupling):

The aromatic halide (typically a bromide) is reacted with the required boronic acid derivative or its boronate ester equivalent (e.g. pinacol ester) in the presence of a palladium catalyst and a base such as K₂CO₃, CS₂CO₃, K₃PO₄, tBuONa or tBuOK between 20 and 120°C in a solvent such as toluene, THF, dioxane, DME or DMF, usually in the presence of water (20 to 50%). Examples of typical palladium catalysts are triarylphosphine palladium complexes such as Pd(PPh₃)₄. These catalysts can also be prepared *in situ* from a common palladium source such as Pd(OAc)₂ or Pd₂(dba)₃ and a ligand such as trialkylphosphines (e.g. PCy₃ or P(tBu)₃), dialkylphosphinobiphenyls (e.g. S-Phos) or ferrocenylphosphines (e.g. Q-phos). Alternatively, one can use a commercially available precatalyst based on palladacycle (e.g. SK-CC01-A) or *N*-heterocyclic carbene complexes (e.g. PEPPSI™-IPr). The reaction can also be performed by using the corresponding aromatic triflate. Further variations of the reaction are described in Miyaura and Suzuki, Chem. Rev. (1995), 95, 2457-2483, Bellina et al., Synthesis (2004), 2419-2440, Mauger and Mignani, Aldrichimica Acta (2006), 39, 17-24, Kantchev et al., Aldrichimica Acta (2006), 39, 97-111, Fu, Acc. Chem. Res. (2008), 41, 1555-1564, and references cited therein.

### General reaction technique 4 (Sonogashira coupling):

The alkyne derivative is reacted with the corresponding bromo derivative, using a catalytic amount of a palladium salt, an org. base such as TEA and a catalytic amount of a copper derivative (usually copper iodide) in a solvent such as DMF between 20°C to 100°C (see Sonogashira, K. in Metal-Catalyzed Reactions, Diederich, F., Stang, P.J., Eds.; Wiley-VCH, New York (1998)).

### General reaction technique 5 (Hydrogenolysis of a benzyl protecting group):

The benzyl protected hydroxamic acid, dissolved in a solvent such as MeOH, EA or THF, is cleaved under hydrogen atmosphere in presence of a noble metal catalyst such as Pd/C or PtO₂, or Raney Ni. At the end of the reaction the catalyst is filtered off and the filtrate is evaporated under reduced pressure. Alternatively the reduction can be performed by catalytic transfer hydrogenation using Pd/C and ammonium formate as hydrogen source.

### General reaction technique 6 (transformation of an ester into an acid):

When the ester side chain is a linear alkyl, the hydrolysis is usually performed by treatment with an alkali hydroxide such as LiOH, KOH or NaOH in a water-dioxane or water-THF mixture between 0°C and 80°C. When the ester side chain is tBu, the release of the corresponding acid can also be performed in neat TFA or diluted TFA or HCl in an org. solvent such as ether or THF. When the ester side chain is the allyl group, the reaction is performed in the presence of tetrakis(triphenylphosphine)palladium(0) in the presence of an allyl cation scavenger such as morpholine, dimedone or tributyltin hydride between 0°C and 50°C in a solvent such as THF. When the ester side chain is benzyl, the reaction is performed under hydrogen in the presence of a noble metal catalyst such as Pd/C in a solvent such as MeOH, THF or EA. Further strategies to introduce other acid protecting groups and general methods to remove them have been described in T.W. Greene & P.G.M. Wuts, Protecting Groups in Organic Synthesis, 3rd Ed. (1999), 369-441 (Publisher: John Wiley and Sons, Inc., New York, N.Y.).

### General reaction technique 7 (alcohol activation):

The alcohol is reacted with MsCl, TfCl or TsCl in the presence of a base such as TEA in a dry aprotic solvent such as Pyr, THF or DCM between -30°C and +50°C. In the case of the triflate or mesylate, Tf₂O or Ms₂O can also be used.

### General reaction technique 8 (transformation of a bromo aryl to the corresponding iodo aryl):

A bromo aryl derivative can be transformed into the corresponding iodo aryl derivative by an aromatic Finkelstein reaction using an excess of NaI in the presence of a catalytic amount of CuI and *trans*-*N*,*N'*-dimethylcyclohexanediamine in a solvent such as toluene or dioxane at a temperature ranging between rt and 100°C, according to Buchwald, S. and al. J. Am. Chem. Soc. 2002, 124, 14844-14845. Alternatively the reaction can be performed in a microwave oven at 150°C.

### General preparation methods:

### Preparation of the compounds of formula I:

The compounds of formula I can be manufactured by the methods given below, by the methods given in the examples or by analogous methods. Optimum reaction conditions may vary with the particular reactants or solvents used, but such conditions can be determined by a person skilled in the art by routine optimisation procedures.

The sections hereafter describe general methods for preparing compounds of formula I. If not indicated otherwise, the generic groups R¹, M, M^{A}, M^{B}, A, R^{1A}, R^{2A}, R^{3A} and R^{1B} are as defined for formula I. General synthetic methods used repeatedly throughout the text below are referenced to and described in the above section entitled "General reaction techniques". In some instances certain generic groups might be incompatible with the assembly illustrated in the procedures and schemes below and so will require the use of protecting groups. The use of protecting groups is well known in the art (see for example T.W. Greene and P.G.M. Wuts, Protective Groups in Organic Synthesis, 3rd Ed. (1999), Wiley-Interscience).

The compounds of formula I can be obtained by deprotecting a compound of formula II 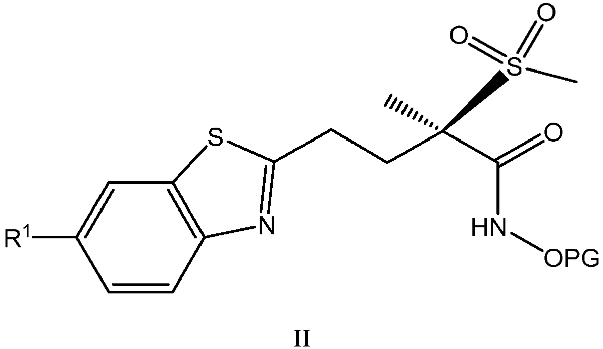 wherein R¹ has the same meaning as in formula I and PG represents THP, TMSE, benzyl, trityl, (2-methylpropoxy)ethyl, methoxymethyl, allyl, tBu, acetyl, COOtBu or COtBu using general reaction technique 1. The reaction can also be performed with racemic material and the (R) enantiomer can be obtained by chiral HPLC separation.

If desired, the compounds of formula I thus obtained may be converted into their salts, and notably into their pharmaceutically acceptable salts using standard methods.

Besides, whenever the compounds of formula I are obtained in the form of mixtures of enantiomers, the enantiomers can be separated using methods known to one skilled in the art, e.g. by formation and separation of diastereomeric salts or by HPLC over a chiral stationary phase such as a Regis Whelk-O1(R,R) (10 µm) column, a Daicel ChiralCel OD-H (5-10 µm) column, or a Daicel ChiralPak IA (10 µm) or AD-H (5 µm) column. Typical conditions of chiral HPLC are an isocratic mixture of eluent A (EtOH, in the presence or absence of an amine such as TEA or diethylamine) and eluent B (Hex), at a flow rate of 0.8 to 150 mL/min.

### Preparation of the compounds of formula II:

The compounds of formula II can be obtained by:
a) reacting a compound of formula III 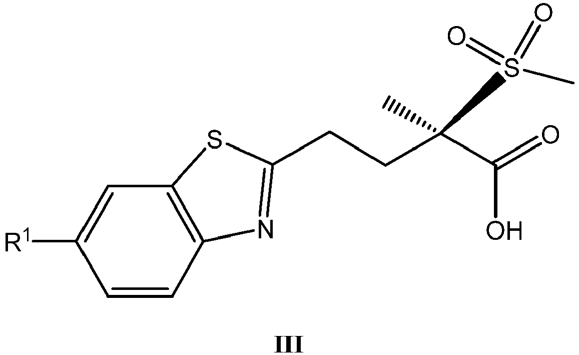 wherein R¹ has the same meaning as in formula I with a compound of formula IV

   H₂N-OPG IV

   wherein PG has the same meaning as in formula II using general reaction technique 2 (this reaction can also be performed with racemic compound of formula III and the (*R*)-enantiomer can then be obtained by chiral HPLC separation of the reaction product), whereby functional groups (e.g. amino or hydroxy) present on R¹ that would be incompatible with the coupling conditions mentioned in general reaction technique 2 can be protected (as carbamates or THP/silyl ethers respectively) before performing said reaction and deprotected after performing said reaction; or
b) reacting a boron derivative of formula V 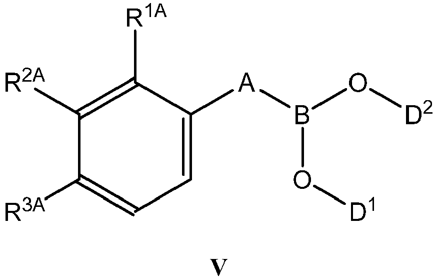 wherein R^{1A}, R^{2A} and R^{3A} have the same respective meanings as in formula I, A represents a bond and D¹ and D² represent H, (C₁-C₄)alkyl such as methyl or ethyl or D¹ and D² together represent CH₂C(Me)₂CH₂ or C(Me)₂C(Me)₂ with a compound of formula VI 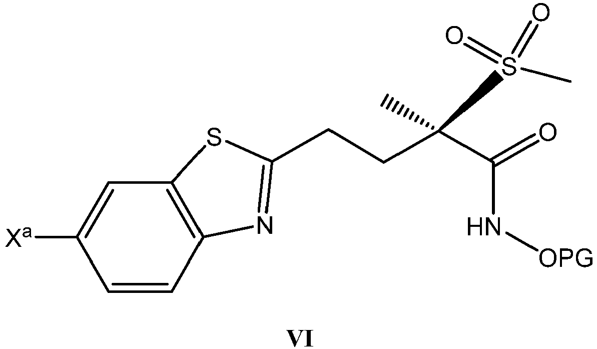 wherein X^{a} represents a halogen such as bromine or iodine and PG has the same meaning as in formula II, using general reaction technique 3 (this reaction can also be performed with racemic compound of formula VI and the (R)-enantiomer can then be obtained by chiral HPLC separation of the reaction product); or
c) reacting a compound of formula VII 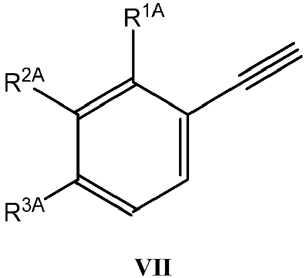 wherein R^{1A}, R^{2A} and R^{3A} have the same respective meanings as in formula I, with a compound of formula VI as defined in section b) above wherein X^{a} represents iodine, using general reaction technique 4 (this reaction can also be performed with racemic compound of formula VI and the (*R*)-enantiomer can then be obtained by chiral HPLC separation of the reaction product); or
d) reacting a compound of formula VIII 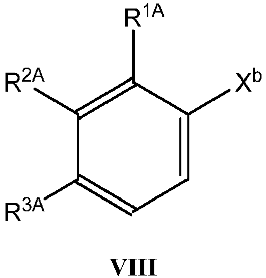 wherein R^{1A}, R^{2A} and R^{3A} have the same respective meanings as in formula I and X^{b} represents iodine or bromine (and preferably iodine), with a compound of formula VIa 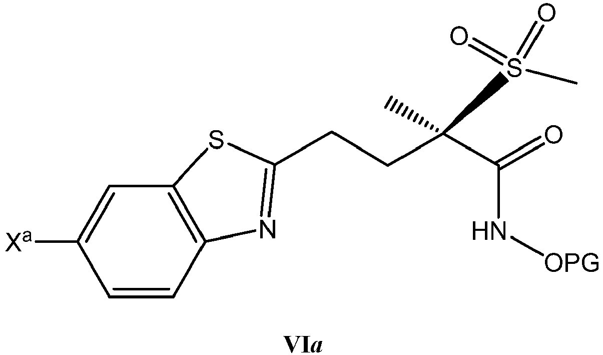 wherein X^{a} represents ethynyl and PG has the same meaning as in formula II, using general reaction technique 4 (this reaction can also be performed with racemic compound of formula VIa and the (*R*)-enantiomer can then be obtained by chiral HPLC separation of the reaction product); or
e) reacting a compound of formula IX 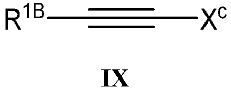 wherein R^{1B} has the same meaning as in formula I and X^{c} represents iodine or bromine, with a compound of formula VI*a* as defined in section d) above, using general reaction technique 4 (this reaction can also be performed with racemic compound of formula VI*a* and the (*R*)-enantiomer can then be obtained by chiral HPLC separation of the reaction product).

### Preparation of the synthesis intermediates of formulae III, IV, V, VI, VIa, VII, VIII and IX:

### Compounds of formula III:

The compounds of formula III can be prepared as summarised in Scheme 1 hereafter.

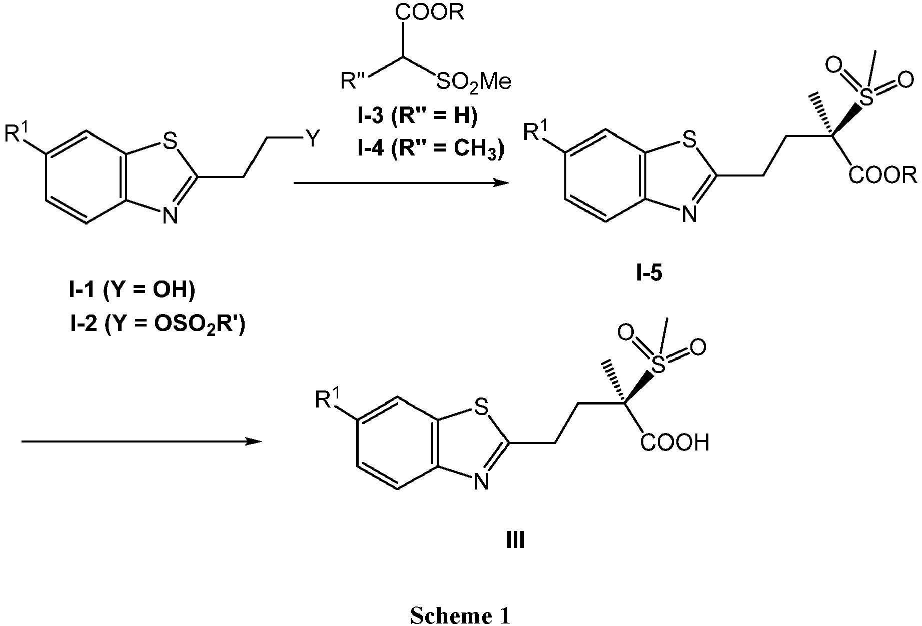

In Scheme 1, R¹ has the same meaning as in formula I, R represents (C₁-C₅)alkyl, allyl or benzyl and R' represents CH₃, CF₃ or tolyl. The reactions can also be performed with racemic material and the (*R*)-enantiomer can be obtained by chiral HPLC separation at any step when suitable.

The alcohols of formula I-1 can be transformed to the compounds of formula 1-2 using general reaction technique 7. The compounds of formula 1-2 can be reacted either with a 2-(methylsulfonyl)acetate derivative of formula 1-3 in the presence of NaH, followed by alkylation with MeI in the presence of NaH, or directly with a 2-(methylsulfonyl)propanoate derivative of formula 1-4 in the presence of NaH, affording the compounds of formula 1-5. The compounds of formula 1-5 are transformed into the carboxylic acid derivatives of formula III using general reaction technique 6.

### Compounds of formula IV:

The compounds of formula IV are commercially available (PG = THP, tBu, COOtBu, Bn, TMSE, Tr, Ac, MOM or allyl) or can be prepared according to WO 2010/060785 (PG = (2-methylpropoxy)ethyl) or Marmer and Maerker, J. Org. Chem. (1972), 37, 3520-3523 (PG = COtBu).

### Compounds of formula V:

The compounds of formula V wherein A is a bond and D¹ and D² each represent H or (C₁-C₄)alkyl are commercially available or can be prepared according to Sleveland et al., Organic Process Research & Development (2012), 16, 1121-1130 starting from tri((C₁-C₂)alkyl)borate and the corresponding commercially available bromo derivatives (optionally followed by acidic hydrolysis). The compounds of formula V wherein A represents a bond and D¹ and D² together represent CH₂C(Me)₂CH₂ or C(Me)₂C(Me)₂ are commercially available or can be prepared according to WO 2012/093809, starting from bis(pinacolato)diborane or 5,5-dimethyl-1,3,2-dioxaborinane (both commercially available) with the corresponding commercially available bromo derivatives of formula VIII.

### Compounds of formulae VI and VIa:

The compounds of formulae VI and VI*a* can be prepared as summarised in Scheme 2 hereafter.

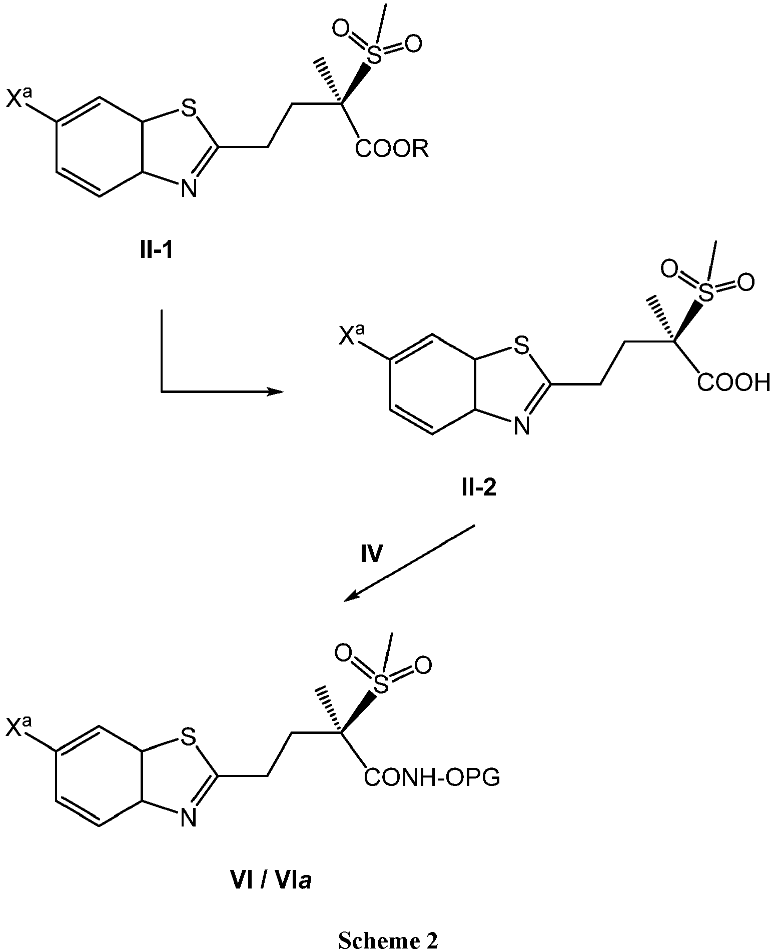

In Scheme 2, R represents (C₁-C₅)alkyl, allyl or benzyl, X^{a} represents iodine, bromine or ethynyl and PG has the same meaning as in formula II. The reactions can also be performed with racemic material and the (*R*)-enantiomer can be obtained by chiral HPLC separation at any step when suitable.

The derivatives of formula II-1 can be transformed into the carboxylic acid derivatives of formula II-2 using general reaction technique 6 and further reacted with the compounds of formula IV using general reaction technique 2, thus affording the compounds of formula VI (X^{a} = iodine of bromine) or VI*a* (X^{a} = ethynyl). The derivatives of formula VI wherein X^{a} represents bromine can be transformed into the corresponding derivatives wherein X^{a} represents iodine using general reaction technique 8. The resulting compounds of formula VI wherein X^{a} represents iodine can be reacted with trimethylsilylacetylene using general reaction technique 4, followed by treatment with an inorganic base such as K₂CO₃ in an appropriate alcoholic solvent such as MeOH, or by treatment with TBAF in THF, affording the derivatives of formula VI*a*.

### Compounds of formula VII:

The compounds of formula VII are commercially available or can be prepared as summarised in Scheme 3 hereafter.

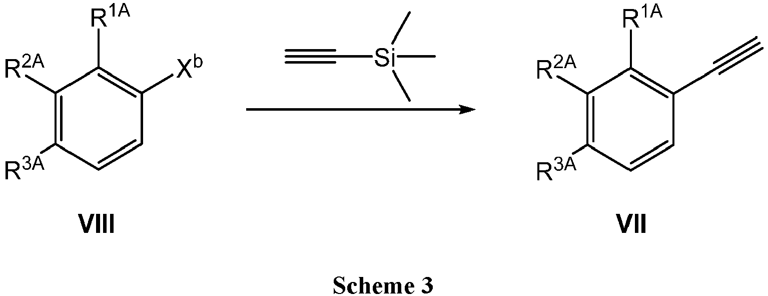

In Scheme 3, R^{1A}, R^{2A} and R^{3A} have the same respective meanings as in formula I and X^{b} represents a halogen such as bromine or iodine.

The derivatives of formula VIII wherein X^{b} represents bromine can be transformed into the corresponding derivatives wherein X^{b} represents iodine using general reaction technique 8. The resulting compounds of formula VIII wherein X^{b} represents iodine can be reacted with trimethylsilylacetylene using general reaction technique 4, followed by treatment with an inorganic base such as K₂CO₃ in an appropriate alcoholic solvent such as MeOH, or by treatment with TBAF in THF, affording the derivatives of formula VII.

### Compounds of formula VIII:

The compounds of formula VIII wherein X^{b} represents bromine are commercially available or can be prepared by standard methods known to one skilled in the art.

### Compounds of formula IX:

The compounds of formula IX wherein X^{c} represents iodine can be prepared from the corresponding compounds wherein X^{c} is H by treatment with iodine in the presence of an inorganic base such as KOH. The compounds of formula IX wherein X^{c} represents bromine can be prepared by reacting the corresponding compounds wherein X^{c} is H with NBS in presence of silver nitrate in a solvent such as acetone or acetonitrile.

### Other synthesis intermediates and starting materials:

The compounds of formula II-1 wherein X^{a} represents bromine can be prepared as summarised in Scheme 4 hereafter.

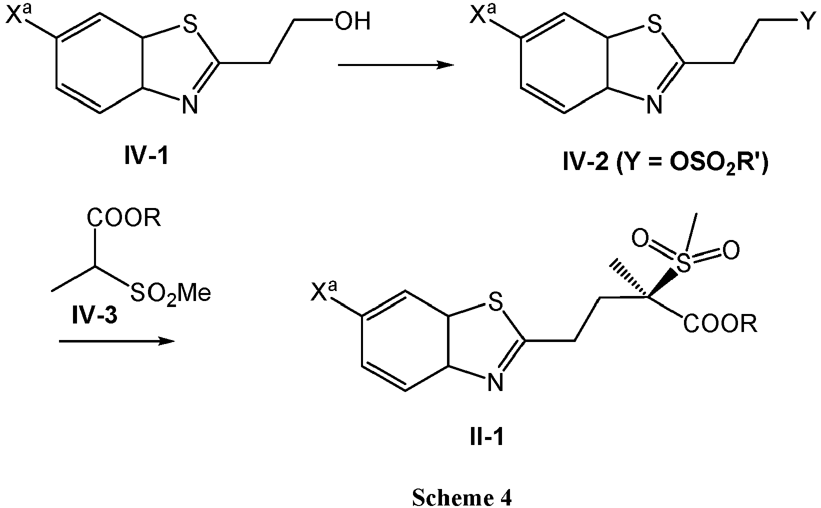

In Scheme 4, R represents (C₁-C₅)alkyl, allyl or benzyl, R' represents CH₃, CF₃ or tolyl and X^{a} represents bromine. The reactions can also be performed with racemic material and the (*R*)-enantiomer can be obtained by chiral HPLC separation at any step when suitable.

The alcohols of formula IV-1 can be transformed into the derivatives of formulae IV-2 using general reaction technique 7. The compounds of formula IV-2 can then be reacted with the compounds of formula IV-3 in the presence of NaH, affording the compounds of formula II-1 wherein X^{a} represents bromine.

The compounds of formula II-1 wherein X^{a} represents an ethynyl group can be prepared from the compounds of formula II-1 wherein X^{a} represents bromine applying first general reaction technique 8. The resulting compounds of formula II-1 wherein X^{a} represents iodine can be reacted with trimethylsilylacetylene using general reaction technique 4, followed by treatment with an inorganic base such as K₂CO₃ in an appropriate alcoholic solvent such as MeOH, or by treatment with TBAF in THF.

The compound of formula IV-1 wherein X^{a} represents bromine is commercially available or can be prepared by standard methods known to one skilled in the art.

The compounds of formula I-1 wherein R¹ has the same meaning as in formula I can be prepared from compounds of formula IV-1 wherein X^{a} represents bromine, iodine or ethynyl using general reaction techniques 3 or 4 and the appropriate compounds of formula V, VII, VIII, or IX as previously described. The compound of formula IV-1 wherein X^{a} represents iodine can be prepared from commercially available compound of formula IV-1 wherein X^{a} represents bromine using general reaction technique 8. The resulting iodo derivative can be reacted with trimethylsilylacetylene using general reaction technique 4, followed by treatment with an inorganic base such as K₂CO₃ in an appropriate alcoholic solvent such as MeOH, or by treatment with TBAF in THF, affording the compound of formula IV-1 wherein X^{a} represents ethynyl.

The compounds of formula I-3, I-4 and IV-3 are commercially available or can be prepared by standard methods known to one skilled in the art.

Particular embodiments of the invention are described in the following Examples, which serve to illustrate the invention in more detail without limiting its scope in any way.

### EXAMPLES

All temperatures are stated in °C. Unless otherwise indicated, the reactions take place at rt.

CCs were performed using Brunschwig 60A silica gel (0.032-0.63 mm) or using an ISCO CombiFlash system and prepacked SiO₂ cartridges, elution being carried out with either Hept-EA or DCM-MeOH mixtures with an appropriate gradient. When the compounds contained an acid function, 1% of AcOH was added to the eluent(s). When the compounds contained a basic function, 25% aq. NH₄OH was added to the eluents.

The compounds were characterized by ¹H NMR. Chemical shifts δ are given in ppm relative to the solvent used; multiplicities: s = singlet, d = doublet, t = triplet, q = quartet, p = pentet, hex = hexet, hep = Heptet, m = multiplet, br. = broad; coupling constants J are given in Hz.

The analytical LC-MS data have been obtained using the following respective conditions:
∘ Column: Zorbax SB-Aq, 30.5 µm, 4.6 x 50 mm;
∘ Injection volume: 1 µL;
∘ Column oven temperature: 40°C;
∘ Detection: UV 210 nm, ELSD and MS;
∘ MS ionization mode: ESI+;
∘ Eluents: A: H₂O + 0.04% TFA; and B: MeCN;
∘ Flow rate: 40.5 mL/min;
∘ Gradient: 5% B to 95% B (0.0 min -1.0 min), 95% B (1.0 min - 1.45 min).

The number of decimals given for the corresponding [M+H⁺] peak(s) of each tested compound depends upon the accuracy of the LC-MS device actually used.

The prep-HPLC purifications were performed on a Gilson HPLC system, equipped with a Gilson 215 autosampler, Gilson 333/334 pumps, Dionex MSQ Plus detector system, and a Dionex UVD340U (or Dionex DAD-3000) UV detector, using the following respective conditions:
- Method 1:
   ∘ Column: Waters Atlantis T3 OBD, 10 µm, 30 × 75 mm;
   ∘ Flow rate: 75 mL/min;
   ∘ Eluents: A: H₂O + 0.1% HCOOH; B: MeCN + 0.1% HCOOH;
   ∘ Gradient: 90% A to 5% A (0.0 min - 4.0 min), 5% A (4.0 min - 6.0 min).
- Method 2:
   ∘ Column: Waters XBridge C18, 10 µm, 30 × 75 mm;
   ∘ Flow rate: 75 mL/min;
   ∘ Eluents: A: H₂O + 0.1% HCOOH; B: MeCN + 0.1% HCOOH;
   ∘ Gradient: 70% A to 5% A (0.0 min - 30.5 min), 5% A (30.5 min - 6.0 min).
- Method 3:
   ∘ Column: Waters XBridge C18, 10 µm, 30×75 mm;
   ∘ Flow rate: 75 mL/min;
   ∘ Eluents: A: H₂O + 0.5% aq. NH₄OH 25% solution; B: MeCN;
   ∘ Gradient: 90% A to 5% A (0.0 min - 4.0 min), 5% A (4.0 min - 6.0 min).

Besides, semi-preparative chiral HPLCs were performed using the conditions herafter.

### Semi-preparative chiral HPLC Method A:

The semi-preparative chiral HPLC is performed on a Daicel ChiralPak IA column (20 x 250 mm, 5 µM) using the eluent mixture, flow rate and detection conditions indicated between brackets in the corresponding experimental protocol. The retention times are obtained by elution of analytical samples on a Daicel ChiralPak IA column (4.6 x 250 mm, 5 µM) using the same eluent mixture with the flow rate indicated between brackets in the corresponding experimental protocol.

### Semi-preparative chiral HPLC Method B:

The semi-preparative chiral HPLC is performed on a Daicel ChiralPak AY-H column (20 x 250 mm, 5 µM) using the eluent mixture, flow rate and detection conditions indicated between brackets in the corresponding experimental protocol. The retention times are obtained by elution of analytical samples on a Daicel ChiralPak AY-H column (4.6 x 250 mm, 5 µM) using the same eluent mixture with the flow rate indicated between brackets in the corresponding experimental protocol.

### PREPARATIONS:

### Preparation A: rac-4-(6-bromobenzo[d]thiazol-2-yl)-2-methyl-2-(methylsulfonyl)-N-(((RS)-tetrahydro-2H-pyran-2-yl)oxy)butanamide:

### A. i. 2-(6-bromobenzo[d]thiazol-2-yl)ethyl methanesulfonate:

To an ice-chilled solution of 2-(6-bromobenzo[*d*]thiazol-2-yl)ethanol (10.2 g, 39.5 mmol, prepared as described in US 2004/224953) in DCM (80 mL) was added dropwise TEA (11.7 mL, 84.2 mmol) and MsCl (5.64 mL, 72.5 mmol). The mixture was stirred at 0°C for 10 min. The mixture was diluted with a sat. NaHCO₃ solution (100 mL), extracted with DCM (100 mL) and the org. layer was washed with brine (100 mL), dried over MgSO₄ and concentrated to dryness to afford the title product as a yellow solid (12 g, 90% yield).
¹H NMR (*d6*-DMSO) δ: 8.39 (d, J = 1.8 Hz, 1H); 7.91 (d, J = 8.7 Hz, 1H); 7.66 (dd, J = 1.8, 8.7 Hz, 1H); 4.66 (t, J = 6.1 Hz, 3H); 3.57 (t, J = 6.1 Hz, 3H).
MS (ESI, m/z): 335.9 [M+H⁺] for C₁₀H₁₀NO₃BrS₂; t_{R} = 0.82 min.

### A.ii. Rac-ethyl 4-(6-bromobenzo[d]thiazol-2-yl)-2-methyl-2-(methylsulfonyl)butanoate:

To a solution of ethyl 2-(methylsulfonyl)propanoate (4.3 g, 23.7 mmol, commercial) in DMF (26 mL) was added portionwise NaH (0.9 g, 22.5 mmol). The mixture was stirred at 0°C for 15 min and was allowed to reach 10°C. Then, a solution of intermediate A.i (7.58 g, 22.5 mmol) in DMF (26 mL) was added dropwise. The mixture was stirred at 10°C for 30 min. EA (100 mL) was added and the mixture was poured into 10% aq. NaHSO₄ (100 mL). The org. layer was then washed with water (100 mL), brine (100 mL), dried over MgSO₄ and concentrated to dryness. The residue was purified by CC (Hept-EA) to afford the title compound as a pale yellow solid (5.46 g, 58% yield).
¹H NMR (*d6*-DMSO) δ: 8.38 (d, J = 2.0 Hz, 1H); 7.89 (d, J = 8.7 Hz, 1H); 7.65 (dd, J = 2.0, 8.6 Hz, 1H); 4.18 (q, J = 7.1 Hz, 2H); 3.28-3.33 (overlapped m, 1H); 3.15 (s, 3H); 3.07-3.11 (m, 1H); 2.66-2.75 (m, 1H); 2.31-2.40 (m, 1H); 1.60 (s, 3H); 1.21 (t, J = 7.1 Hz, 3H).
MS (ESI, m/z): 422.0 [M+H⁺] for C₁₅H₁₈NO₄BrS₂₂; t_{R} = 0.89 min.

### A.iii. Rac-4-(6-bromobenzo[d]thiazol-2-yl)-2-methyl-2-(methylsulfonyl)butanoic acid lithium salt:

To a solution of intermediate A.ii (6.93 g, 16.4 mmol) in MeOH (34 mL) and THF (34 mL) was added a solution of LiOH.H₂O (1.461 g, 34.8 mmol) in water (17 mL). The mixture was stirred at 50°C for 1 h. The mixture was concentrated to dryness and dried to a constant weight to afford the title product as a yellow foam (8.28 g, quant.).
¹H NMR (*d6*-DMSO) δ: 8.34 (d, J = 2 Hz, 1H); 7.87 (d, J = 8.7 Hz, 1H); 7.62 (dd, J = 2.0, 8.7 Hz, 1H); 3.13-3.20 (m, 2H); 3.08 (s, 3H); 2.50-2.58 (m, 1H); 2.06-2.18 (m, 1H); 1.40 (s, 3H).
MS (ESI, m/z): 391.9 [M+H⁺] for C₁₃H₁₅NO₄BrS₂; t_{R} = 0.76 min.

### A. iv. Rac-4-(6-bromobenzo[d]thiazol-2-yl)-2-methyl-2-(methylsulfony)-N-(((RS)-tetrahydro-2H-pyran-2-yl)oxy)butanamide:

To a solution of intermediate A.iii (6.83 g, 17.4 mmol) in DMF (68 mL) was added *O*-(tetrahydro-*2H*-pyran-2-yl)hydroxylamine (6.12 g, 52.2 mmol), EDC (10.02 g, 52.2 mmol), HOBT.H₂O (7.05 g, 52.2 mmol) and TEA (7.39 mL, 53.1 mmol). It was stirred at 30°C overnight. The mixture was concentrated to dryness and diluted in EA. The org. phase was washed with aq. sat. NaHCO₃ and brine, dried over MgSO₄ and concentrated to dryness. The residue was purified by CC (Hept-EA) to afford the title compound as a yellow foam (5.92 g, 69% yield).
¹H NMR (*d6*-DMSO) δ: 11.39 (s, 1H); 8.37 (d, J = 1.7 Hz, 1H); 7.89 (d, J = 8.7 Hz, 1H); 7.65 (dd, J = 2.0, 8.6 Hz, 1H); 4.96 (d, J = 2.0 Hz, 1H); 3.98-4.11 (m, 2H); 3.45-3.54 (m, 1H); 3.07 (s, 1.5H); 3.05 (s, 1.5H); 2.91-3.04 (overlapped m, 1H); 2.68-2.84 (m, 1H); 2.19-2.33 (m, 1H); 1.47-1.65 (m, 9H).
MS (ESI, m/z): 491.4 [M+H⁺] for C₁₈H₂₃N₂O₅BrS₂; t_{R} = 0.84 min.

### Preparation B: rac-tert-butyl 2-(methylsulfonyl)propanoate:

To a suspension of sodium methanesulfinate (6.20 g, 57.7 mmol) in *tert*-butanol (50 mL) was added at rt and in one portion tert-butyl 2-bromopropionate (8.6 mL, 52 mmol). The mixture was refluxed overnight. The mixture was cooled down at rt and the solvent was removed under reduced pressure. The residue was taken up in EA (200 mL), filtered through Celite and the pad was rinsed with EA (200 mL). The filtrate was concentrated to dryness to afford the title product as a white solid (9.91 g, 92% yield).
¹H NMR (*d6*-DMSO) δ: 4.24 (q, J = 7.2 Hz, 1H); 3.11 (s, 3H); 1.45 (s, 9H); 1.40 (d, J = 7.2 Hz, 3H).

### Preparation C: rac-tert-butyl 4-(6-ethynylbenzo[d]thiazol-2-yl)-2-methyl-2-(methylsulfonyl)butanoate:

### C.i. Tert-butyl 4-(6-bromobenzo[d]thiazol-2-yl)-2-methyl-2-(methylsulfonyl)butanoate:

To a solution of the compound of Preparation B (2.07 g, 9.94 mmol) in DMF (11 mL) was added at 0°C and portionwise NaH (0.320 g, 2.75 mmol). The mixture was stirred at 0°C for 15 min and allowed to reach 10°C. A solution of intermediate A.i. (0.980 g, 2.91 mmol) in DMF (4 mL) was added dropwise. The mixture was stirred at rt for 10 min. The DMF was removed in vacuo and the residue was taken in water (20 mL) and EA (20 mL). The org. layer was dried over MgSO₄ and concentrated to dryness. The residue was purified by CC (Hept-EA) to afford the title product as a yellow oil (2 g, quant.).
¹H NMR (*d6*-DMSO) δ: 8.39 (d, J = 1.9 Hz, 1H); 7.91 (d, J = 8.7 Hz, 1H); 7.66 (dd, J = 1.9, 8.7 Hz, 1H); 3.29-3.39 (overlapped m, 1H); 3.15 (s, 3 H); 2.96-3.07 (m, 1H); 2.62-2.73 (m, 1H); 2.29-2.40 (m, 1H); 1.56 (s, 3H); 1.45 (s, 9H).
MS (ESI, m/z): 450.0 [M+H⁺] for C₁₇H₂₂NO₄BrS₂; t_{R} = 0.96 min.

### C.ii. Tert-butyl (RS)-4-(6-ethynylbenzo[d]thiazol-2-yl)-2-methyl-2-(methylsulfonyl)butanoate:

A mixture of intermediate C.i (0.860 g, 2.19 mmol), ethynyltributylstannane (0.960 g, 3.06 mmol) and Pd(PPh₃)₄ (0.845 g, 0.0732 mmol) in degassed THF (14 mL) was stirred at rt for 8 h under a nitrogen atmosphere. The mixture was then concentrated to dryness and purified by CC (Hept-EA) to afford the title compound as an orange solid (0.205 g, 24% yield).
¹H NMR (*d6*-DMSO) δ: 8.26 (d, J = 1.7 Hz, 1H); 7.94 (d, J = 8.5 Hz, 1H); 7.57 (dd, J = 1.7, 8.5 Hz, 1H); 4.26 (s, 1H); 3.30-3.40 (overlapped m, 1H); 3.13 (s, 3H); 2.92-3.07 (m, 1H); 2.56-2.73 (m, 1H); 2.24-2.40 (m, 1H); 1.56 (s, 3H); 1.45 (s, 9H).
MS (ESI, m/z): 394.1 [M+H⁺] for C₁₉H₂₃NO₄S₂; t_{R} = 0.93 min.

### Preparation D: 3-(4-iodophenyl)oxetan-3-amine hydrochloride:

### D.i. N-(3-(4-iodophenyl)oxetan-3-yl)-2-methylpropane-2-sulfinamide:

*n*-BuLi (1.1*M* in hexanes, 11.4 mL) was added dropwise to a solution of 1,4-iodobenzene (4.36 g) in THF (50 mL) at -78°C. After stirring for 1 h, a solution of 2-methyl-*N*-oxetan-3-ylidenepropane-2-sulfinamide (1.64 g; commercial) in THF (10 mL) was added dropwise over the course of 30 min at -78°C. The reaction mixture was gradually warmed to rt. After 1 h, sat. NH₄Cl was added and the aq. layer was extracted with EA. The combined org. layer was washed with aq. sat. NaHCO₃ and brine, dried over Na₂SO₄, filtered and concentrated to dryness. The residue was purified by CC (EA-Hept) to give the title compound as a colourless oil (0.751 g, 21% yield).
¹H NMR (*d6*-DMSO) δ: 7.77 (d, J = 8.4 Hz, 2H); 7.30 (d, J = 8.4 Hz, 2H); 6.35 (s, 1H); 4.98 (d, J = 6.3 Hz, 1H); 4.90-4.94 (m, 1H); 4.85-4.88 (m, 1H); 4.67 (d, J = 6.3 Hz, 1H); 1.11 (s, 9H).
MS (ESI, m/z): 379.97 [M+H⁺] for C₁₃H₁₈NO₂IS ; t_{R} = 0.78 min.

### D.ii. 3-(4-iodophenyl)oxetan-3-amine hydrochloride:

To a solution of intermediate D.i. (0.751 g, 1.98 mmol) in DCM (20 mL) was added a 4*M* solution of HCl in dioxane (1.06 mL). After stirring for 30 min at rt, the solids were filtered off and washed with Hex (3 mL) to afford the title compound as a white solid (0.624 g, 100% yield).
¹H NMR (*d6*-DMSO) δ: 9.14-9.30 (m, 3H); 7.82-7.90 (m, 2H); 7.34-7.40 (d, J = 8.5 Hz, 2H); 4.80-5.00 (m, 4H).
MS (ESI, m/z): 299.89 [M+Na⁺] for C₉H₁₀NOI; t_{R} = 0.50 min.

### Preparation E: rac-4-(6-ethynylbenzo[d]thiazol-2-yl)-2-methyl-2-(methylsulfonyl)-N-(((RS)-tetrahydro-2H-pyran-2-yl)oxy)butanamide:

To a mixture of the compound of Preparation A (2 g, 4.07 mmol), cesium fluoride (1.233 g, 8.14 mmol) and bis(tri-*tert*-butylphosphine)palladium (0.152 g, 0.297 mmol) in degassed dioxane (20 mL) was added ethynyltributylstannane (1.77 mL, 6.1 mmol). The mixture was stirred at 80°C for 10 min under a nitrogen atmosphere. The mixture was diluted with DCM (100 mL) and aq. sat. NaHCO₃ (100 mL). The org. layer was dried over MgSO₄ and concentrated to dryness. The crude residue was purified by CC (Hept-EA) to afford the title compound as a yellow foam (1.33 g, 75% yield).
¹H NMR (*d6*-DMSO) δ: 11.41-11.45 (m, 1H); 8.26-8.28 (m, 1H); 7.94 (d, J = 8.4 Hz, 1H); 7.57 (dd, J = 1.5, 8.4 Hz, 1H); 4.94-5.00 (m, 1H); 4.28 (s, 1H); 4.06-4.15 (m, 1H); 3.47-3.55 (m, 1H); 3.22-3.31 (overlapped m, 1H); 3.08 (s, 1.5H); 3.06 (s, 1.5H); 2.96-3.05 (m, 1H), 2.72-2.84 (m, 1H); 2.21-2.33 (m, 1H); 1.47-1.77 (m, 9H).
MS (ESI, m/z): 437.2 [M+H⁺] for C₂₀H₂₄N₂O₅S₂; t_{R} = 0.82 min.

### Preparation F: 3-(iodoethynyl)oxetan-3-ol:

To a solution of 3-ethynyloxetan-3-ol (1.097 g; 11.2 mmol; commercial) in MeOH (50 mL) and 1*M* aq. KOH (28 mL) was added iodine (3.549 g; 14 mmol). The reaction mixture was stirred for 2 h at rt. Water (150 mL) and DCM (500 mL) were added. The aq. layer was extracted with EA (500 mL). The org. layer were washed with brine, dried over MgSO₄, filtered and concentrated down to afford the desired compound as a light yellow solid (2.21 g, 88% yield).
¹H NMR (*d6*-DMSO) δ: 4.60 (d, J = 6.5 Hz, 2H); 4.45 (d, J = 6.5 Hz, 2H).

### Preparation G: (2R)-4-(6-bromobenzo[d]thiazol-2-yl)-2-methyl-2-(methylsulfonyl)-N-(((RS)-tetrahydro-2H-pyran-2-yl)oxy)butanamide:

### G.i. (R)-ethyl 4-(6-bromobenzo[d]thiazol-2-yl)-2-methyl-2-(methylsulfonyl)butanoate:

Intermediate A.ii (8.42 g) was separated by semi-preparative chiral HPLC Method A (MeOH-DEA-DCM 74.92-0.08-25; flow rate: 16 mL/min; UV detection at 227 nM); the respective retention times (flow rate: 0.8 mL/min) were 5.45 and 6.17 min. The title (R)-enantiomer was identified as the second-eluting enantiomer and was obtained as a yellow solid (4 g).
¹H NMR (*d6*-DMSO) δ: 8.38 (d, J = 2.0 Hz, 1H); 7.89 (d, J = 8.7 Hz, 1H); 7.65 (dd, J = 2.0, 8.6 Hz, 1H); 4.18 (q, J = 7.1 Hz, 2H); 3.28-3.33 (overlapped m, 1H); 3.15 (s, 3H); 3.07-3.11 (m, 1H); 2.66-2.75 (m, 1H); 2.31-2.40 (m, 1H); 1.60 (s, 3H); 1.21 (t, J = 7.1 Hz, 3H).
MS (ESI, m/z): 419.8 [M+H⁺] for C₁₅H₁₈NO₄BrS₂; t_{R} = 0.90 min.

### G.ii. (2R)-4-(6-bromobenzo[d]thiazol-2-yl)-2-methyl-2-(methylsulfonyl)-N-(((RS)-tetrahydro-2H-pyran-2-yl)oxy)butanamide:

Starting from the intermediate G.i (3.98 g, 9.45 mmol) and proceeding in analogy to Preparation A, steps A.iii-A.iv (yields: saponification 100%; amide coupling with THP-O-NH₂ 75%), the title product was obtained, after purification by CC (Hept-EA), as a pale yellow foam (3.69 g, 75% yield).
¹H NMR (*d6*-DMSO) δ: 11.37 (s, 1H); 8.34 (s, 1H); 7.89 (d, J = 8.7 Hz, 1H); 7.65 (dd, J = 2.0, 8.6 Hz, 1H); 4.96 (d, J = 2.0 Hz, 1H); 3.98-4.11 (m, 2H); 3.45-3.54 (m, 1H); 3.07 (s, 1.5H); 3.05 (s, 1.5H); 2.91-3.04 (overlapped m, 1H); 2.68-2.84 (m, 1H); 2.19-2.33 (m, 1H); 1.47-1.65 (m, 9H).
MS (ESI, m/z): 491.4 [M+H⁺] for C₁₈H₂₃N₂O₅BrS₂; t_{R}= 0.84 min.

### Preparation H: (2R)-4-(6-ethynylbenzo[d]thiazol-2-yl)-2-methyl-2-(methylsulfonyl)-N-(((RS)-tetrahydro-2H-pyran-2-yl)oxy)butanamide:

Starting from intermediate G.ii (1.5 g, 3.05 mmol) and proceeding in analogy to Preparation F, the title product was obtained, after purification by CC (Hept-EA), as an orange foam (1 g, 75% yield).
¹H NMR (*d6*-DMSO) δ: 11.40 (s, 1H); 8.26 (s, 1H); 7.94 (d, J = 8.4 Hz, 1H); 7.57 (dd, J = 1.5, 8.4 Hz, 1H); 4.96 (s, 1H); 4.26 (s, 1H); 3.98-4.15 (m, 2H); 3.44-3.53 (m, 1H); 3.20-3.29 (overlapped m, 1H); 3.07 (s, 1.5H); 3.05 (s, 1.5H); 2.70-2.88 (m, 1H); 2.19-2.32 (m, 1H); 1.47-1.77 (m, 9H).
MS (ESI, m/z): 436.9 [M+H⁺] for C₂₀H₂₄N₂O₅S₂ ; t_{R} = 0.82 min.

### Preparation I: 3-(4-iodophenyl)oxetan-3-ol:

A solution of 1,4-diiodobenzene (0.8 g, 2.43 mmol) in THF (8 mL) was treated at -78°C with *n*-BuLi (1.68M in Hex; 2.23 mL). After stirring at this temperature for 30 min, the solution was treated with a suspension of 3-oxetanone (0.24 g, 3.34 mmol) in THF (3 mL). The reaction mixture was allowed to reach rt and was further stirred overnight. The reaction mixture was treated with 10% aq. NaHSO₄ solution (4 mL) and diluted with water (10 mL) and EA (10 mL). The aq. layer was extracted with EA. The combined org. layers were washed with brine, dried over MgSO₄, filtered and concentrated under reduced pressure. The residue was purified by CC (Hept-EA) to afford the title alcohol as a colourless solid (0.2 g; 55% yield).
¹H NMR (*d6*-DMSO) δ: 7.73 (d, J = 8.5 Hz, 2H); 7.39 (d, J = 8.5 Hz, 2H); 6.39 (s, 1H); 4.73 (d, J = 6.8 Hz, 2H); 4.60 (d, J = 6.8 Hz, 2H).

### Preparation J: 4-iodo-2-methylbut-3-yn-2-ol:

To a solution of 2-methyl-3-butyl-2-ol (4 g, 19 mmol) in MeOH (144 mL) were added KI (3.48 g, 21 mmol) and *tert*-butylhydroperoxide (70% in water, 2.74 mL, 28.6 mmol). The mixture was stirred overnight at rt. The mixture was quenched with sat. aq. Na₂S₂O₃ and extracted twice with EA (45 mL). The combined org. layers were washed with brine, dried over MgSO₄ and concentrated to dryness. The crude residue was purified by CC (Hept-EA) to afford the title compound as a colourless oil (1.60 g, 40% yield).
¹H NMR (*d6*-DMSO): 5.35 (s, 1H); 1.31 (s, 6H).

### Preparation K: (S)-4-iodobut-3-yne-1,2-diol:

### K.i. (S)-4-(2,2-dibromovinyl)-2,2-dimethyl-1,3-dioxolane:

To an ice-chilled solution of PPh₃ (63.5 g, 242 mmol) in DCM (130 mL) was added dropwise a solution of CBr₄ (40.3 g, 121 mmol) in DCM (50 mL) while maintaining the internal temperature below 15°C. The mixture was cooled to 0°C and solution of (*R*)-2,2-dimethyl-1,3-dioxolane-4-carbaldehyde (12.2 g, 93.4 mmol) and TEA (13 mL) in DCM (10 mL) was added dropwise. The mixture was stirred at 0°C for 30 min. The mixture was then allowed to warm at rt and poured into PE (120 mL). The mixture was filtered through Celite and the solid was washed with Et₂O (3 x 30 mL). The filtrate was concentrated to dryness and the residue was triturated in PE. The mixture was filtered, the filtrate was concentrated to dryness to afford the title compound as a pale yellow oil (18.02 g, 68% yield).
¹H NMR (*d6*-DMSO) δ: 1.30 (s, 3H); 1.34 (s, 3H); 3.70 (t, J = 7.1 Hz, 1H); 4.14 (t, J = 7.1 Hz, 1H); 4.61 (dd, J = 6.6, 13.3 Hz, 1H); 6.70 (d, J = 7.9 Hz, 1H).

### K. ii. (S)-4-(iodoethynyl)-2,2-dimethyl-1,3-dioxolane:

To a solution of intermediate K.i (5 g, 17.5 mmol) in THF (75 mL) cooled to -78°C was added dropwise *n*-BuLi (1.99 M in Hept; 17.5 mL, 35 mmol). The mixture was stirred 2 h at -78°C and then warmed to 0°C. A solution of iodine (7.1 g, 28 mmol) in THF (50 mL) was added dropwise. The mixture was stirred at rt overnight. The reaction was quenched with sat. Na₂S₂O₃ (75 mL) and the aq. layer was extracted twice with DCM (2 x 250 mL). The combined org. layers were dried over MgSO₄ and concentrated to dryness. The crude residue was purified by CC (Hept-EA) to afford the title compound as a pale yellow oil (0.747 g, 17% yield).
¹H NMR (CDCl₃) δ: 4.82 (t, J = 6.2 Hz, 1H); 4.13 (dd, J = 6.4, 7.9 Hz, 1H); 3.94 (dd, J = 6.4, 7.9 Hz, 1H); 1.48 (s, 3H); 1.36 (s, 3H).

### K.iii. (S)-4-iodobut-3-yne-1,2-diol:

To a solution of intermediate K.ii (747 mg, 2.96 mmol) in water (1.25 mL) was added TFA (2.5 mL, 32.7 mmol). The mixture was stirred at rt for 2 h. The mixture was concentrated to dryness and the residue was diluted in sat. NaHCO₃ (20 mL). The aq. layer was extracted with DCM-MeOH (9-1, 3x20 mL) and the combined org. layers were dried over MgSO₄ and concentrated to dryness to afford the title compound as a white solid (366 mg, 60% yield).
¹H NMR (CDCl₃) δ: 4.61 (dd, J = 3.8, 6.2 Hz, 1H); 3.70-3.80 (m, 2H).

### Preparation L: 4-iodo-2-methylbut-3-yn-2-amine:

To a solution of 2-methylbut-3-yn-2-amine (0.633 mL, 6 mmol) in MeOH (30 mL) and KOH (1M; 15 mL, 15 mmol) was added iodine (1.9 g, 7.52 mmol). The mixture was stirred at rt for 2 h. Water (100 mL) and DCM (120 mL) were added. The aq. layer was extracted with DCM (120 mL). The combined org. layers were washed with brine, dried over MgSO₄, filtered and concentrated down to afford the desired compound as a yellow solid (0.985 g, 78% yield).
¹H NMR (*d6*-DMSO) δ: 2.01 (s, 2H); 1.24 (s, 6H).
MS (ESI, m/z*):* 210.01 [M+H⁺] for C₅H₈NI; t_{R} = 0.33 min.

### Preparation M: ((1S,2S)-2-(bromoethynyl)cyclopropyl)methyl acetate AND ((1R,2R)-2-(bromoethynyl)cyclopropyl)methyl acetate:

### M.i. ((1S*,2S*)-2-(2,2-dibromovinyl)cyclopropyl)methyl acetate:

To a solution of CBr₄ (36.56 g; 108 mmol) in DCM (76 mL) cooled to -20°C, was added dropwise over 1 h a solution of PPh₃ (55.39 g, 211 mmol) in DCM (127 mL). The mixture was kept stirred at this temperature for 45 min and then cooled to -78°C. A solution of ((*1S*,2S**)-2-formylcyclopropyl)methyl acetate (7.54 g, 53 mmol, prepared as described in WO 2012/154204) in DCM (100 mL) was added dropwise over 1.5 h, keeping the internal temperature below -70°C. The mixture was stirred at this temperature for 30 min and allowed to warm to rt over 30 min. The solvent was removed in vacuo and the residue was purified by CC (EA-Hept) to afford the title acetate as a colourless oil (7.98 g, 50% yield).
¹H NMR (CDCl₃) δ: 5.84 (d, *J* = 9.0 Hz, 1H); 3.97 (m, 2H); 2.07 (s, 3H); 1.61 (m, 1H); 1.33 (m, 1H); 0.78-0.92 (m, 2H).
MS (ESI, m/z): 295.0 [M+H⁺] for C₈H₁₀O₂Br₂ ; t_{R} = 0.87 min.

### M.ii. ((1S,2S)-2-(bromoethynyl)cyclopropyl)methyl acetate AND ((1R,2R)-2-(bromoethynyl)cyclopropyl)methyl acetate:

To a solution of intermediate M.i (7.98 g, 26.8 mmol) in THF (160 mL) was added TBAF trihydrate (48 g, 151 mmol). The reaction mixture was heated at 60°C for 3 h. The reaction mixture was cooled to rt and diluted with diethyl ether (150 mL). The org. phase was washed with water (60 mL) and brine (60 mL), dried over MgSO₄ and concentrated to dryness. The residue was purified by CC (EA-Hept) and by prep-HPLC (Method 1) to afford the title compound as a colourless oil (2.94 g, 51% yield). The racemic product was separated by semi-preparative chiral HPLC Method B (Hept-EtOH 9-1; flow rate: 16 mL/min, UV detection at 220 nm), the respective retention times (flow rate: 0.8 mL/min) were 5.9 and 8.7 min. The title enantiomers were obtained as colourless oils (1.4 g each).

### First-eluting enantiomer, (1S,2S)-configurated:

¹H NMR (CDCl₃) δ: 3.97 (dd, J = 6.5, 11.7 Hz, 1H); 3.84 (dd, J = 7.5, 11.7 Hz, 1H); 2.06 (s, 3H); 1.50 (m, 1H); 1.25 (m, 1H); 0.97 (m, 1H); 0.76 (m, 1H).
[α]_{D} = +96° (c = 1.03; MeOH).

### Second-eluting enantiomer, (1R,2R)-configurated:

¹H NMR (CDCl₃) δ: 3.97 (dd, J = 6.5, 11.7 Hz, 1H); 3.84 (dd, J = 7.5, 11.7 Hz, 1H); 2.06 (s, 3H); 1.50 (m, 1H); 1.25 (m, 1H); 0.97 (m, 1H); 0.76 (m, 1H).
[α]_{D} = -94° (c = 1.01; MeOH).
The respective absolute configurations of these compounds have been determined though transformation of the second-eluting enantiomer into the corresponding (*S*) and (*R*) α-methoxy-α-trifluoromethylphenylacetyl esters and the subsequent analysis of their NMR spectra as described by Kobayashi et al. in Chem. Pharm. Bull. (2003), 51, 448.

### Preparation N: ((1-(bromoethynyl)cyclopropyl)methoxy)(tert-butyl)diphenylsilane:

To a mixture of (dibromomethyl)triphenylphosphonium bromide (8.527 g; 16.6 mmol; commercial) and THF (40 mL) was added a solution of *t*BuOK (1*M* in THF) (16.6 mL, 16.6 mmol). The resulting dark brown solution was stirred for 3 min at rt, then cooled to 0°C. A solution of 1-(((*tert*-butyldiphenylsilyl)oxy)methyl)cyclopropanecarbaldehyde (2.2 g; 6.62 mmol; prepared as described in WO 2010/135536) in THF (23 mL) was added dropwise. The reaction was stirred at 0°C for 40 min. The reaction mixture was cooled to -78°C and tBuOK (1M in THF, 29.1 mL, 29.1 mmol) was added rapidly and stirred at -78°C for 30 min. The reaction mixture was quenched with brine (150 mL). The aq. layer was separated and extracted with Et₂O (3 x 150 mL). The combined org. phases were washed with brine, dried over MgSO₄, filtered, and concentrated to dryness. The residue was purified by CC (Hept-EA) to afford the title compound as a colourless oil (2.052 g, 75% yield).
¹H NMR (*d6*-DMSO) δ: 7.60-7.66 (m, 4H); 7.42-7.48 (m, 6H); 3.57 (s, 2H); 1.02 (s, 9H); 0.84-0.88 (m, 2H); 0.72-0.76 (m, 2H).

### Preparation O: tert-butyl (3-(iodoethynyl)oxetan-3-yl)carbamate:

### O.i. Tert-butyl (3-((trimethylsilyl)ethynyl)oxetan-3-yl)carbamate:

To a solution of 3-((trimethylsilyl)ethynyl)oxetan-3-amine hydrochloride (0.123 g; 0.6 mmol; commercial) in DCM (3 mL) were added TEA (0.18 mL; 1.29 mmol) and Boc₂O (0.272 g; 1.25 mmol). The reaction mixture was stirred at rt for 6 h. Boc₂O (0.272 g; 1.25 mmol) was added again and the reaction was stirred overnight. The reaction mixture was diluted with DCM (5 mL) and sat. aq. NaHCO₃ (5 mL) was added. The phases were separated and the aq. layer was extracted twice with DCM (2 x 5 mL). The combined org. layers were washed with brine (5 mL), dried over MgSO₄, filtered and the filtrate concentrated to dryness to afford the title compound, slightly contaminated by Boc₂O, as a white gum (0.312 g).
¹H NMR (CDCl₃) *δ*: 4.72-4.81 (m, 4H); 3.05 (br. s, 1H); 1.47 (s, 9H); 0.18 (s, 9H).

### O.ii. Tert-butyl (3-ethynyloxetan-3-yl)carbamate:

To a solution of intermediate O.i (0.211 g; 0.783 mmol) in MeOH (1.6 mL) was added K₂CO₃ (0.162 g; 1.17 mmol). The mixture was stirred at rt for 30 min. Water (5 mL) was added. The mixture was extracted twice with DCM (2 x 10 mL) and the org. layer was dried over MgSO₄, filtered and the filtrate concentrated to dryness. The crude was purified by CC (PE-EA) to afford the title compound as a white solid (0.173 g).
¹H NMR (CDCl₃) *δ*: 5.02 (br. s, 1H); 4.84 (d, J = 6.2 Hz, 2H); 4.73 (d, J = 6.2 Hz, 2H); 2.57 (s, 1H); 1.47 (s, 9H).

### O.iii. Tert-butyl (3-(iodoethynyl)oxetan-3-yl)carbamate:

To a solution of intermediate O.ii (0.154 g; 0.783 mmol) in THF (2.4 mL) cooled to -78°C was added, dropwise over 15 min, *n*-BuLi (2.11M in hexanes; 0.74 mL; 1.56 mmol), keeping the internal temperature below -70°C. After stirring for 1 h, a solution of iodine (0.201 g; 0.79 mmol) in THF (1.2 mL) was added dropwise over 5 min. The reaction mixture was stirred at -78°C for 1.5 h, then was allowed to warm at rt for 30 min and stirred at rt for 30 min. The reaction mixture was quenched with a sat. Na₂S₂O₃ solution (5 mL). The aq. layer was extracted with Et₂O (2 x 10 mL). The combined org. layers were dried over MgSO₄, filtered and concentrated to dryness to give the desired compound as a yellow oil (0.234 g, 92% yield).
¹H NMR (CDCl₃) *δ*: 5.02 (br. s, 1H); 4.81-4.85 (m, 2H); 4.70-4.75 (m, 2H); 1.47 (s, 9H).

### Preparation P: 1-(iodoethynyl)cyclopropan-1-amine hydrochloride:

### P.i. Tert-butyl (1-(iodoethynyl)cyclopropyl)carbamate:

Starting from tert-butyl (1-ethynylcyclopropyl)carbamate (0.885 g, 4.88 mmol; commercially available) and proceeding in analogy to Preparation L (iodination), the title compound was obtained as a crude light yellow solid (1.36 g, 91% yield).
¹H NMR (CDCl₃) δ: 5.00 (br. s, 1H); 1.49 (s, 9H); 1.23 (s, 2H); 1.11 (s, 2H).

### P.ii. 1-(iodoethynyl)cyclopropan-1-amine hydrochloride:

Starting from intermediate P.i (0.600 g, 1.95 mmol) and proceeding in analogy to Preparation D, step D.ii, the title compound was obtained, after trituration in Et₂O, as a beige solid (0.358 g, 75% yield).
¹H NMR (*d6*-DMSO)) δ: 8.78 (br. s, 3H); 1.23-1.29 (m, 2H); 1.16-1.22 (m, 2H).

### Preparation Q: (1-(4-iodophenyl)cyclopropyl)methanol:

A sealed tube was charged with NaI (0.83 g, 5.49 mmol), CuI (0.1 g, 0.55 mmol) and 1-(4-bromophenyl)cyclopropyl)methanol (0.62 g, 2.74 mmol). 1,4-Dioxane (3 mL) and *trans-N-N'*-dimethylcyclohexa-1,2-diamine (0.17 mL, 1.1 mmol) were added. The reaction mixure was heated at 180°C for 40 min under microwave irradiation. The mixture was filtered over Celite. Solids were washed with EA and the filtrate was concentrated to dryness. The residue was purified by CC (Hept-EA) to obtain the title product as a white solid (0.654 g, 87% yield).
¹H NMR (CDCl₃) δ: 7.61 (d, J = 8.5 Hz, 1H), 7.11 (d, J = 8.5 Hz, 1H), 4.70 (t, J = 5.7 Hz, 1H), 3.50 (d, J = 5.7 Hz, 1H), 0.70-0.73 (m, 1H), 0.81-0.84 (m, 1H).

### Preparation R: ((1-(bromoethynyl)cyclobutyl)methoxy)(tert-butyl)diphenylsilane:

### R.i. (1-(((tert-butyldiphenylsilyl)oxy)methyl)cyclobutyl)methanol:

To a solution of cyclobutane-1,1-diyldimethanol (3.03 g, 24.8 mmol; commercial) in THF (190 mL) was added portionwise at 0°C NaH (60% in dispersion, 0.99 g, 24.8 mmol). The resulting suspension was stirred at rt for 1 h and TBDPS-Cl (6.47 mL, 24.8 mmol) was added dropwise for 40 min. The reaction mixture was stirred at rt overnight. The mixture was diluted with sat. aq. NH₄Cl (100 mL) and extracted with EA (3 x 80 mL). The combined org. layers were dried over Na₂SO₄ and the filtrate was concentrated to dryness. The residue was purified by CC (Hept-EA) to afford the title compound as a colourless oil (8.61 g, 98% yield).
¹H NMR (CDCl₃) δ: 7.69-7.72 (m, 4H), 7.41-7.49 (m, 6H), 3.76 (d, J = 5.7 Hz, 2H), 3.74 (s, 2H), 2.51 (t, J = 5.7 Hz, 1H), 1.89-1.95 (m, 1H), 1.80-1.87 (m, 3H), 1.73-1.77 (m, 2H), 1.08 (s, 9H).
MS (ESI, m/z): 355.0 [M+H⁺] for C₈H₁₀O₂Br₂; t_{R} = 1.07 min.

### R.ii. 1-(((tert-butyldiphenylsilyl)oxy)methyl)cyclobutane-1-carbaldehyde:

To a suspension of intermediate R.i (5 g, 14.1 mmol) in DCM (34 mL) cooled to 0°C was added dropwise DIPEA (7.24 mL, 42.3 mmol). A solution of sulfur trioxide pyridine complex (2.69 g, 7.61 mmol) in DMSO (17 mL) was added dropwise. The reaction mixture was stirred for 2 h with gradual warming to rt. Sat. aq. NaHCO₃ (180 mL) and DCM (100 mL) were added. The two layers were separated and the aq. layer was extracted once with DCM (100 mL). The org. layer was washed with brine (80 mL), dried over Na₂SO₄, filtered and concentrated to dryness. The residue was purified by CC (Hept-EA gradient) to afford the title ketone as a colourless oil (4.28 g, 86% yield).
¹H NMR (CDCl₃) δ: 9.73 (s, 1H), 7.65-7.69 (m, 4H), 7.40-7.49 (m, 6H), 3.91 (s, 2H), 2.25-2.34 (m, 2H), 1.85-1.94 (m, 4H), 1.06 (s, 9H).

### R.iii. Tert-butyl((1-(2,2-dibromovinyl)cyclobutyl)methoxy)diphenylsilane:

Starting from intermediate R.ii (8.25 g, 24.6 mmol) and proceeding in analogy to Preparation M, step M.i., the title compound was obtained as a colourless oil (5.73 g, 93% yield).
¹H NMR (CDCl₃) δ: 7.69-7.72 (m, 4H), 7.41-7.48 (m, 6H), 6.68 (s, 1H), 3.79 (s, 2H), 2.15-2.29 (m, 4H), 1.79-1.96 (m, 2H), 1.09 (s, 9H).

### R.iv. ((1-(bromoethynyl)cyclobutyl)methoxy)(tert-butyl)diphenylsilane:

A solution of intermediate R.iii (5.72 g, 11.3 mmol) in dry THF (26 mL) cooled to -78°C was treated with a solution of tBuOK (1M in THF, 49.5 mL) over 45 min. The reaction mixture was stirred for 30 min at -78°C then was diluted with brine (80 mL) and was allowed to reach rt. Et₂O (150 mL) was added. The aq. layer was separated and extracted once again with Et₂O (150 mL). The combined org. layers were washed with brine (80 mL), dried over Na₂SO₄, filtered and concentrated to afford the title compound as a colourless oil (4.79 g, quant.).
¹H NMR (CDCl₃) δ: 7.70-7.74 (m, 4H), 7.40-7.48 (m, 6H), 3.67 (s, 2H), 2.18-2.29 (m, 4H), 2.00-2.08 (m, 1H), 1.86-1.95 (m, 1H), 1.11 (s, 9H).

### Preparation S: (1R,2S,4s)-4-(bromoethynyl)cyclopentane-1,2-diol:

### S.i. (3aR, 5S, 6aS)-5-(bromoethynyl)-2,2-dimethyltetrahydro-4H-cyclopenta[d][1,3]dioxole:

Starting from (*3aR*,*5S*,*6aS*)-5-(2,2-dibromovinyl)-2,2-dimethyltetrahydro-4*H*-cyclopenta[*d*][1,3]dioxole (2.06 g; 6.32 mmol; prepared as described in WO 2013/170030) and proceeding in analogy to Preparation R, step R.iv, the title compound was obtained as a yellow oil (1.37 g, 88% yield).
¹H NMR (CDCl₃) δ: 4.60-4.63 (m, 2H); 2.85-2.93 (m, 1H); 2.12-2.17 (m, 2H); 1.51-1.60 (overlapped m, 2H); 1.41 (s, 3H); 1.26 (s, 3H).

### S.ii. (1R,2S,4s)-4-(bromoethynyl)cyclopentane-1,2-diol:

A solution of intermediate S.i (1 g, 0.204 mmol) in 1*M* HCl (20 mL, 20 mmol) and THF (20 mL) was stirred at rt overnight. EA (50 mL) was added and the phases were separated. The aq. layer was saturated with NaCl and extracted with EA (2 x 50 mL). The combined org. layers were dried over MgSO₄, filtered and concentrated to dryness. The residue was purified by CC (Hept-EA) to afford the title compound as a white solid (0.652 g, 78% yield).
¹H NMR (CDCl₃) δ: 4.25 (quint, J = 4.2 Hz, 2H), 3.10-3.18 (m, 1H), 2.05-2.13 (m, 2H), 1.98 (m, 2H).

### Preparation T: 3-(3-iodoprop-2-yn-1-yl)oxetan-3-ol:

A flask charged with ZnBr₂ (1.08 g, 4.80 mmol) and Mg turnings (5.85 g) was heated with stirrring under vacuum at 150 °C for 2 h and then cooled to rt. To this were added, Et₂O (90 mL), a few drops of 1,2-dibromoethane, followed by dropwise addition of propargyl bromide (9 mL, 118.78 mmol) in Et₂O (70 mL). The mixture was stirred at the same temperature for 1 h. In a separate flask, were introduced 3-oxetanone (3.15 g, 43.71 mmol) and THF (420 mL). The Grignard reagent solution (127 mL, 65.56 mmol), cannulated in a graduated addition funnel, was added dropwise. The solution was stirred at the same temperature for 1 h and diluted with sat. NH₄Cl and Hex (100 mL). The two layers were separated and the aq. layer was extracted with Hex (100 mL). The combined org. layers were dried over MgSO₄, filtered and concentrated under reduced pressure. Starting from the crude product (4.33 g, 38.63 mmol) and proceeding in analogy to Preparation L, the title compound was obtained as a yellow solid (3.01 g; 33% yield).
¹H NMR (CDCl3) δ: 4.51 (d, J = 7.4 Hz, 2H), 4.66 (d, J = 7.1 Hz, 2H), 2.98 (s, 2H), 2.55 (s, 1H).

### Preparation U: (((1R,2R)-2-(bromoethynyl)-2-methylcyclopropyl)methoxy)(tert-butyl)diphenylsilane:

### U.i. ((1R,2R)-2-(hydroxymethyl)-1-methylcyclopropyl)methyl acetate:

To a solution of ((*1R,2R*)-2-formyl-1-methylcyclopropyl)methyl acetate (0.925 g; 5.92 mmol; prepared as described in WO 2012/154204) in MeOH (10 mL) was added NaBH₄ (0.297 g; 7.7 mmol) portion-wise at 0°C. The reaction was stirred for 80 min at 0°C then for 30 min at rt. Water (10 mL) and DCM (40 mL) were added and the phases were separated. The aq. layer was extracted with DCM-MeOH 9-1 (2 x 15 mL) and the combined org. layers were dried over Na₂SO₄ and filtered. The filtrate was evaporated under reduced pressure to give the title compound as a colourless oil (0.968 g; quant.).
¹H NMR (CDCl₃) δ: 3.89 (d, J = 11.3 Hz, 1H); 3.82 (d, J = 11.3 Hz, 1H); 3.74-3.80 (m, 1H); 3.49-3.56 (m, 1H); 2.08 (s, 3H); 1.19 (s, 3H); 1.09-1.15 (m, 1H); 0.70-0.76 (m, 1H); 0.27-0.31 (m, 1H).

### U.ii. ((1R,2R)-2-(((tert-butyldiphenylsilyl)oxy)methyl)-1-methylcyclopropyl)methyl acetate:

To a solution of intermediate U.i (0.94 g; 5.92 mmol) in DCM (12 mL) was added imidazole (0.819 g; 11.9 mmol). The solution was cooled to 0°C and TBDPSCl (1.6 mL; 6.03 mmol) was added dropwise. The reaction mixture was stirred at 0°C for 20 min then at rt for 2.5 h. Aq. NaHSO₄ (15%, 20 mL) was added. The aq. phase was extracted with DCM (10 mL). The combined org. layers were dried over MgSO₄, filtered and the filtrate concentrated to dryness. The residue was purified by CC (Hept-EA) to afford the title compound as a colourless oil (2.29 g; 97% yield).
¹H NMR (CDCl₃) δ: 7.66-7.70 (m, 4H); 7.35-7.45 (m, 6H); 3.84 (s, 2H); 3.82-3.88 (overlapped m, 1H); 3.46-3.55 (m, 1H); 2.07 (s, 3H); 1.14 (s, 3H); 1.05 (s, 9H), 1.03-1.11 (overlapped m, 1H); 0.59-0.65 (m, 1H); 0.14-0.19 (m, 1H).
MS (ESI, m/z): 397.01 [M+H⁺] for C₂₄H₃₂O₃Si; t_{R} = 1.13 min.

### U.iii. ((1R,2R)-2-(((tert-butyldiphenylsilyl)oxy)methyl)-1-methylcyclopropyl)methanol:

To a solution of intermediate U.ii (2.29 g; 5.77 mmol) in MeOH (50 mL) was added K₂CO₃ (1.59 g; 11.5 mmol). The suspension was stirred at rt for 4 h. The reaction mixture was filtered and the solid was washed with DCM. The filtrate was evaporated under reduced pressure. The residue was partitioned between water (30 mL) and DCM (40 mL). The aq. layer was extracted with DCM-MeOH 9-1 (40 mL) and EA-MeOH 9-1 (40 mL). The combined org. layers were dried over MgSO₄, filtered and evaporated under reduced pressure. The residue was purified by CC (Hept-EA) to afford the title compound as a colourless oil (1.59 g; 78% yield).
¹H NMR (CDCl₃) δ: 7.66-7.72 (m, 4H); 7.36-7.45 (m, 6H); 3.86 (dd, J = 5.8, 11.1 Hz, 1H); 3.49 (dd, J = 8.7, 11.1 Hz, 1H); 3.38 (d, J = 11.0 Hz, 1H); 3.30 (d, J = 11.0 Hz, 1H); 1.16 (s, 3H); 1.05 (s, 9H); 0.95-1.02 (m, 1H); 0.55 (dd, J = 4.8, 9.0 Hz, 1H); 0.12-0.16 (m, 1H).

### U.iv. (((1R,2R)-2-(bromoethynyl)-2-methylcyclopropyl)methoxy)(tert-butyl)diphenylsilane:

Starting from intermediate U.iii (1.59 g; 4.5 mmol) and proceeding successively in analogy to Preparation R, step R.ii (92% yield), Preparation M, step M.i (85% yield) and Preparation R, step R.iv (98% yield), the title compound was obtained as a yellow oil (1.48 g).
¹H NMR (CDCl₃) δ: 7.65-7.72 (m, 4H); 7.36-7.46 (m, 6H); 3.79 (dd, J = 5.6, 11.5 Hz, 1H); 3.49 (dd, J = 8.4, 11.5 Hz, 1H); 1.43-1.51 (m, 1H); 1.25 (s, 3H); 1.05 (s, 9H); 1.02 (dd, J = 4.7, 9.1 Hz, 1H); 0.37 (dd, J = 4.7, 6.4 Hz, 1H).

### Preparation V: (3R,6S)-6-(bromoethynyl)tetrahydro-2H-pyran-3-amine hydrochloride:

### V.i. Tert-butyl ((3R,6S)-6-(bromoethynyl)tetrahydro-2H-pyran-3-yl)carbamate:

Starting from *tert-butyl* ((3*R*,6*S*)-6-formyltetrahydro-2*H*-pyran-3-yl)carbamate (3.1 g; 13.6 mmol, prepared as described by Surivet et al. in J. Med. Chem. (2013), 56, 7396-7415) and proceeding successively in analogy to Preparation M, step M.i (68% yield) and Preparation R, step R.iv (97% yield), the title compound was obtained, after purification by CC (Hept-EA), as a white solid (2.7 g).
¹H NMR *(d6*-DMSO) δ: 6.84 (d, J = 7.6 Hz, 1H); 4.13 (dd, J = 2.7, 10.1 Hz, 1H); 3.76 (dd, J = 3.0, 10.5 Hz, 1H); 3.59-3.63 (m, 1H); 3.00-3.05 (m, 1H); 1.87-1.93 (m, 1H); 1.80-1.86 (m, 1H); 1.75-1.79 (m, 1H); 1.52-1.61 (m, 1H); 1.38 (s, 9H).

### V.ii. ((3R, 6S)-6-(bromoethynyl)tetrahydro-2H-pyran-3-amine hydrochloride:

A solution of intermediate V.i. (0.5 g, 1.64 mmol) in 4*M* HCl in dioxane (4 mL) was stirred for 3 h. The mixture was evaporated, taken in ether (2 mL) and filtered to afford a white solid (0.353 g, 89% yield).
¹H NMR (*d6*-DMSO) δ: 8.21-8.38 (m, 3H), 4.41 (dd, J = 3.2, 7.4 Hz, 1H), 3.97 (dd, J = 3.2, 11.6 Hz, 1H), 3.45 (dd, J = 7.4, 11.6 Hz, 1H), 3.12-3.21 (m, 1H), 1.98-2.08 (m, 2H), 1.55-1.72 (m, 2H).

### Preparation W: 3-iodo-N,N-dimethylprop-2-yn-1-amine:

Starting from *N*,*N*-dimethylprop-2-yn-1-amine (1 g; 12 mmol; commercial) and proceeding in analogy to Preparation L, the title compound was obtained as a yellow solid (0.746 g; 56% yield).
¹H NMR (CDCl₃) δ: 3.45 (s, 2H); 2.33 (s, 6H).

### Preparation X: 4-(iodoethynyl)tetrahydro-2H-pyran-4-ol:

Starting from 4-ethynyltetrahydro-2*H*-pyran-4-ol (1.17 g; 9.33 mmol; commercial) and proceeding in analogy to Preparation L, the title iodide was obtained, after purification by CC (Hept-EA), as a yellowish solid (1.57 g, 67% yield).
¹H NMR (*d6*-DMSO) δ: 5.64 (s, 1H); 3.64-3.74 (m, 2H); 3.40-3.51 (m, 2H); 1.68-1.79 (m, 2H); 1.51-1.62 (m, 2H).

### Preparation Y: 4-(iodoethynyl)piperidine hydrochloride:

### Y.i. Tert-butyl 4-(iodoethynyl)piperidine-1-carboxylate:

Starting from tert-butyl 4-ethynylpiperidine-1-carboxylate (0.952 g; 4.55 mmol; commercial) and proceeding in analogy to Preparation L (99% yield), the title compound was obtained as a yellow solid (1.51 g).
¹H NMR (CDCl₃) δ: 3.62-3.74 (m, 2H); 3.14-3.23 (m, 2H); 2.70-2.78 (m, 1H); 1.72-1.80 (m, 2H); 1.55-1.63 (m, 2H); 1.45 (s, 9H).
MS (ESI, m/z): 335.85 [M+H⁺] for C₁₂H₁₈NO₂I; t_{R} = 0.93 min.

### Y.ii. 4-(iodoethynyl)piperidine hydrochloride:

Starting from the intermediate Y.i (0.5 g, 1.5 mmol), and proceeding in analogy to Preparation V, step V.ii, the title compound (0.365 g, 90% yield) was obtained after drying as a yellow solid.
¹H NMR *(d6*-DMSO) δ: 8.90-9.04 (m, 2H), 3.06-3.18 (m, 2H), 2.80-2.99 (m, 3H), 1.89-1.99 (m, 2H), 1.65-1.77 (m, 2H).

### Preparation Z: tert-butyl((3-(iodoethynyl)oxetan-3-yl)methoxy)diphenylsilane:

### Z.i. 3-(((tert-butyldiphenylsilyl)oxy)methyl)oxetane-3-carbaldehyde:

Starting from oxetane-3,3-diyldimethanol (5 g; 42.3 mmol; commercial) and proceeding successively in analogy to Preparation R, step R.i (95% yield) and step R.ii (90% yield), the title compound was obtained, after purification by CC (Hept-EA), as a colourless oil (12.87 g).
¹H NMR *(d6*-DMSO) *δ*: 9.82 (s, 1H); 7.59-7.62 (m, 4H); 7.44-7.50 (m, 6H); 4.66 (d, J = 6.3 Hz, 2H); 4.43 (d, J = 6.3 Hz, 2H); 4.15 (s, 2H); 0.98 (s, 9H).

### Z.ii. Tert-butyl((3-ethynyloxetan-3-yl)methoxy)diphenylsilane:

A suspension of intermediate Z.i (2 g, 5.64 mmol) and K₂CO₃ (1.56 g, 11.3 mmol) in methanol (50 mL) was treated dropwise with with dimethyl 1-diazo-2-oxopropylphosphonate (1.19 g, 6.21 mmol; commercial). The reaction mixture was stirred at rt overnight. The solvent was evaporated and the residue was dissolve in DCM (20 mL) and water (15 mL). The aq. layer was extracted once with DCM (15 mL). The combined org. layers were washed with brine, filtered and concentrated down. The crude product was purified by CC using a Hept-EA gradient to afford the desired product as a colourless oil (1.71 g).
¹H NMR *(d6*-DMSO): 7.64-7.72 (m, 5H); 7.43-7.53 (m, 5H); 4.59 (d, J = 5.6 Hz, 2H); 4.52 (d, J = 5.6 Hz, 2H); 3.89 (s, 2H); 3.45 (s, 1H); 1.04 (s, 9H).

### Z.iii. Tert-butyl((3-(iodoethynyl)oxetan-3-yl)methoxy)diphenylsilane:

Starting from intermediate Z.ii (2 g; 5.64 mmol) and proceeding in analogy to Preparation L (41% yield), the title compound was obtained, after purification by prep-HPLC (Method 3), as a colourless oil (0.94 g).
¹H NMR *(d6*-DMSO) δ: 7.64-7.72 (m, 4H); 7.42-7.54 (m, 6H); 4.58 (d, J = 5.8 Hz, 2H); 4.48 (d, J = 5.8 Hz, 2H); 3.90 (s, 2H); 1.03 (s, 9H).

### Preparation AA: cis-3-(hydroxymethyl)-1-(3-iodoprop-2-yn-1-yl)cyclobutan-1-ol:

### AA.i. Cis-3-(((tert-butyldiphenylsilyl)oxy)methyl)-1-(3-(trimethylsilyl)prop-2-yn-1-yl)cyclobutan-1-ol:

To a solution of 3-(((*tert*-butyldiphenylsilyl)oxy)methyl)cyclobutan-1-one (2 g; 3.54 mmol; prepared as described in WO 2006/063281) in dry THF (5.9 mL) at rt under nitrogen atmosphere, was added a solution of trimethyl(3-(4,4,5,5-tetramethyl-1,3,2-dioxaborolan-2-yl)prop-1-yn-1-yl)silane (1.27 g; 5.32 mmol; commercial) in dry THF (5.9 mL) followed by diethylzinc (15% in toluene; 0.73 mL; 1.06 mmol). The reaction was stirred at rt for 4 h. Water (10 mL) was added carefully followed by aq. HCl (6 M, 0.3 mL) and the reaction was stirred for 15 min. The mixture was extracted with EA (3 x 15 mL). The combined org. layers were washed with brine (15 mL), dried over Na₂SO₄, filtered and the filtrate concentrated under reduced pressure. The residue was purified by CC (Hept-EA) to afford the dirty desired product as a colourless oil (2 g, quant.).
¹H NMR *(d6*-DMSO) δ: 7.59-7.63 (m, 4H); 7.41-7.49 (m, 6H); 5.09 (s, 1H); 3.62 (d, J = 6.8 Hz, 2H); 2.31 (s, 2H); 1.88-1.99 (m, 3H); 1.22-1.31 (m, 2H); 1.00 (s, 9H); 0.07 (s, 9H).
MS (ESI, m/z): 451.0 [M+H⁺] for C₂₇H₃₈O₂Si₂; t_{R} = 1.14 min.

### AA.ii. Cis-3-(hydroxymethyl)-1-(3-iodoprop-2-yn-1-yl)cyclobutan-1-ol:

Starting from intermediate AA.i (crude, 2 g; 1.77 mmol) and proceeding successively in analogy to Preparation M, step M.ii (72% yield) and Preparation G (48% yield), the title compound was obtained, after purification by CC (Hept-EA), as a yellow oil (0.4 g) which crystallized.
¹H NMR *(d6*-DMSO) δ: 5.06 (s, 1H); 4.45 (t, J = 5.4 Hz, 1H); 3.32-3.36 (overlapped m, 2H); 2.48-2.52 (overlapped m, 1H); 1.98-2.04 (m, 2H); 1.88 (m, 1H); 1.64-1.70 (m, 2H). MS (ESI, m/z): 266.95 [M+H⁺] for C₈H₁₁O₂I; t_{R} = 0.52 min.

### Preparation AB: 2-hydroxy-1-(4-(iodoethynyl)piperidin-1-yl)ethan-1-one:

### AB.i. 1-(4-ethynylpiperidin-1-yl)-2-hydroxyethan-1-one:

To a solution of 4-ethynylpiperidine hydrochloride (0.720 g, 4.94 mmol, commercial) in MeCN (9.5 mL) and DMF (4.5 mL) was added TEA (3 mL, 21.5 mmol), EDC (1.17 g, 5.97 mmol), HOBT (0.935 g, 6.71 mmol) and glycolic acid (0.425 g, 5.54 mmol. The reaction mixture was stirred at rt for 20 h. The solvent was removed under reduced pressure. The residue was diluted with water (15 mL) and EA (15 mL). The two phases were separated and the aq. layer was extracted with EA (3 x 15 mL). The combined org. layers were washed with NaHCO₃ (30 mL) and brine (30 mL), dried over MgSO₄ and concentrated to dryness. The residue was purified by CC (DCM-MeOH) to afford the title product as a white solid (0.569 g).
¹H NMR (300 MHz, DMSO-*d6*) δ: 4.44 (t, J = 5.4 Hz, 1H); 4.05 (d, J = 5.3 Hz, 2H); 3.80 (m, 1H); 3.47 (m, 1H); 3.05-3.18 (m, 2H); 2.95 (d, J = 2.4 Hz, 1H); 2.65 (m, 1H); 1.66-1.81 (m, 2H); 1.31-1.53 (m, 2H).

### AB.ii. 2-hydroxy-1-(4-(iodoethynyl)piperidin-1-yl)ethan-1-one:

Starting from intermediate AB.i (0.255 g; 1.52 mmol; commercial) and proceeding in analogy to Preparation L, the title compound was obtained as a yellow solid (0.400 g; 90% yield).
MS (ESI, m/z): 293.84 [M+H⁺] for C₉H₁₂NO₂I; t_{R} = 0.63 min.

### Preparation AC: (((1R*,2R*)-2-(bromoethynyl)-2-fluorocyclopropyl)methoxy)(tert-butyl)diphenylsilane:

### AC.i. ((1R*,2R*)-2-(((tert-butyldiphenylsilyl)oxy)methyl)-1-fluorocyclopropyl)methanol:

To a solution of ethyl (1*R**,2*R**)-2-(((*tert*-butyldiphenylsilyl)oxy)methyl)-1-fluorocyclopropane-1-carboxylate (7.01 g; 17.5 mmol; prepared as described in Sakagami and al., Bioorg. & Med. Chem. (2008), 16(8), 4359-4366) in THF (125 mL), cooled to -78°C, was added LiBH₄ (2M in THF; 31 mL; 62 mmol) dropwise over 10 min, keeping the IT below -70°C. The reaction mixture was allowed to reach rt and stirred for 1 day. The reaction mixture was cooled to -78°C and LiBH₄ (2M in THF; 10 mL; 20 mmol) was slowly added. The cooling bath was removed and the reaction mixture was stirred for 20 h. MeOH (34 mL) was slowly added (strong gas evolution) and the reaction mixture was stirred for 20 min before concentration. The solid residue was taken up in DCM (150 mL) and water (250 mL). The phases were separated and the aq. layer was extracted with DCM (3 x 50 mL). The combined org. layers were washed with brine (150 mL), dried over MgSO₄ and evaporated under reduced pressure to afford the title compound as a colourless oil (6.89 g; quant.).
¹H NMR (CDCl₃) δ: 7.67-7.71 (m, 4H); 7.36-7.45 (m, 6H); 3.89 (ddd, J = 1.7, 6.0, 11.0 Hz, 1H); 3.80-3.83 (m, 1H); 3.70-3.79 (m, 2H); 1.76 (t, J = 6.4 Hz, 1H); 1.25-1.33 (m, 1H); 1.06 (s, 9H); 0.79-0.88 (m, 2H).
MS (ESI, m/z): 358.95 [M+H⁺] for C₂₁H₂₇O₂FSi; t_{R} = 1.01 min.

### AC.ii. (1R*,2R*)-2-(((tert-butyldiphenylsilyl)oxy)methyl)-1-fluorocyclopropane-1-carbaldehyde:

Starting from intermediate AC.i (2.043 g; 5.70 mmol) and proceeding in analogy to Preparation R, step R.ii, the title compound was obtained as a colourless oil (1.687 g; 83% yield).
¹H NMR (CDCl₃) δ: 9.73 (d, J = 5.6 Hz, 1H); 7.65-7.69 (m, 4H); 7.37-7.46 (m, 6H); 3.96 (ddd, J = 1.5, 5.3, 11.4 Hz, 1H); 3.73 (ddd, J = 0.7, 8.2, 11.3 Hz, 1H); 1.86-1.95 (m, 1H); 1.50 (ddd, J = 6.6, 8.7, 10.5 Hz, 1H); 1.26 (ddd, J = 6.6, 8.5, 18.7 Hz, 1H); 1.04 (s, 9H).
MS (ESI, m/z): 357.12 [M+H⁺] for C₂₁H₂₅O₂FSi; t_{R} = 1.06 min.

### AC.iii. Tert-butyl(((1R*,2R*)-2-(2,2-dibromovinyl)-2-fluorocyclopropyl)methoxy)diphenylsilane:

Starting from intermediate AC.ii (1.687 g; 4.73 mmol) and proceeding in analogy to Preparation M, step M.i, the title compound was obtained as a colourless oil (0.421 g; 17% yield).
¹H NMR (CDCl₃) δ: 7.67-7.72 (m, 4H); 7.36-7.46 (m, 6H); 6.72 (d, J = 8.7 Hz, 1H); 3.79-3.89 (m, 2H); 1.42-1.51 (m, 1H); 1.24-1.33 (m, 1H); 0.96-1.12 (overlapped m, 1H); 1.06 (s, 9H).
t_{R} = 1.16 min.

### AC.iv. (((1R*,2R*)-2-(bromoethynyl)-2-fluorocyclopropyl)methoxy)(tert-butyl)diphenylsilane :

Starting from intermediate AC.iii (0.421 g; 0.82 mmol) and proceeding in analogy to Preparation R, step R.iv, the title compound was obtained as a brown oil (0.351 g; 99% yield).
¹H NMR (CDCl₃) δ: 7.66-7.70 (m, 4H); 7.36-7.45 (m, 6H); 3.84 (ddd, J = 1.6, 5.8, 11.3 Hz, 1H); 3.71 (ddd, J = 1.1, 8.0, 11.3 Hz, 1H); 1.56-1.64 (m, 1H); 1.14-1.20 (m, 1H); 1.06 (s, 9H); 0.98-1.04 (m, 1H).
t_{R} = 1.13 min.

### Preparation AD: ((1R*,2R*)-2-(bromoethynyl)-1-fluorocyclopropyl)methyl acetate:

### AD.i. ((1R*,2R*)-2-(((tert-butyldiphenylsilyl)oxy)methyl)-1-fluorocyclopropyl)methyl acetate:

Starting from intermediate AC.i (2.12 g; 5.91 mmol) and proceeding in analogy to Preparation U, step U.i, the crude product was obtained as a yellow oil (2.3 g).
¹H NMR (CDCl₃) δ: 7.66-7.71 (m, 4H); 7.36-7.45 (m, 6H); 4.27-4.35 (m, 2H); 3.90 (ddd, J = 1.6, 5.8, 11.0 Hz, 1H); 3.69 (ddd, J = 1.2, 8.3, 11.0 Hz, 1H); 2.11 (s, 3H); 1.31-1.40 (m, 1H); 1.06 (s, 9H); 0.80-0.94 (m, 2H).
MS (ESI, m/z): 400.98 [M+H⁺] for C₁₂H₁₈NO₂; t_{R} = 1.09 min.

### AD.ii. ((1R*,2R*)-1-fluoro-2-(hydroxymethyl)cyclopropyl)methyl acetate:

To a solution of intermediate AD.i (2.16 g; 5.39 mmol) in THF (10 mL) was added TBAF (1*M* in THF; 7 mL). The reaction mixture was stirred at rt for 1 h. The reaction mixture was concentrated *in vacuo* and purified by CC (DCM-MeOH) to afford the title alcohol as a yellow oil (0.726 g; 83% yield).
¹H NMR (CDCl₃) δ: 4.27-4.41 (m, 2H); 3.94 (m, 1H); 3.64 (m, 1H); 2.13 (s, 3H); 1.51 (m, 1H); 1.41 (m, 1H); 0.98-1.06 (m, 2H).

### AD.iii. ((1R*,2R*)-2-(bromoethynyl)-1-fluorocyclopropyl)methyl acetate:

Starting from intermediate AD.ii (0.725 g; 4.46 mmol) and proceeding successively in analogy to Preparation R, step R.ii (100% yield), Preparation M, step M.i (52% yield) and Preparation R, step R.iv (57% yield), the title compound was obtained as a colourless oil (0.351 g).
¹H NMR (CDCl₃) δ: 6.21 (dd, J = 1.3, 8.8 Hz, 1H); 4.32-4.38 (m, 2H); 2.14 (s, 3H); 1.90-1.98 (m, 1H); 1.22-1.35 (m, 2H).

### Preparation AE: 1-(3-(bromoethynyl)azetidin-1-yl)-2-hydroxyethan-1-one:

### AE.i. Tert-butyl 3-(bromoethynyl)azetidine-1-carboxylate:

To a solution of *tert-*butyl 3-ethynylazetidine-1-carboxylate (0.5 g; 2.76 mmol; prepared as described in WO 2014/165075) and NBS (0.591 g, 3.32 mmol) in acetone (11 mL) was added AgNO₃ (0.05 g, 0.29 mmol). The mixture was stirred at rt for 90 min. The reaction mixture was filtered through Celite and the filtrate was concentrated to dryness. The residue was purified by CC (Hex-TBME) to give the title compound as a colourless oil (0.673 g, 94% yield).
¹H NMR (CDCl₃) δ: 4.14 (m, 2H); 3.96 (dd, J = 6.3, 8.4 Hz, 2H); 3.34 (m, 1H); 1.46 (s, 9H).

### AE.ii. 3-(bromoethynyl)azetidine hydrochloride:

Starting from intermediate AE.i (0.670 g, 2.58 mmol) and proceeding in analogy to Preparation V, step V.ii, the title compound was obtained, after trituration in Et₂O, as an off-white solid (0.49 g; 97% yield).
¹H NMR (CDCl₃) δ: 9.10-9.44 (m, 2H); 4.06-4.15 (m, 2H); 3.87-3.96 (m, 2H); 3.74 (m, 1H).
MS (ESI, m/z): 162.0 [M+H⁺] for C₅H₆NBr; t_{R} = 0.23 min.

### AE. iii. 1-(3-(bromoethynyl)azetidin-1-yl)-2-hydroxyethan-1-one:

To a solution of intermediate AE.ii (0.49 g; 2.48 mmol) in DMF (5 mL) were added successively HOBT (0.7 g; 5.05 mmol), TEA (1.21 mL; 8.69 mmol), glycolic acid (0.2 g; 2.63 mmol) and EDC (0.85 g; 4.38 mmol). The reaction mixture was diluted with DMF (4 mL) and the reaction mixture was stirred at 60°C for 90 min. The solvent was removed in vacuo and the residue was partitioned between brine (20 mL) and EA-MeOH (9-1; 30 mL). The aq. layer was extracted with EA-MeOH (9-1; 4 x 20 mL). The org. layer was dried over Na₂SO₄, filtered and concentrated to dryness. The residue was purified by CC (Hept-EA) to afford the title compound as an off-white solid (0.32 g, 60% yield).
¹H NMR (*d6*-DMSO) δ: 4.97 (t, J = 6.1 Hz, 1H); 4.40 (t, J = 8.7 Hz, 1H); 4.11 (m, 2H), 3.89 (d, J = 6.0 Hz, 2H); 3.77 (dd, J = 6.2, 9.0 Hz, 1H); 3.55 (m, 1H).
MS (ESI, m/z): 220.1 [M+H⁺] for C₇H₈NO₂Br; t_{R} = 0.48 min.

### Preparation AF: ((1S,2S)-2-(bromoethynyl)-1-methylcyclopropyl)methyl acetate:

### AF.i. (R,E)-3-(2,2-dimethyl-1,3-dioxolan-4-yl)-2-methylallyl acetate:

To a solution of (*R*,*E*)-3-(2,2-dimethyl-1,3-dioxolan-4-yl)-2-methylprop-2-en-1-ol (1.4 g; 8.1 mmol; prepared as reported in Smith III et al., Tetrahedron (2009), 65(33), 6470-6488) in THF (48 mL) was added TEA (2.8 mL; 20.1 mmol). Then AcCl (1.2 mL; 16.5 mmol) was added dropwise over 10 min at 0°C. The reaction mixture was stirred at 0°C for 2 h. The reaction mixture was poured into water (80 mL) and extracted with EA (3 x 50 mL). The combined org. layers were dried over MgSO₄, filtered and the filtrate concentrated under reduced pressure. The crude product was purified by CC (PE-EA) to afford the title compound as a colourless oil (1.64 g; 94% yield).
¹H NMR (CDCl₃) δ: 5.48-5.51 (m, 1H); 4.79-4.84 (m, 1H); 4.44-4.52 (m, 2H); 4.07-4.11 (m, 1H); 3.55 (t, J = 8.0 Hz, 1H); 2.09 (s, 3H); 1.75 (d, J = 1.3 Hz, 3H); 1.43 (s, 3H); 1.40 (s, 3H).

### AF.ii. ((1S,2S)-2-((R)-2,2-dimethyl-1,3-dioxolan-4-yl)-1-methylcyclopropyl)methyl acetate:

To a mechanically stirred solution of intermediate AF.i (1.64 g; 7.65 mmol) in toluene (102 mL), cooled to -25°C, was added dropwise ZnEt₂ (15% in toluene; 34.5 mL; 38.3 mmol) over 20 min, keeping IT below -20°C. Then diiodomethane (6.5 mL; 79.9 mmol) was added dropwise over 10 min, keeping IT below -20°C. The reaction mixture was stirred at -20°C for 2 h, then allowed to slowly warm up to rt and stirred overnight. The reaction mixture was quenched with sat. aq. NH₄Cl (33 mL) and extracted with Et₂O (4 x 30 mL). The combined org. layers were washed with sat. aq. Na₂S₂O₃ (30 mL), water (30 mL) and brine (30 mL), then dried over MgSO₄ and filtered. After evaporation of the filtrate under reduced pressure, a yellow oil (22.4 g) was obtained. The crude product was purified by CC (PE-EA) to afford the title compound as a colourless oil (1.4 g; 80% yield).
¹H NMR (CDCl₃) δ: 4.09 (dd, J = 5.9, 7.9 Hz, 1H); 3.89 (d, J = 11.3 Hz, 1H); 3.77 (d, J = 11.3 Hz, 1H); 3.70-3.76 (overlapped m, 1H); 3.61-3.66 (m, 1H); 2.07 (s, 3H); 1.45 (s, 3H); 1.36 (s, 3H); 1.13 (s, 3H); 0.85-0.95 (m, 2H); 0.56 (t, J = 5.0 Hz, 1H).

### AF.iii. ((1S,2S)-2-((R)-1,2-dihydroxyethyl)-1-methylcyclopropyl)methyl acetate:

A mixture of intermediate AF.ii (1.4 g; 6.1 mmol) in AcOH (80%; 14 mL) was stirred at rt for 23 h. The mixture was added to sat. aq. NaHCO₃ (100 mL; pH 6-7) and the aq. layer was extracted with DCM (3 x 60 mL). The combined org. layers were washed with water (10 mL) and brine (20 mL), dried over MgSO₄, filtered and concentrated to dryness. The residue was co-evaporated with cyclohexane. The crude product was purified by CC (DCM-MeOH) to afford the title compound as a colourless oil (1 g; 87% yield).
¹H NMR (CDCl₃) δ: 3.89 (d, J = 11.3 Hz, 1H); 3.74 (d, J = 11.3 Hz, 1H); 3.68 (dd, J = 3.4, 11.2 Hz, 1H); 3.57 (dd, J = 7.4, 11.2 Hz, 1H); 3.33-3.39 (m, 1H); 2.07 (s, 3H); 1.16 (s, 3H); 0.89 (td, J = 5.7, 9.0 Hz, 1H); 0.80 (dd, J = 4.9, 8.8 Hz, 1H); 0.48 (t, J = 5.3 Hz, 1H).

### AF.iv. ((1S,2S)-2-formyl-1-methylcyclopropyl)methyl acetate:

To a solution of intermediate AF.iii (1 g; 5.3 mmol) in THF (16.5 mL), water (3.4 mL) and sat. aq. NaHCO₃ (1.6 mL), cooled to 0°C, was added NaIO₄ (1.48 g; 6.9 mmol). The reaction mixture was stirred at 0°C for 30 min, then filtered and the precipitate washed with Et₂O. The layers were separated and the aq. layer was extracted with Et₂O (3 x 40 mL). The combined org. layers were dried over MgSO₄, filtered and concentrated to dryness. The title compound was obtained as a colourless oil (0.81 g; 98% yield).
¹H NMR (CDCl₃) δ: 9.47 (d, J = 4.7 Hz, 1H); 4.00 (d, J = 11.4 Hz, 1H); 3.85 (d, J = 11.4 Hz, 1H); 2.09 (s, 3H); 1.92-1.97 (m, 1H); 1.39 (t, J = 5.3 Hz, 1H); 1.32 (s, 3H); 1.21 (dd, J = 5.0, 8.3 Hz, 1H).

### AF.v. ((1S,2S)-2-(bromoethynyl)-1-methylcyclopropyl)methyl acetate:

Starting from intermediate AF.iv (0.81 g; 5.19 mmol) and proceeding successively in analogy to Preparation M, steps M.i (81% yield) and M.ii (62% yield), the title compound was obtained, after purification by CC (PE/TBME), as a colourless oil (0.6 g).
¹H NMR (CDCl₃) δ: 3.89 (d, J = 11.4 Hz, 1H); 3.80 (d, J = 11.4 Hz, 1H); 2.07 (s, 3H); 1.39 (dd, J = 5.5, 8.9 Hz, 1H); 1.27 (s, 3H); 0.94 (dd, J = 4.8, 8.9 Hz, 1H); 0.65 (t, J = 5.1 Hz, 1H).

### Preparation AG: ((1R,2R)-2-(bromoethynyl)-1-fluorocyclopropyl)methyl benzoate:

### AG.i. ((1R*,2R*)-2-(((tert-butyldiphenylsilyl)oxy)methyl)-1-fluorocyclopropyl)methanol:

To a solution of ethyl (*1R**,*2R**)-2-(((*tert*-butyldiphenylsilyl)oxy)methyl)-1-fluorocyclopropane-1-carboxylate (0.5 g; 1.25 mmol; prepared as described in Sakagami et al., Bioorg. Med. Chem. (2008), 16(8), 4359-4366) in THF (9 mL), cooled to -78°C, was added dropwise LiBH₄ (2*M* in THF; 2.2 mL; 4.4 mmol). The reaction mixture was allowed to reach rt and stirred at rt for 24 h. MeOH (2 mL) was carefully added, the reaction mixture was stirred for 20 min, concentrated to dryness and partitioned between water (10 mL) and DCM (15 mL). The aq. layer was extracted with DCM (2 x 10 mL). The combined org. layers were dried over Na₂SO₄ and filtered. After concentration of the filtrate to dryness, the title compound was obtained as a colourless oil (0.429 g; 96% yield).

¹H NMR (CDCl₃) δ: 7.66-7.72 (m, 4H); 7.36-7.45 (m, 6H); 3.89 (ddd, J = 1.6, 6.0, 11.0 Hz, 1H); 3.80-3.83 (m, 1H); 3.70-3.78 (m, 2H); 1.74 (t, J = 6.4 Hz, 1H); 1.24-1.33 (m, 1H); 1.05 (s, 9H); 0.79-0.88 (m, 2H).
MS (ESI, m/z): 358.95 [M+H⁺] for C₂₁H₂₇O₂FSi; t_{R} = 1.01 min.

### AG.ii. ((1R*,2R*)-2-(((tert-butyldiphenylsilyl)oxy)methyl)-1-fluorocyclopropyl)methyl benzoate:

To a solution of intermediate AG.i (5.51 g, 15.4 mmol) in THF (93 mL) was added TEA (6 mL; 43.1 mmol). Benzoyl chloride (3.6 mL; 30.7 mmol) was added dropwise over 2 min at 0°C. The reaction mixture was stirred at 0°C for 5 h before being poured onto water (75 mL). The aq. layer was extracted with EA (3 x 50 mL). The combined org. layers were dried over MgSO₄ and concentrated to dryness. The residue was purified by CC (Hept-EA) to afford the title compound as a colourless oil (6.49 g; 91% yield).
¹H NMR (CDCl₃) δ: 8.09-8.12 (m, 2H); 7.67-7.70 (m, 4H); 7.56 (m, 1H); 7.40-7.44 (m, 4H); 7.35-7.38 (m, 4H); 4.62 (m, 1H); 4.51 (ddd, J = 1.1, 13.0, 23.8 Hz, 1H); 3.93 (ddd, J = 1.5, 5.6, 11.0 Hz, 1H); 3.70 (ddd, J = 1.1, 8.4, 10.9 Hz, 1H); 1.46 (m, 1H); 1.30 (m, 1H); 1.02 (s, 7H); 0.97 (m, 1H); 0.84-0.91 (m, 2H).
MS (ESI, m/z): 463.07 [M+H⁺] for C₂₈H₃₁O₃FSi; t_{R} = 1.14 min.

### AG.iii. ((1R*,2R*)-1-fluoro-2-(hydroxymethyl)cyclopropyl)methyl benzoate:

To a solution of intermediate AG.ii (6.49 g; 14 mmol) in THF (26 mL) was added TBAF (1M in THF, 17 mL). The reaction mixture was stirred at rt for 45 min. The reaction mixture was concentrated in vacuo and the residue was purified by CC (DCM-MeOH) to afford the title compound (2.81 g; 89% yield) as a yellow oil.
¹H NMR (CDCl₃) δ: 8.08-8.10 (m, 2H); 7.58 (m, 1H); 7.45-7.48 (m, 2H); 4.64 (m, 1H); 4.55 (m, 1H); 3.97 (ddd, J = 1.5, 5.8, 11.8 Hz, 1H); 3.68 (ddd, J = 1.4, 8.7, 11.8 Hz, 1H); 1.52 (m, 1H); 1.04-1.12 (m, 2H).

### AG.iv. ((1R,2R)-2-(2,2-dibromovinyl)-1-fluorocyclopropyl)methyl benzoate:

Starting from intermediate AG.iii (2.77 g; 12.4 mmol) and proceeding successively in analogy to Preparation R, step R.ii (84% yield) and Preparation M, step M.i (77% yield), a mixture of enantiomers (2.71 g) was obtained. After separation by semi-preparative chiral HPLC Method B (Hept-EtOH 3-7; flow rate: 16 mL/min, UV detection at 224 nM), the title enantiomer (first-eluting enantiomer) was obtained as a white solid (1.25 g). The retention time on analytical chiral HPLC (flow rate 0.80 mL/min) was 5.3 min.
¹H NMR (*d6*-DMSO) δ: 7.99-8.01 (m, 2H); 7.69 (m, 1H); 7.54-7.58 (m, 2H); 6.38 (dd, J = 1.4, 8.9 Hz, 1H); 4.57-4.75 (m, 2H); 2.09 (m, 1H); 1.48-1.55 (m, 2H).

### AG.v.((1R,2R)-2-(bromoethynyl)-1-fluorocyclopropyl)methyl benzoate:

To a solution of intermediate AG.iv (2.05 g, 5.42 mmol) in THF (20 mL) was added TBAF (1*M* in THF, 22 mL; 21.7 mmol). The mixture was stirred overnight. The reaction mixture was diluted with EA (50 mL) and water (30 mL). The two layers were separated and the org. layer was extracted with EA (3 x 50 mL). The evaporation residue was purified by CC (Hept-EA) to afford the title compound as a yellowish oil (1.1 g; 68% yield).
¹H NMR (*d6*-DMSO) δ: 7.99-8.03 (m, 2H); 7.70 (m, 1H); 7.55-7.60 (m, 2H); 4.51-4.67 (m, 2H); 2.04-2.09 (m, 1H); 1.37-1.49 (m, 2H).

### Reference Example 1: (RS)-N-hydroxy-4-(6-(4-methoxyphenyl)benzo[d]thiazol-2-yl)-2-methyl-2-(methylsulfonyl)butanamide:

A sealed tube was charged with the compound of Preparation A (0.194 g, 0.395 mmol), 4-methoxyphenylboronic acid (0.101 g, 0.592 mmol, commercial), bis(tri-*tert*-butylphosphine)palladium(0) (0.0127 g, 0.0249 mmol) and degassed TEA (0.0974 mL, 0.701 mmol). Dioxane (3 mL) was added and the mixture was refluxed under an Argon atmosphere for 2 h. The mixture was diluted with EA (50 mL) and water (50 mL). The org. phase was dried over MgSO₄ and concentrated to dryness. The crude residue was purified by prep-HPLC (Method 2) to afford the title compound as a yellow solid (0.04 g, 23% yield).
¹H NMR (*d6*-DMSO) *δ*: 8.27-8.34 (m, 1H); 7.92-8.00 (m, 1H); 7.70-7.79 (m, 1H); 7.65-7.70 (m, 2H); 7.00-7.08 (m, 2H); 3.79 (s, 3H); 3.17-3.25 (m, 1H); 3.06 (s, 3H); 2.88-3.02 (m, 1H); 2.70-2.83 (m, 1H); 2.17-2.31 (m, 1H); 1.55 (s, 3H).
MS (ESI, m/z): 435.1 [M+H⁺] for C₂₀H₂₂N₂O₅S₂; t_{R} = 0.69 min.

### Reference Example 2: (RS)-N-hydroxy-4-(6-((4-(hydroxymethyl)phenyl)ethynyl)benzo[d]thiazol-2-yl)-2-methyl-2-(methylsulfonyl)butanamide:

### RE2.i. (RS)-tert-butyl 4-(6-((4-(hydroxymethyl)phenyl)ethynyl)benzo[d]thiazol-2-yl)-2-methyl-2-(methylsulfonyl)butanoate:

To a solution of the compound of Preparation C (0.198 g, 0.503 mmol) in degassed THF (5 mL) were added 4-iodobenzyl alcohol (0.118 g, 0.503 mmol, commercial), CuI (24.7 mg, 0.13 mmol), TEA (0.245 mL, 3.5 mmol) and PdCl₂(PPh₃)₂ (0.038 g, 0.0553 mmol). The mixture was stirred at rt for 3 h. The mixture was then concentrated to dryness and purified by CC (Hept-EA) to afford the title compound as a yellow solid (0.203 g, 81% yield).
¹H NMR (*d6*-DMSO) δ: 8.32 (d, J = 1.1 Hz, 1H); 7.98 (d, J = 8.4 Hz, 1H); 7.64 (dd, J = 1.5, 8.4 Hz, 1H); 7.50-7.56 (m, 2H); 7.34-7.42 (m, 2H); 5.28 (t, J = 5.8 Hz, 1H); 4.54 (d, J = 5.8 Hz, 2H); 3.30-3.42 (overlapped m, 1H); 3.14 (s, 3H); 2.95-3.11 (overlapped m, 1H); 2.53-2.75 (m, 1H); 2.24-2.43 (m, 1H); 1.57 (s, 3H); 1.45 (s, 9H).
MS (ESI, m/z): 500.1 [M+H⁺] for C₂₆H₂₉NO₅S₂; t_{R} = 0.95 min.

### RE2.ii. (RS)-4-(6-((4-(hydroxymethyl)phenyl)ethynyl)benzo[d]thiazol-2-yl)-2-methyl-2-(methylsulfonyl)butanoic acid:

To a solution of intermediate RE2.i (0.186 g, 0.372 mmol) in dioxane (4.8 mL) and water (0.34 mL) was added a HCl solution (4*M* in dioxane, 2.8 mL). The mixture was stirred at rt overnight. The mixture was then concentrated to dryness and the residue was triturated in water (5 mL) and filtered. The solid was triturated in EA (3 mL), filtered and dried to afford the title product as a yellow solid (0.08 g, 48% yield).
¹H NMR (*d6*-DMSO) δ: 8.28-8.32 (m, 1H); 7.94-7.99 (m, 2H); 7.64 (dd, J = 1.3, 8.6 Hz, 1H); 7.50-7.56 (m, 2H); 7.35-7.41 (m, 2H); 4.54 (br. s, 2H); 3.31-3.41 (overlapped m, 1H); 3.15 (s, 3H); 3.02-3.14 (overlapped m, 1H); 2.62-2.74 (m, 1H); 2.27-2.45 (m, 1H): 1.58 (m, 3H).
MS (ESI, m/z): 444.2 [M+H⁺] for C₂₂H₂₁NO₅S₂; t_{R} = 0. 77 min.

### RE2.iii. Rac-4-(6-((4-(hydroxymethyl)phenyl)ethynyl)benzo[d]thiazol-2-yl)-2-methyl-2-(methylsulfonyl)-N-(((RS)-tetrahydro-2H-pyran-2-yl)oxy)butanamide:

Starting from intermediate RE2.ii (0.08 g, 0.18 mmol), and proceeding in analogy to Preparation A, step A.iv, the title compound was obtained, after purification by CC (Hept-EA), as a colourless oil (0.03 g; 31% yield).
¹H NMR (*d6*-DMSO) δ: 11.38 (s, 1H); 8.27-8.30 (m, 1H); 7.95 (d, J = 8.4 Hz, 1H); 7.61-7.66 (m, 1H); 7.53 (d, J = 8.0 Hz, 2H); 7.36 (d, J = 8.0 Hz, 2H); 5.26 (t, J = 5.7 Hz, 1H); 4.91-5.00 (m, 1H); 4.54 (d, J = 5.7 Hz, 2H); 3.98-4.20 (m, 1H); 3.31-3.51 (m, 1H); 3.17-3.30 (overlapped m, 1H); 3.06 (s, 1.5H); 3.08 (s, 1.5H); 2.93-3.04 (overlapped m, 1H); 2.68-2.81 (m, 1H); 2.18-2.31 (m, 1H); 1.47-1.71 (m, 9H).
MS (ESI, m/z): 543.22 [M+H⁺] for C₂₇H₃₀N₂O₆S₂; t_{R} = 0. 84 min.

### RE2.iv. (RS)-N-hydroxy-4-(6-((4-(hydroxymethyl)phenyl)ethynyl)benzo[d]thiazol-2yl)-2-methyl-2-(methylsulfonyl)butanamide:

To a solution of intermediate RE2.iii (0.027 g, 0.05 mmol) in EtOH (1 mL) was added pyridinium *p*-toluenesulfonate (0.07 g, 0.25 mmol). The mixture was stirred at 80°C for 2 h. Water (1 mL) was added. The mixture was cooled to 0°C and stirred for 30 min at this temperature. The precipitate was filtered, washed with EtOH (1 mL) and dried to afford the title compound as an off-white solid (0.018 g, 80% yield).
¹H NMR (*d6*-DMSO) δ: 10.98 (m, 1H); 9.21 (m, 1H); 8.29 (d, J = 0.9 Hz, 1H); 7.95 (d, J = 8.4 Hz, 1H); 7.62 (m, 1H); 7.52 (d, J = 8.1 Hz, 2H); 7.36 (d, J = 8.1 Hz, 2H); 5.26 (t, J = 6 Hz, 1H); 4.51 (d, J = 6 Hz, 2H); 3.15-3.25 (overlapped m, 1H); 3.05 (m, 3H); 2.86-3.00 (overlapped m, 1H); 2.68-2.83 (m, 1H); 2.16-2.30 (m, 1H); 1.55 (s, 3H).
MS (ESI, m/z): 459.13 [M+H⁺] for C₂₂H₂₂N₂O₅S₂; t_{R} = 0.71 min.

### Reference Example 3: (RS)-4-(6-((4-(3-aminooxetan-3-yl)phenyl)ethynyl)-benzo[d]thiazol-2-yl)-N-hydroxy-2-methyl-2-(methylsulfonyl)butanamide 4-methylbenzenesulfonate:

Starting from the compound of Preparation E (0.1 g, 0.229 mmol) and the compound of Preparation D (0.070 g, 0.228 mmol) and proceeding in analogy to Reference Example 2, steps RE2.i and RE2.iv (yields: Sonogashira coupling 71%; deprotection 46%), the title compound was obtained as a yellow solid (0.050 g).
¹H NMR (*d6*-DMSO) δ: 11.01 (s, 1H); 9.24 (s, 1H); 8.35 (s, 1H); 8.00 (d, J = 8.4 Hz, 1H); 7.56-7.76 (m, 5H); 7.47 (d, J = 7.9 Hz, 2H); 7.11 (d, J = 7.9 Hz, 2H); 4.87-4.99 (m, 4H); 3.21-3.30 (overlapped m, 1H); 3.07 (s, 3H); 2.91-3.03 (m, 1H); 2.71-2.84 (m, 1H); 2.18-2.28 (overlapped m, 1H); 2.30 (s, 3H); 1.57 (m, 3H).
MS (ESI, m/z): 541.1 [M+CH₃CN⁺] for C₂₄H₂₅N₃O₅S₂; t_{R} = 0. 46 min.

### Reference Example 4: (RS)-N-hydroxy-4-(6-(5-hydroxypenta-1,3-diyn-1-yl)benzo[d]thiazol-2-yl)-2-methyl-2-(methylsulfonyl)butanamide:

Starting from the compound of Preparation E (0.3 g, 0.687 mmol) and 3-iodoprop-2-yn-1-ol (0.175 g, 0.962 mmol) and proceeding in analogy to Reference Example 2, steps RE2.i and RE2.iv (yields: Sonogashira coupling 23%; deprotection 61%), the title compound was obtained as a beige solid (0.015 g).
¹H NMR (*d6*-DMSO) *δ*: 11.03 (s, 1H), 9.26 (s, 1H), 8.37 (s, 1H), 7.97 (d, J = 8.4 Hz, 1H), 7.65 (d, J = 8.4 Hz, 1H), 5.50 (t, J = 5.8 Hz, 1H), 4.28 (d, J = 5.8 Hz, 2H), 3.24-3.32 (m, 1H), 3.08 (s, 3H), 2.92-3.02 (m, 1H), 2.74-2.82 (m, 1H), 2.21-2.31 (m, 1H), 1.56 (s, 3H). MS (ESI, m/z): 406.7 [M+H⁺] for C₂₀H₂₂N₂O₅S₂; t_{R} = 0.66 min.

### EXAMPLES OF COMPOUNDS ACCORDING TO THE INVENTION:

### Example 1: (R)-N-hydroxy-4-(6-((3-hydroxyoxetan-3-yl)buta-1,3-diyn-1-yl)benzo[d]thiazol-2-yl)-2-methyl-2-(methylsulfonyl)butanamide:

### 1.i. (RS)-4-(6-((3-hydroxyoxetan-3-yl)buta-1,3-diyn-1-yl)benzo[d]thiazol-2-yl)-2-methyl-2-(methylsulfonyl)-N-((tetrahydro-2H-pyran-2-yl)oxy)butanamide:

Starting from the compound of Preparation E (0.5 g, 1.15 mmol) and the compound of Preparation F (0.282 g, 1.26 mmol) and proceeding in analogy to Reference Example 2, steps RE2.i and RE2.iv (yields: Sonogashira coupling 51%; deprotection 78%), the title compound (0.197 g) was obtained as a grey solid.
¹H NMR (*d6*-DMSO) δ: 11.01 (s, 1H); 9.14-9.29 (m, 1H); 8.37-8.40 (m, 1H); 7.98 (d, J = 8.4 Hz, 1H); 7.67 (dd, J = 1.5, 8.4 Hz, 1H); 6.66-6.85 (m, 1H); 4.70-4.75 (m, 2H); 4.53-4.59 (m, 2H); 3.20-3.31 (m, 1H); 3.07 (s, 3H); 2.91-3.02 (overlapped m, 1H); 2.70-2.83 (m, 1H); 2.19-2.32 (m, 1H); 1.56 (s, 3H).
MS (ESI, m/z): 449.1 [M+H⁺] for C₂₀H₂₀N₂O₆S₂; t_{R} = 0.66 min.

### 1.ii. (R)-N-hydroxy-4-(6-((3-hydroxyoxetan-3-yl)buta-1,3-diyn-1-yl)benzo[d]thiazol-2-yl)-2-methyl-2-(methylsulfonyl)butanamide:

Intermediate 1.i (0.197 g) was separated by semi-preparative chiral HPLC Method A (DCM-MeOH-TFA-DEA 10-90-0.09-0.036; flow rate: 20 mL/min; UV detection at 297 nM); the respective retention times (flow rate: 1 mL/min) were 4.98 and 7.27 min. The title (*R*)-enantiomer, identified as the first eluting compound, was obtained as a beige solid (0.053 g).
¹H NMR (*d6*-DMSO) δ: 11.01 (s, 1H); 9.14-9.29 (m, 1H); 8.37-8.40 (m, 1H); 7.98 (d, J = 8.4 Hz, 1H); 7.67 (dd, J = 1.5, 8.4 Hz, 1H); 6.66-6.85 (m, 1H); 4.73 (d, J = 6.7 Hz, 2H); 4.56 (d, J = 6.7 Hz, 2H); 3.20-3.31 (m, 1H); 3.07 (s, 3H); 2.91-3.02 (overlapped m, 1H); 2.70-2.83 (m, 1H); 2.19-2.32 (m, 1H); 1.56 (s, 3H).
MS (ESI, m/z): 449.1 [M+H⁺] for C₂₀H₂₀N₂O₆S₂; t_{R} = 0.66 min.

### Example 2: (R)-4-(6-(2-fluoro-4-methoxyphenyl)benzo[d]thiazol-2-yl)-N-hydroxy-2-methyl-2-(methylsulfonyl)butanamide:

Starting from the compound of Preparation G (0.15 g, 0.305 mmol) and 2-fluoro-4-methoxyphenylboronic acid (0.078 g, 0.458 mmol; commercial) and proceeding in analogy to Reference Example 1, the title compound was obtained, after purification by prep-HPLC (Method 2), as a white solid (0.104 g, 74% yield).
¹H NMR (*d6*-DMSO) δ: 11.00 (s, 1H); 9.27 (s, 1H); 8.21 (s, 1H); 8.01 (d, J = 8.5 Hz, 1H); 7.62 (dt, J = 1.7, 8.5 Hz, 1H); 7.53 (t, J = 9.0 Hz, 1H); 6.98 (dd, J = 2.5, 13.0 Hz, 1H); 6.92 (dd, J = 2.5, 8.6 Hz, 1H); 3.83 (s, 3H); 3.20-3.30 (m, 1H); 3.08 (s, 3H); 2.89-3.00 (m, 1H); 2.73-2.83 (m, 1H); 2.20-2.30 (m, 1H); 1.56 (s, 3H).
MS (ESI, m/z): 453.1 [M+H⁺] for C₂₀H₂₁N₂O₅FS₂; t_{R}= 0.78 min.

### Example 3: (R)-N-hydroxy-4-(6-((4-(3-hydroxyoxetan-3-yl)phenyl)ethynyl)benzo[d]thiazol-2-yl)-2-methyl-2-(methylsulfonyl)butanamide:

Starting from the compound of Preparation I (0.060 g, 0.137 mmol) and the compound of Preparation J (0.053 g, 0.194 mmol) and proceeding in analogy to Reference Example 2, steps RE2.i and RE2.iv (yields: Sonogashira coupling 65%; deprotection 55%), the title product was obtained as an orange solid (0.024 g).
¹H NMR *(d6-* DMSO) δ: 11.04 (s, 1H); 9.27 (s, 1H); 8.34 (s, 1H); 7.99 (d, J = 8.4 Hz, 1H); 7.66 (m, 5H); 6.49 (s, 1H); 4.80 (d, J = 6.4 Hz, 2H); 4.69 (d, J = 6.4 Hz, 2H); 3.23-3.31 (m, 1H); 3.09 (s, 3H); 2.92-3.01 (m, 1H); 2.79 (td, J = 4.3, 12.5 Hz, 1H); 2.20-2.29 (m, 1H); 1.57 (s, 3H).
MS (ESI, m/z): 500.8 [M+H⁺] for C₂₀H₂₂N₂O₅S₂; t_{R} = 0.70 min.

### Example 4: (R)-N-hydroxy-4-(6-(5-hydroxy-5-methylhexa-1,3-diyn-1-yl)benzo[d]thiazol-2-yl)-2-methyl-2-(methylsulfonyl)butanamide:

Starting from the compound of Preparation H (0.060 g, 0.137 mmol) and the compound of Preparation J (0.034 g, 0.165 mmol) and proceeding in analogy to Reference Example 2, steps RE2.i and RE2.iv (yields: Sonogashira coupling 56%; deprotection 42%), the title compound was obtained, after purification by prep-HPLC (Method 2), as a yellow solid (0.014 g).
¹H NMR (*d6*-DMSO) *δ*: 10.85-11.29 (m, 1H); 9.20-9.32 (m, 1H); 8.33-8.38 (m, 1H); 7.97 (d, J = 8.4 Hz, 1H); 7.65 (d, J = 8.4 Hz, 1H); 5.69 (s, 1H); 3.23-3.31 (m, 1H); 3.08 (s, 3H); 2.91-3.01 (m, 1H); 2.73-2.82 (m, 1H); 2.21-2.30 (m, 1H); 1.56 (s, 3H); 1.44 (s, 6H). MS (ESI, m/z): 434.9 [M+H⁺] for C₂₀H₂₂N₂O₅S₂; t_{R} = 0.71 min.

### Example 5: (R)-4-(6-((S)-5,6-dihydroxyhexa-1,3-diyn-1-yl)benzo[d]thiazol-2-yl)-N-hydroxy-2-methyl-2-(methylsulfonyl)butanamide:

Starting from the compound of Preparation H (0.060 g, 0.137 mmol) and the compound of Preparation K (0.040 g, 0.192 mmol) and proceeding in analogy to Reference Example 2, steps RE2.i and RE2.iv (yields: Sonogashira coupling 80%; deprotection 14%), the title compound was obtained, after purification by prep-HPLC (Method 2), as a yellowish foam (0.008 g).
¹H NMR (*d6-* DMSO) δ: 10.5-11.3 (s, 1H); 9.09-9.45 (s, 1H); 8.37 (s, 1H); 7.97 (d, J = 8.5 Hz, 1H); 7.65 (d, J = 8.5 Hz, 1H); 5.73 (d, J = 6 Hz, 1H); 5.08 (t, J = 6 Hz, 1H); 4.36 (q, J = 5.8 Hz, 1H); 3.48 (t, J = 5.8 Hz, 2H); 3.24-3.30 (m, 1H); 3.06 (s, 3H); 2.92-3.01 (m, 1H); 2.73-2.81 (m, 1H); 2.25 (d, J = 4.8 Hz, 1H); 1.56 (s, 3H).
MS (ESI, m/z): 436.9 [M+H⁺] for C₁₉H₂₀N₂O₆S₂; t_{R} = 0.59 min.

### Example 6: (R)-4-(6-(5-amino-5-methylhexa-1,3-diyn-1-yl)benzo[d]thiazol-2-yl)-N-hydroxy-2-methyl-2-(methylsulfonyl)butanamide:

Starting from the compound of Preparation H (0.100 g, 0.229 mmol) and the compound of Preparation L (0.060 g, 0.287 mmol) and proceeding in analogy to Reference Example 2, steps RE2.i and RE2.iv (yields: Sonogashira coupling 47%; deprotection 56%), the title compound was obtained after precipitation in water, as a beige solid (0.026 g).
¹H NMR (*d6-* DMSO) δ: 9.33 (s, 1H); 8.32 (s, 1H); 7.96 (d, J = 8.5 Hz, 1H); 7.62 (d, J = 8.5 Hz, 1H); 3.22-3.32 (m, 1H); 3.07 (s, 3H); 2.92-3.0 (m, 1H); 2.71-2.80 (m, 1H); 2.20-2.29 (m, 1H); 1.56 (s, 3H); 1.34 (s, 6H).
MS (ESI, m/z): 416.9 [M+H⁺] for C₂₀H₂₃N₃O₄S₂; t_{R} = 0.56 min.

### Example 7: (R)-N-hydroxy-4-(6-(((1S,2S)-2-(hydroxymethyl)cyclopropyl)buta-1,3-diyn-1-yl)benzo[d]thiazol-2-yl)-2-methyl-2-(methylsulfonyl)butanamide:

### 7.i. ((1S,2S)-2-((2-(3-methyl-3-(methylsulfonyl)-4-oxo-4-(((tetrahydro-2H-pyran-2-yl)oxy)amino)butyl)benzo[d]thiazol-6-yl)buta-1,3-diyn-1-yl)cyclopropyl)methyl acetate:

Starting from the compound of Preparation H (0.3 g, 0.687 mmol) and the compound of Preparation M ((*S*,*S*)-enantiomer, 156 mg, 0.719 mmol) and proceeding in analogy to Reference Example 2, step RE2.i, the title compound was obtained, after purification by CC (Hept-EA), as a white solid (0.126 g, 32% yield).
¹H NMR (*d6*-DMSO) δ: 11.43 (m, 1H); 8.32 (s, 1H); 7.95 (d, J = 8.5 Hz, 1H); 7.61 (dd, J = 1.2, 8.4 Hz, 1H); 4.94-4.97 (m, 1H); 3.96-4.14 (m, 2H); 3.84 (dd, J = 7.6, 11.7 Hz, 1H); 3.46-3.53 (m, 1H); 3.24-3.30 (m, 1H); 3.08 (s, 1.5H); 3.06 (s, 1.5H); 2.96-3.05 (m, 1H); 2.76-2.81 (m, 1H); 2.22-2.31 (m, 1H); 2.05 (s, 3H); 1.50-1.71 (m, 9H); 1.22-1.29 (m, 1H); 1.05-1.10 (m, 1H); 0.95-1.00 (m, 1H); 0.84-0.88 (t, J = 6.7 Hz, 1H).
MS (ESI, m/z): 572.9 [M+H⁺] for C₂₈H₃₂N₂O₇S₂; t_{R} = 0.94 min.

### 7.ii. 4-(6-(((1S,2S)-2-(hydroxymethyl)cyclopropyl)buta-1,3-diyn-1-yl)benzo[d]thiazol-2-yl)-2-methyl-2-(methylsulfonyl)-N-((tetrahydro-2H-pyran-2-yl)oxy)butanamide:

To a solution of intermediate 7.i (0.100 g, 0.175 mmol) in MeOH (1.1 mL) was added K₂CO₃ (0.048 g, 0.349 mmol). The suspension was stirred at rt for 40 min. The mixture was diluted with DCM (20 mL) and washed with 15% aq. NaHSO₄ solution (10 mL). The aq. layer was extracted with DCM-MeOH (9:1, 2 x 20 mL). The combined org. layers were dried over MgSO₄ and concentrated to dryness. The crude residue was purified by CC (DCM-MeOH) to afford the title compound as a yellow oil (0.089 g, 97% yield).
¹H NMR (*d6*-DMSO) δ: 11.44 (s, 1H); 8.31 (s, 1H); 7.94 (d, J = 8.5 Hz, 1H); 7.59-7.60 (m, 1H); 4.91-4.97 (s, 1H); 4.72 (t, J = 5.7 Hz, 1H); 4.05-4.11 (m, 1H); 3.41-3.51 (m, 2H); 3.22-3.29 (m, 2H); 3.08 (s, 1.5H); 3.06 (s, 1.5H); 2.97-3.05 (m, 1H); 2.69-2.80 (m, 1H); 2.20-2.30 (m, 1H); 1.40-1.76 (m, 11H); 0.92-0.97 (m, 1H); 0.91 (m, 1H).
MS (ESI, m/z): 530.9 [M+H⁺] for C₂₆H₃₀N₂O₆S₂; t_{R} = 0.85 min.

### 7.iii. (R)-N-hydroxy-4-(6-(((1S,2S)-2-(hydroxymethyl)cyclopropyl)buta-1,3-diyn-1-yl)benzo[d]thiazol-2-yl)-2-methyl-2-(methylsulfonyl)butanamide:

Starting from intermediate 7.ii (0.090 g, 0.169 mmol) and proceeding in analogy to Reference Example 2, step RE2.iv, the title compound was obtained, after precipitation in water, as a yellow solid (0.048 g, 64% yield).
¹H NMR (*d6*-DMSO) δ: 11.03 (s, 1H); 9.26 (s, 1H); 8.31 (s, 1H); 7.94 (d, J = 8.5 Hz, 1H); 7.61 (d, J = 8.5 Hz, 1H); 4.72 (t, J = 5.7 Hz, 1H); 3.40-3.46 (m, 1H); 3.23-3.30 (m, 2H); 3.08 (s, 3H); 2.90-2.99 (m, 1H); 2.72-2.80 (m, 1H); 2.20-2.27 (m, 1H); 1.56 (s, 3H); 1.40-1.48 (m, 2H); 0.93-0.96 (m, 1H); 0.84-0.90 (m, 1H).
MS (ESI, m/z): 446.9 [M+H⁺] for C₂₁H₂₂N₂O₅S₂; t_{R} = 0.71 min.

### Example 8: (R)-N-hydroxy-4-(6-((1-(hydroxymethyl)cyclopropyl)buta-1,3-diyn-1-yl)benzo[d]thiazol-2-yl)-2-methyl-2-(methylsulfonyl)butanamide:

### 8.i. (R)-4-(6-((1-(((tert-butyldiphenylsilyl)oxy)methyl)cyclopropyl)buta-1,3-diyn-1-yl)benzo[d]thiazol-2-yl)-2-methyl-2-(methylsulfonyl)-N-((tetrahydro-2H-pyran-2-yl)oxy)butanamide:

To a solution of *n*-butylamine (0.116 mL, 2.61 mmol) in water (0.2 mL) was added CuCl (0.061 g, 0.062 mmol). Then, NH₂OH.HCl (0.060 g, 0.852 mmol) was added, followed by the compound of Preparation H (0.100 g, 0.237 mmol). The resulting suspension was immediately cooled with an ice bath. *n*-Butylamine (0.116 mL, 2.37 mmol) was added. The compound of Preparation N (0.503 g, 1.24 mmol) was added at once and the ice bath was removed. The mixture was stirred at rt for 4 h. MgSO₄ was added followed by EA (50 mL). MgSO₄ was removed by filtration and the filtrate was concentrated to dryness. The residue was purified by CC (Hept-EA) to afford the title compound as a yellow foam (0.124 g, 69% yield).
¹H NMR (*d6*-DMSO) δ: 11.43 (s, 1H); 8.34 (s, 1H); 7.94-7.99 (m, 1H); 7.67 (m, 4H); 7.60-7.62 (m, 1H); 7.42-7.51 (m, 6H); 4.95-4.98 (m, 1H); 3.96-4.00 (overlapped m, 1H); 3.65 (s, 2H); 3.47-3.52 (m, 1H); 3.24-3.32 (m, 1H); 3.07 (m, 1.5H); 3.06 (s, 1.5H); 2.97-3.06 (m, 1H); 2.73-2.82 (m, 1H); 2.24-2.30 (m, 1H); 1.50-1.76 (m, 9H); 0.97-1.06 (m, 11H); 0.84-0.90 (m, 2H).
MS (ESI, m/z): 768.9 [M+H⁺] for C₄₂H₄₈N₂O₆S₂Si; t_{R} = 1.15 min.

### 8.ii. (2R)-4-(6-((1-(hydroxymethyl)cyclopropyl)buta-1,3-diyn-1-yl)benzo[d]thiazol-2-yl)-2-methyl-2-(methylsulfonyl)-N-((tetrahydro-2H-pyran-2-yl)oxy)butanamide:

To a solution of intermediate 8.i (56 mg, 0.073 mmol) in THF (1 mL) was added TBAF (1M in THF, 0.2 mL). The mixture was stirred at rt for 1 h. The mixture was impregnated on silica and concentrated to dryness. The residue was purified by CC (Hept-EA) to afford the title compound as a yellow oil (10 mg, 26% yield).
MS (ESI, m/z): 530.9 [M+H⁺] for C₂₆H₃₀N₂O₆S₂; t_{R} = 0.84 min.

### 8.iii. (R)-N-hydroxy-4-(6-((1-(hydroxymethyl)cyclopropyl)buta-1,3-diyn-1-yl)benzo[d]thiazol-2-yl)-2-methyl-2-(methylsulfonyl)butanamide:

Starting from intermediate 8.ii (10 mg, 0.0188 mmol) and proceeding in analogy to Reference Example 2, step RE2.iv, the title compound was obtained, after precipitation in water, as a beige solid (2.6 mg, 31% yield).
¹H NMR (*d6*-DMSO) δ: 11.03 (s, 1H); 9.26 (s, 1H); 8.31 (s, 1H); 7.95 (d, J = 8.4 Hz, 1H); 7.61 (d, J = 8.4 Hz, 1H); 5.06 (t, J = 6.0 Hz, 1H); 3.40 (overlapped m, 2H); 3.23-3.29 (m, 1H); 3.07 (s, 3H); 2.91-2.99 (m, 1H); 2.77 (td, J = 4.5, 12.5 Hz, 1H); 2.25 (td, J = 4.5, 12.5 Hz, 1H); 1.55 (s, 3H); 0.88-0.97 (m, 4H).
MS (ESI, m/z): 446.9 [M+H⁺] for C₂₅H₃₀N₂O₆S₂; t_{R} = 0.72 min.

### Example 9: (R)-4-(6-((3-aminooxetan-3-yl)buta-1,3-diyn-1-yl)benzo[d]thiazol-2-yl)-N-hydroxy-2-methyl-2-(methylsulfonyl)butanamide:

Starting from the compound of Preparation H (0.168 g, 0.385 mmol) and the compound of Preparation O (0.124 g, 0.385 mmol) and proceeding successively in analogy to Reference Example 2, step RE2.i (42% yield) and to Preparation K, step K.iii (2% yield), the title compound was obtained, after purification by prep-HPLC (Method 1), as a white solid (0.0013 g, 2% yield).
¹H NMR (*d6*-DMSO) δ: 11.02 (br. s, 1H); 8.87 (br. s, 1H); 8.37 (s, 1H); 7.97 (d, J = 8.0 Hz, 1H); 7.66 (d, J = 8.0 Hz, 1H); 4.68 (d, J = 4.1 Hz, 2H); 4.46 (d, J = 4.5 Hz, 2H); 3.20-3.48 (overlapped m, 2H); 3.07 (s, 3H); 2.67-2.81 (m, 2H); 1.50 (s, 3H).
MS (ESI, m/z): 488.9 [M+MeCN+H⁺] for C₂₀H₂₁N₃O₅S₂; t_{R} = 0.51 min.

### Example 10: (R)-4-(6-((1-aminocyclopropyl)buta-1,3-diyn-1-yl)benzo[d]thiazol-2-yl)-N-hydroxy-2-methyl-2-(methylsulfonyl)butanamide hydrochloride:

Starting from the compound of Preparation H (0.181 g, 0.415 mmol) and the compound of Preparation P (0.131 g, 0.539 mmol) and proceeding successively in analogy to Reference Example 2, step RE2.i (quant.) and to Preparation K, step K.iii (30% yield), the title compound was obtained, after purification by prep-HPLC (Method 1), as a beige solid (0.042 g).
¹H NMR (*d6*-DMSO) δ: 10.99-11.09 (m, 1H); 9.22-9.31 (m, 1H); 8.60-8.99 (m, 3H); 8.37-8.43 (m, 1H); 7.99 (d, J = 8.4 Hz, 1H); 7.67 (d, J = 8.4 Hz, 1H); 3.24-3.32 (m, 1H); 3.08 (s, 3H); 2.93-3.02 (m, 1H); 2.73-2.83 (m, 1H); 2.21-2.31 (m, 1H); 1.56 (s, 3H); 1.34-1.44 (m, 4H).
MS (ESI, m/z): 472.9 [M+MeCN+H⁺] for C₂₀H₂₂N₃O₄ClS₂; t_{R} = 0.53 min.

### Example 11: (R)-N-hydroxy-4-(6-((4-(1-(hydroxymethyl)cyclopropyl)phenyl)ethynyl)benzo[d]thiazol-2-yl)-2-methyl-2-(methylsulfonyl)butanamide:

Starting from the compound of Preparation H (0.100 g, 0.229 mmol) and the compound of Preparation Q (0.063 g, 0.229 mmol) and proceeding successively in analogy to Reference Example 2, step RE2.i (53% yield) and step RE2.iv (66% yield), the title product was obtained, after purification by prep-HPLC (Method 2), as a yellow solid (0.040 g).
¹H NMR (*d6*-DMSO) δ: 11.03 (br. s, 1H); 9.26 (br. s, 1H); 8.32 (d, J = 1.3 Hz, 1H); 7.98 (d, J = 8.5 Hz, 1H); 7.64 (dd, J = 1.7, 8.4 Hz, 1H); 7.48 (d, J = 8.4 Hz, 2H); 7.36 (d, J = 8.4 Hz, 2H); 4.74 (t, J = 5.6 Hz, 1H); 3.57 (d, J = 5.6 Hz, 2H); 3.23-3.31 (m, 1H); 3.09 (s, 3H); 2.93-3.01 (m, 1H); 2.78 (td, J = 4.4, 12.7 Hz, 1H); 2.26 (td, J = 5.0, 12.5 Hz, 1H); 1.57 (m, 3H); 0.88-0.91 (m, 2H); 0.78-0.81 (m, 2H).
MS (ESI, m/z): 499.0 [M+H⁺] for C₂₅H₂₆N₂O₅S₂; t_{R} = 0.77 min.

### Example 12: (R)-N-hydroxy-4-(6-((1-(hydroxymethyl)cyclobutyl)buta-1,3-diyn-1-yl)benzo[d]thiazol-2-yl)-2-methyl-2-(methylsulfonyl)butanamide:

### 12.i. (2R)-4-(6-((1-(hydroxymethyl)cyclobutyl)buta-1,3-diyn-1-yl)benzo[d]thiazol-2-yl)-2-methyl-2-(methylsulfonyl)-N-((tetrahydro-2H-pyran-2-yl)oxy)butanamide:

Starting from the compound of Preparation H (0.100 g, 0.229 mmol) and the compound of Preparation R (0.127 g, 0.3 mmol) and proceeding successively in analogy to Example 8, steps 8.i to 8.ii (yields: Cadiot coupling 79%; deprotection 83%), the title product was obtained, after purification by CC using (Hept-EA), as a yellowish foam (0.083 g).
¹H NMR (*d6*-DMSO)) δ: 11.43 (s, 1H); 8.33 (s, 1H); 7.96 (d, J = 8.4 Hz, 1H); 7.63 (dd, J = 1.6, 8.4 Hz, 1H); 5.20 (t, J = 5.8 Hz, 1H); 4.94-4.99 (m, 1H); 4.04-4.15 (m, 1H); 3.47-3.56 (m, 3H); 3.24-3.32 (m, 1H); 3.09 (s, 1.5H); 3.07 (s, 1.5H); 2.97-3.05 (m, 1H); 2.72-2.83 (m, 1H); 2.23-2.32 (m, 1H); 2.12-2.20 (m, 4H); 1.86-2.01 (m, 2H); 1.63-1.76 (m, 3H); 1.50-1.62 (m, 6H).
MS (ESI, m/z): 544.90 [M+H⁺] for C₂₇H₃₂N₂O₆S₂; t_{R} = 0.88 min.

### 12.ii. (R)-N-hydroxy-4-(6-((1-(hydroxymethyl)cyclobutyl)buta-1,3-diyn-1-yl)benzo[d]thiazol-2-yl)-2-methyl-2-(methylsulfonyl)butanamide:

To a solution of intermediate 12.i (0.0345 g, 0.0633 mmol) in EtOH (1 mL) was added amberlyst 15 (0.044 g). The mixture was stirred 1 h at 80°C. The solvent was evaporated in vacuo and the residue was taken in DMF (2 mL). The amberlyst was filtered and the filtrate was evaporated. The residue was taken up in water (1 mL) and filered to afford the title compound as a pale yellow solid (0.020 g, 70% yield).
¹H NMR (*d6*-DMSO)) δ: 11.03 (br. s, 1 H); 9.26 (br. s, 1H); 8.33 (d, J = 1.5 Hz, 1H); 7.96 (d, J = 8.4 Hz, 1H); 7.63 (dd, J = 1.5, 8.4 Hz, 1H); 5.20 (s, 1H); 3.51 (d, J = 5.8 Hz, 2H); 3.23-3.30 (m, 1H); 3.08 (s, 3H); 2.93-3.01 (m, 1H); 2.74-2.83 (m, 1H); 2.22-2.31 (m, 1H); 2.15 (t, J = 7.9 Hz, 4H); 1.86-2.04 (m, 2H); 1.56 (s, 3H).
MS (ESI, m/z): 460.97 [M+H⁺] for C₂₂H₂₄N₂O₅S₂; t_{R} = 0.76 min.

### Example 13: (R)-4-(6-(((1s,3R,4S)-3,4-dihydroxycyclopentyl)buta-1,3-diyn-1-yl)benzo[d]thiazol-2-yl)-N-hydroxy-2-methyl-2-(methylsulfonyl)butanamide:

Starting from the compound of Preparation H (0.100 g, 0.229 mmol) and the compound of Preparation S (0.061 g, 0.3 mmol) and proceeding successively in analogy to Example 8, step 8.i and Example 12, step 12.ii (yields: Cadiot coupling 46%; deprotection 17%), the title product was obtained, after purification by prep-HPLC (Method 2), as a yellow solid (0.009 g).
¹H NMR (*d6*-DMSO)) δ: 10.54-11.22 (m, 1H); 9.11-9.51 (m, 1H); 8.32 (d, J = 1.6 Hz, 1H); 7.95 (d, J = 8.5 Hz, 1H); 7.60-7.64 (m, 1H); 4.55-4.59 (m, 2H); 3.94-4.00 (m, 2H); 3.23-3.30 (m, 1H); 3.14-3.22 (m, 1H); 3.08 (s, 3H); 2.92-3.00 (m, 1H); 2.73-2.81 (m, 1H); 2.20-2.30 (m, 1H); 1.90-1.99 (m, 2H); 1.76-1.83 (m, 2H); 1.56 (s, 3H).
MS (ESI, m/z): 476.96 [M+H⁺] for C₂₂H₂₄N₂O₆S₂; t_{R} = 0.65 min.

### Example 14: (R)-N-hydroxy-4-(6-(5-(3-hydroxyoxetan-3-yl)penta-1,3-diyn-1-yl)benzo[d]thiazol-2-yl)-2-methyl-2-(methylsulfonyl)butanamide:

Starting from the compound of Preparation H (0.08 g, 0.18 mmol) and the compound of Preparation T (0.056 g, 0.23 mmol) and proceeding successively in analogy to Example 8, step 8.i and Example 12, step 12.ii (yields: Cadiot coupling 55%; deprotection 16%), the title product was obtained, after purification by prep-HPLC (Method 2), as a yellow solid (0.008 g).
¹H NMR (*d6*-DMSO) δ: 11.02 (m, 1H); 9.25 (s, 1H); 8.35 (d, J = 1.5 Hz, 1H); 7.96 (d, J = 8.5 Hz, 1H); 7.64 (dd, J = 1.6, 8.4 Hz, 1H); 6.10 (s, 1H); 4.47 (d, J = 6.6 Hz, 2H); 4.43 (d, J = 6.7 Hz, 2H); 3.22-3.31 (m, 1H); 3.08 (s, 3H); 2.92-3.02 (m, 1H); 2.89 (s, 2H), 2.71-2.83 (m, 1H); 2.20-2.30 (m, 1H); 1.56 (s, 3H).
MS (ESI, m/z): 462.92 [M+H⁺] for C₂₁H₂₂N₂O₆S₂; t_{R} = 0.65 min.

### Example 15: (R)-N-hydroxy-4-(6-(((1R,2R)-2-(hydroxymethyl)-1-methylcyclopropyl)buta-1,3-diyn-1-yl)benzo[d]thiazol-2-yl)-2-methyl-2-(methylsulfonyl)butanamide:

Starting from the compound of Preparation H (0.100 g, 0.229 mmol) and the compound of Preparation U (0.057 g, 0.24 mmol) and proceeding successively in analogy to Example 8, steps 8.i to 8.iii (yields: Cadiot coupling 78%; deprotections 26%), the title product was obtained, after purification by prep-HPLC (Method 2), as a yellow solid (0.022 g).
¹H NMR (*d6*-DMSO)) δ: 11.03 (d, J = 0.7 Hz, 1H); 9.26 (d, J = 1.2 Hz, 1H); 8.31 (d, J = 1.5 Hz, 1H); 7.95 (d, J = 8.6 Hz, 1H); 7.61 (dd, J = 1.6, 8.4 Hz, 1H); 4.69 (t, J = 5.4 Hz, 1H); 3.60-3.66 (m, 1H); 3.33 (s, 3H); 3.23-3.31 (m, 2H); 3.08 (s, 3H); 2.92-3.00 (m, 1H); 2.75-2.81 (m, 1H); 2.21-2.30 (m, 1H); 1.56 (s, 3H); 1.42-1.50 (m, 1H); 1.11-1.16 (m, 1H); 0.62-0.68 (m, 1H).
MS (ESI, m/z): 460.96 [M+H⁺] for C₂₂H₂₄N₂O₅S₂; t_{R} = 0.74 min.

### Example 16: (R)-4-(6-(((2S,5R)-5-aminotetrahydro-2H-pyran-2-yl)buta-1,3-diyn-1-yl)benzo[d]thiazol-2-yl)-N-hydroxy-2-methyl-2-(methylsulfonyl)butanamide:

Starting from the compound of Preparation H (0.08 g, 0.183 mmol) and the compound of Preparation V (0.127 g, 0.3 mmol) and proceeding successively in analogy to Example 8, step 8.i and Example 12, step 12.ii (yields: Cadiot coupling 59%; deprotection 9%), the title product was obtained, after purification by CC (DCM-MeOH containing 1% aq. NH₄OH), as a white solid (0.005 g).
¹H NMR (*d6*-DMSO)) δ: 8.38 (d, J = 1.3 Hz, 1H); 7.97 (d, J = 8.5 Hz, 1H); 7.66 (dd, J = 1.6, 8.5 Hz, 1H); 4.31 (dd, J = 2.5, 10.0 Hz, 1H); 3.76-3.82 (m, 1H); 3.22-3.32 (m, 1H); 3.08 (s, 3H); 2.94-3.03 (m, 2H); 2.70-2.83 (m, 1H); 2.60-2.68 (m, 1H); 2.17-2.30 (m, 1H); 1.86-1.98 (m, 2H); 1.57-1.70 (m, 1H); 1.55 (s, 3H); 1.19-1.32 (m, 1H).
MS (ESI, m/z): 516.96 [M+H⁺] for C₂₂H₂₅N₃O₅S₂; t_{R} = 0.56 min.

### Example 17: (R)-4-(6-(5-(dimethylamino)penta-1,3-diyn-1-yl)benzo[d]thiazol-2-yl)-N-hydroxy-2-methyl-2-(methylsulfonyl)butanamide:

Starting from the compound of Preparation H (0.1 g, 0.23 mmol) and the compound of Preparation W (0.06 g, 0.3 mmol) and proceeding successively in analogy to Example 8, step 8.i and Example 12, step 12.ii (yields: Cadiot coupling 67%; deprotection 11%), the title product was obtained, after purification by prep-HPLC (Method 2), as a yellow beige solid (0.007 g).
¹H NMR *(d6*-DMSO) δ: 10.02 (br. s., 1 H); 9.28 (br. s., 1H); 8.38 (d, J = 1.5 Hz, 1H); 7.97 (d, J = 8.5 Hz, 1H); 7.66 (dd, J = 1.5, 8.5 Hz, 1H); 3.24-3.33 (m, 3H); 3.33 (s, 3H); 2.91-3.03 (m, 1H); 2.74-2.84 (m, 1H); 2.24-2.30 (m, 1H); 2.23 (s, 6H); 1.56 (s, 3H).
MS (ESI, m/z): 433.95 [M+H⁺] for C₂₀H₂₃N₃O₄S₂; t_{R} = 0.53 min.

### Example 18: (R)-N-hydroxy-4-(6-((4-hydroxytetrahydro-2H-pyran-4-yl)buta-1,3-diyn-1-yl)benzo[tl]thiazol-2-yl)-2-methyl-2-(methylsulfonyl)butanamide:

Starting from the compound of Preparation H (0.08 g, 0.18 mmol) and the compound of Preparation X (0.06 g, 0.24 mmol) and proceeding successively in analogy to Example 8, step 8.i and Reference Example RE2, step RE2.iv (yields: Cadiot coupling 64%; deprotection 76%), the title product was obtained, after precipitation in water and filtration, as a white solid (0.049 g).
¹H NMR *(d6*-DMSO) δ: 11.03 (d, J = 1.6 Hz, 1H); 9.26 (d, J = 1.6 Hz, 1H); 8.38 (d, J = 1.3 Hz, 1H); 7.98 (d, J = 8.5 Hz, 1H); 7.67 (dd, J = 1.6, 8.5 Hz, 1H); 5.96 (s, 1H); 3.72-3.84 (m, 2H); 3.48-3.57 (m, 2H); 3.24-3.31 (m, 1H); 3.08 (s, 3H); 2.92-3.01 (m, 1H); 2.78 (td, J = 4.5, 12.5 Hz, 1H); 2.26 (td, J = 5.0, 12.5 Hz, 1H); 1.82-1.90 (m, 2H); 1.65-1.74 (m, 2H); 1.56 (s, 3H).
MS (ESI, m/z): 476.95 [M+H⁺] for C₂₂H₂₄N₂O₆S₂; t_{R} = 0.68 min.

### Example 19: (R)-N-hydroxy-2-methyl-2-(methylsulfonyl)-4-(6-(piperidin-4-ylbuta-1,3-diyn-1-yl)benzo[d]thiazol-2-yl)butanamide:

Starting from the compound of Preparation H (0.08 g, 0.18 mmol) and the compound of Preparation Y (0.065 g, 0.24 mmol) and proceeding successively in analogy to Example 8, step 8.i and Reference Example RE2, step RE2.iv (yields: Cadiot coupling 56%; deprotection 32%), the title product was obtained, after precipitation in water and filtration, as a white solid (0.015 g).
¹H NMR *(d6*-DMSO) δ: 8.33 (d, J = 1.4 Hz, 1H); 7.95 (d, J = 8.5 Hz, 1H); 7.63 (dd, J = 1.6, 8.4 Hz, 1H); 3.20-3.29 (m, 1H); 3.07 (s, 3H); 2.94-3.03 (m, 1H); 2.82-2.92 (m, 2H); 2.70-2.78 (m, 2H); 2.50 (overlapped m, 2H); 2.18-2.26 (m, 1H); 1.72-1.80 (m, 2H); 1.52 (s, 3H); 1.42-1.51 (m, 2H).
MS (ESI, m/z): 500.9 [M+H⁺] for C₂₂H₂₅N₃O₄S₂; t_{R} = 0.57 min.

### Example 20: (R)-N-hydroxy-4-(6-((3-(hydroxymethyl)oxetan-3-yl)buta-1,3-diyn-1-yl)benzo[d]thiazol-2-yl)-2-methyl-2-(methylsulfonyl)butanamide:

Starting from the compound of Preparation H (0.08 g, 0.18 mmol) and the compound of Preparation Z (0.088 g, 0.183 mmol) and proceeding successively in analogy to Example 8, steps 8.i to 8.iii (yields: Cadiot coupling 66%; deprotections 37%), the title product was obtained, after precipitation in water and filtration, as a yellow solid (0.021 g).
¹H NMR *(d6*-DMSO) δ: 11.03 (br. s, 1H); 9.26 (br. s, 1H); 8.36-8.38 (m, 1H); 7.98 (d, J = 8.5 Hz, 1H); 7.66 (dd, J = 1.7, 8.4 Hz, 1H); 5.50 (t, J = 5.9 Hz, 1H); 4.62 (d, J = 5.7 Hz, 2H); 4.54 (d, J = 5.8 Hz, 2 H); 3.74 (d, J = 5.9 Hz, 2 H); 3.24-3.31 (m, 1H); 3.08 (s, 3H); 2.92-3.01 (m, 1H); 2.78 (td, J = 4.5, 12.6 Hz, 1H); 2.26 (td, J = 5.1, 12.4 Hz, 1H); 1.56 (s, 3H).
MS (ESI, m/z): 462.92 [M+H⁺] for C₂₁H₂₂N₂O₆S₂; t_{R} = 0.66 min.

### Example 21: (R)-N-hydroxy-4-(6-((cis-1-hydroxy-3-(hydroxymethyl)cyclobutyl)buta-1,3-diyn-1-yl)benzo[tl]thiazol-2-yl)-2-methyl-2-(methylsulfonyl)butanamide:

Starting from the compound of Preparation H (0.08 g, 0.18 mmol) and the compound of Preparation AA (0.088 g, 0.183 mmol) and proceeding successively in analogy to Example 8, step 8.i and Reference Example RE2, step RE2.iv (yields: Cadiot coupling 62%; deprotection 69%), the title product was obtained, after precipitation in water and filtration, as a yellow solid (0.033 g).
¹H NMR *(d6*-DMSO) δ: 11.02 (m, 1H); 9.24 (m, 1H); 8.34 (d, J = 1.3 Hz, 1H); 7.96 (d, J = 8.5 Hz, 1H); 7.64 (dd, J = 1.6, 8.4 Hz, 1H); 5.27 (s, 1H); 4.49 (t, J = 5.3 Hz, 1H); 3.37 (t, J = 5.8 Hz, 2H); 3.23-3.31 (m, 1H); 3.08 (s, 3H); 2.91-3.00 (m, 1H); 2.77 (td, J = 4.5, 12.5 Hz, 1H); 2.65 (s, 2H); 2.25 (td, J = 5.0, 12.4 Hz, 1H); 2.05-2.11 (m, 2H); 1.91-2.00 (m, 1H); 1.71-1.80 (m, 2H); 1.56 (s, 3H).
MS (ESI, m/z): 490.96 [M+H⁺] for C₂₃H₂₆N₂O₆S; t_{R} = 0.65 min.

### Example 22: (R)-N-hydroxy-4-(6-((1-(2-hydroxyacetyl)piperidin-4-yl)buta-1,3-diyn-1-yl)benzo[d]thiazol-2-yl)-2-methyl-2-(methylsulfonyl)butanamide:

Starting from the compound of Preparation H (0.08 g, 0.18 mmol) and the compound of Preparation AB (0.086 g, 0.29 mmol) and proceeding successively in analogy to Example 8, step 8.i and Reference Example RE2, step RE2.iv (yields: Cadiot coupling 54%; deprotection 63%), the title product was obtained, after precipitation in water and filtration, as a white foam (0.031 g).
¹H NMR *(d6*-DMSO) δ: 11.02 (m, 1H); 9.24 (m, 1H); 8.34 (d, J = 1.3 Hz, 1 H); 7.96 (d, J = 8.5 Hz, 1H); 7.64 (dd, J = 1.6, 8.4 Hz, 1H); 5.27 (s, 1H); 4.49 (t, J = 5.3 Hz, 1H); 3.37 (t, J = 5.8 Hz, 2H); 3.23-3.31 (m, 1H); 3.08 (s, 3H); 2.91-3.00 (m, 1H); 2.77 (td, J = 4.5, 12.5 Hz, 1H); 2.65 (s, 2H); 2.25 (td, J = 5.0, 12.4 Hz, 1H); 2.05-2.11 (m, 2H); 1.91-2.00 (m, 1H); 1.71-1.80 (m, 2H); 1.56 (s, 3H).
MS (ESI, m/z): 517.83 [M+H⁺] for C₂₁H₂₂N₂O₆S₂; t_{R} = 0.71 min.

### Example 23: (R)-4-(6-(((1R*,2R*)-1-fluoro-2-(hydroxymethyl)cyclopropyl)buta-1,3-diyn-1-yl)benzo[d]thiazol-2-yl)-N-hydroxy-2-methyl-2-(methylsulfonyl)butanamide:

Starting from the compound of Preparation H (0.1 g, 0.23 mmol) and the compound of Preparation AC (0.128 g, 0.3 mmol) and proceeding successively in analogy to Example 8, steps 8.i and 8.ii and Reference Example RE2, step RE2.iv (yields: Cadiot coupling 86%; TBAF 76%; THP deprotection 79%), the title product was obtained, after purification by prep-HPLC (Method 2), as a white foam (0.048 g).
¹H NMR *(d6*-DMSO) δ: 10.78-11.24 (br. s, 1H); 9.12-9.46 (br. s, 1H); 8.41 (d, J = 1.3 Hz, 1H); 7.99 (d, J = 8.5 Hz, 1H); 7.69 (dd, J = 1.7, 8.5 Hz, 1H); 4.91 (t, J = 5.6 Hz, 1H); 3.66-3.74 (m, 1H); 3.34-3.42 (m, 1 H); 3.24-3.32 (m, 1H); 3.08 (s, 3H); 2.93-3.02 (m, 1H); 2.77 (td, J = 4.5, 12.5 Hz, 1H); 2.26 (td, J = 5.0, 12.5 Hz, 1H); 1.66-1.75 (m, 1H); 1.56 (s, 3H); 1.40-1.47 (m, 1H); 1.26-1.34 (m, 1H).
MS (ESI, m/z): 464.92 [M+H⁺] for C₂₁H₂₁N₂O₅FS₂; t_{R} = 0.73 min.

### Example 24: (R)-4-(6-(((1R*,2R*)-2-fluoro-2-(hydroxymethyl)cyclopropyl)buta-1,3-diyn-1-yl)benzo[d]thiazol-2-yl)-N-hydroxy-2-methyl-2-(methylsulfonyl)butanamide:

Starting from the compound of Preparation H (0.1 g, 0.23 mmol) and the compound of Preparation AD (0.128 g, 0.3 mmol) and proceeding successively in analogy to Example 8, steps 8.i and 8.ii and Reference Example RE2, step RE2.iv (yields: Cadiot coupling 79%; TBAF deprotection 59%; THP deprotection 65%), the title product was obtained, after purification by prep-HPLC (Method 2), as a white foam (0.030 g).
¹H NMR *(d6*-DMSO) δ: 10.69-11.29 (br. s, 1H); 9.18-9.52 (br. s, 1H); 8.35 (d, J = 1.5 Hz, 1H); 7.96 (d, J = 8.5 Hz, 1H); 7.64 (dd, J = 1.5, 8.5 Hz, 1H); 5.26 (t, J = 6.1 Hz, 1H); 3.59-3.76 (m, 2H); 3.23-3.31 (m, 1H); 3.08 (s, 3H); 2.93-3.01 (m, 1H); 2.72-2.84 (m, 1H); 2.25 (td, J = 5.0, 12.5 Hz, 1H); 1.96-2.03 (m, 1H); 1.56 (s, 3H); 1.35-1.46 (m, 2H).
MS (ESI, m/z): 464.92 [M+H⁺] for C₂₁H₂₁N₂O₅FS₂; t_{R} = 0.71 min.

### Example 25: (R)-N-hydroxy-4-(6-((1-(2-hydroxyacetyl)azetidin-3-yl)buta-1,3-diyn-1-yl)benzo[d]thiazol-2-yl)-2-methyl-2-(methylsulfonyl)butanamide:

Starting from the compound of Preparation H (0.100 g, 0.229 mmol) and the compound of Preparation AE (0.065 g, 0.298 mmol) and proceeding successively in analogy to Example 8, step 8.i and Reference Example RE2, step RE2.iv (yields: Cadiot coupling 75%; deprotection 56%), the title product was obtained, after purification by prep-HPLC (Method 2), as a white solid (0.048 g).
¹H NMR *(d6*-DMSO) δ: 9.10-10.20 (br. s, 2H); 8.37 (d, J = 1.3 Hz, 1H); 7.97 (d, J = 8.5 Hz, 1H); 7.65 (dd, J = 1.6, 8.4 Hz, 1H); 5.03 (m, 1H); 4.48 (t, J = 8.8 Hz, 1H); 4.18-4.22 (m, 2H); 3.92 (s, 2H); 3.87 (m, 1H); 3.78 (m, 1H); 3.27 (m, 1 H); 3.08 (s, 3H); 2.97 (m, 1H); 2.77 (td, J = 4.4, 12.6 Hz, 1H); 2.25 (td, J = 5.0, 12.4 Hz, 1H); 1.56 (s, 3H).
MS (ESI, m/z): 489.99 [M+H⁺] for C₂₂H₂₃N₃O₆S₂; t_{R} = 0.65 min.

### Example 26: (R)-N-hydroxy-4-(6-(((1R,2S)-2-(hydroxymethyl)-2-methylcyclopropyl)buta-1,3-diyn-1-yl)benzo[d]thiazol-2-yl)-2-methyl-2-(methylsulfonyl)butanamide:

Starting from the compound of Preparation H (0.150 g, 0.344 mmol) and the compound of Preparation AF (0.103 g, 0.447 mmol) and proceeding successively in analogy to Example 8, step 8.i, Example 7, step 7.ii and Reference Example RE2, step RE2.iv (yields: Cadiot coupling 69%; deprotection 92%; deprotection 68%), the title product was obtained, after purification by prep-HPLC (Method 2), as a beige solid (0.07 g).
¹H NMR *(d6*-DMSO) δ: 10.64-11.27 (m, 1H); 9.21-9.31 (m, 1H); 8.32 (d, J = 1.3 Hz, 1 H); 7.94 (d, J = 8.5 Hz, 1H); 7.62 (dd, J = 1.6, 8.4 Hz, 1H); 4.76 (t, J = 5.8 Hz, 1H); 3.20-3.32 (m, 3H); 3.08 (s, 3H); 2.96 (m, 1 H); 2.77 (td, J = 4.5, 12.6 Hz, 1H); 2.25 (td, J = 5.0, 12.4 Hz, 1H); 1.59 (dd, J = 5.3, 8.7 Hz, 1H); 1.56 (s, 3H); 1.21 (s, 3H); 1.07 (dd, J = 4.0, 8.7 Hz, 1H); 0.67 (m, 1H).
MS (ESI, m/z): 460.98 [M+H⁺] for C₂₂H₂₄N₂O₅S₂; t_{R} = 0.74 min.

### Example 27: (R)-4-(6-(((1R,2R)-2-fluoro-2-(hydroxymethyl)cyclopropyl)buta-1,3-diyn-1-yl)benzo[d]thiazol-2-yl)-N-hydroxy-2-methyl-2-(methylsulfonyl)butanamide:

Starting from the compound of Preparation H (0.146 g, 0.334 mmol) and the compound of Preparation AG ((*R*,*R*)-enantiomer, 0.167 g, 0.502 mmol) and proceeding successively in analogy to Example 8, step 8.i, Example 7, step 7.ii and Reference Example RE2, step RE2.iv (yields: Cadiot coupling and deprotection 69%; deprotection 73%), the title product was obtained, after purification by prep-HPLC (Method 2), as a white solid (0.076 g).
¹H NMR *(d6*-DMSO) δ: 10.10-11.10 (br. s, 1H); 8.97-9.67 (br. s, 1H); 8.32 (m, 1H); 7.96 (m, 1H); 7.58-7.72 (m, 1H); 5.26 (m, 1H); 3.58-3.79 (m, 2H); 3.25 (m, 1H); 3.08 (s, 3H); 2.97 (m, 1H); 2.77 (m, 1H); 2.25 (m, 1H); 1.97 (m, 1H); 1.51-1.60 (s, 3H); 1.32-1.46 (m, 2H).
MS (ESI, m/z): 464.95 [M+H⁺] for C₂₁H₂₁N₂O₅FS₂; t_{R} = 0.71 min.

The racemic mixtures of Reference Examples 1 to 4 can be separated into their enantiomers using, for example, chiral HPLC. Thus the following further invention compounds or salts thereof would be obtained:
(*R*)-*N*-hydroxy-4-(6-(4-methoxyphenyl)benzo[*d*]thiazol-2-yl)-2-methyl-2-(methylsulfonyl)butanamide,
(*R*)-*N*-hydroxy-4-(6-((4-(hydroxymethyl)phenyl)ethynyl)benzo[*d*]thiazol-2-yl)-2-methyl-2-(methylsulfonyl)butanamide,
(*R*)-4-(6-((4-(3-aminooxetan-3-yl)phenyl)ethynyl)-benzo[*d*]thiazol-2-yl)-*N*-hydroxy-2-methyl-2-(methylsulfonyl)butanamide, and
(*R*)-*N*-hydroxy-4-(6-(5-hydroxypenta-1,3-diyn-1-yl)benzo[*d*]thiazol-2-yl)-2-methyl-2-(methylsulfonyl)butanamide.

### Pharmacological properties of the invention compounds

### In vitro assays

### Bacterial growth minimal inhibitory concentrations:

### Experimental methods:

Minimal Inhibitory Concentrations (MICs; mg/L) were determined in cation-adjusted Mueller-Hinton Broth by a microdilution method following the description given in *"*Methods for Dilution Antimicrobial Susceptibility Tests for Bacteria that Grow Aerobically", Approved standard, 7th ed., Clinical and Laboratory Standards Institute (CLSI) Document M7-A7, Wayne, PA, USA (2006).

### Results:

All Example compounds were tested against several Gram-positive and Gram-negative bacteria. Typical antibacterial test results are given in Table 1 hereafter (MICs in mg/L). *K*. *pneumoniae* A-651 is a multiply-resistant (in particular quinolone-resistant) strain, while *E*. *coli* ATCC25922 and *P. aeruginosa* ATCC27853 are quinolone-sensitive strains.

**Table 1**

| Example No. | MIC for *E. coli* ATCC25922 | MIC for *P. aeruginosa* A TCC27853 | MIC for *K. Pneumoniae* A-651 |
|---|---|---|---|
| RE1 | 0.25 | 16 | 2 |
| RE2 | 0.125 | 16 | 0.5 |
| RE3 | 4 | 8 | 1 |
| RE4 | 0.5 | 1 | 8 |
| 1 | 0.25 | 1 | 0.25 |
| 2 | ≤ 0.063 | 2 | 0.25 |
| 3 | 4 | 8 | 1 |
| 4 | 0.5 | 1 | 0.5 |
| 5 | 1 | 1 | 8 |
| 6 | 0.25 | 1 | 1 |
| 7 | ≤ 0.063 | 0.5 | 0.125 |
| 8 | 0.25 | 0.5 | 0.5 |
| 9 | 1 | 2 | 2 |
| 10 | 0.125 | 1 | 0.25 |
| 11 | 0.125 | 4 | 0.5 |
| 12 | 0.125 | 2 | 0.5 |
| 13 | 0.25 | 1 | 0.5 |
| 14 | 0.5 | 1 | 1 |
| 15 | 0.125 | 1 | 0.5 |
| 16 | 4 | 4 | 8 |
| 17 | 0.125 | 2 | 0.25 |
| 18 | 1 | 2 | 2 |
| 19 | 8 | 4 | 16 |
| 20 | 0.5 | 1 | 0.5 |
| 21 | 1 | 2 | 2 |
| 22 | 0.5 | 2 | 1 |
| 23 | ≤ 0.063 | 0.5 | 0.25 |
| 24 | ≤ 0.063 | 0.25 | 0.25 |
| 25 | 0.5 | 1 | 1 |
| 26 | 0.063 | 0.5 | 0.125 |
| 27 | 0.063 | 0.25 | 0.125 |
| Cipro | ≤ 0.063 | 0.25 | > 32 |

## Claims

1. A compound of formula I 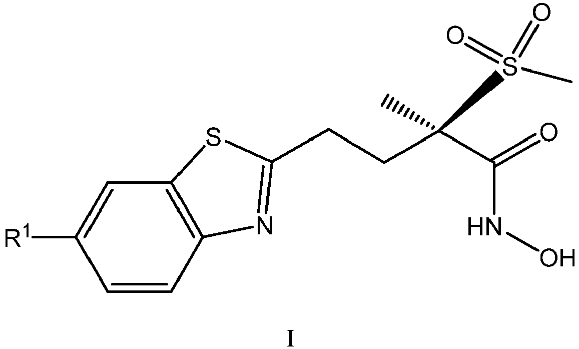 wherein
R¹ is the group M, whereby M is one of the groups M^{A} and M^{B} represented below 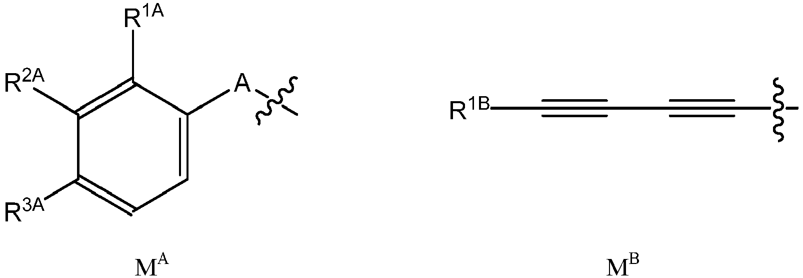
wherein A represents a bond or C≡C;
R^{1A} is H or halogen;
R^{2A} is H or halogen; and
R^{3A} is H, (C₁-C₃)alkoxy, hydroxy(C₂-C₄)alkoxy, hydroxy(C₁-C₄)alkyl, dihydroxy(C₂-C₄)alkyl, 2-hydroxyacetamido, 1-hydroxymethyl-cycloprop-1-yl, *trans*-2-hydroxymethyl-cycloprop-1-yl, 3-hydroxyoxetan-3-yl, 3-(hydroxy(C₁-C₃)alkyl)oxetan-3-yl, 3-aminooxetan-3-yl or 1-aminocycloprop-1-yl;
and wherein R^{1B} is hydroxy(C₁-C₄)alkyl, dihydroxy(C₂-C₄)alkyl, amino(C₁-C₄)alkyl, di(C₁-C₄)alkylamino(C₁-C₃)alkyl, 1-amino-cycloprop-1-yl, 1-hydroxymethyl-cycloprop-1-yl, *trans*-2-hydroxymethyl-cycloprop-1-yl, *trans*-2-aminomethyl-cycloprop-1-yl, *trans*-2-hydroxymethyl-1-methyl-cycloprop-1-yl, *trans*-2-hydroxymethyl-2-methyl-cycloprop-1-yl, *cis*-1-fluoro-2-(hydroxymethyl)cycloprop-1-yl, *cis*-2-fluoro-2-(hydroxymethyl)cycloprop-1-yl, 2-(1,2-dihydroxyethyl)-cycloprop-1-yl, 1-(hydroxymethyl)-cyclobutan-1-yl, *cis*-3-(hydroxymethyl)-1-hydroxy-cyclobutan-1-yl, 3-hydroxyoxetan-3-yl, 3-hydroxyoxetan-3-yl-(C₁-C₃)alkyl, 3-aminooxetan-3-yl, 3-hydroxymethyl-oxetan-3-yl, *trans*-(*cis*-3,4-dihydroxy)-cyclopent-1-yl, 3-hydroxymethylbicyclo[1,1,1]pentan-1-yl, 4-hydroxytetrahydro-2*H*-pyran-4-yl, (3*R*,6*S*)-3-aminotetrahydro-2*H*-pyran-6-yl, piperidin-4-yl, 1-(2-hydroxyacetyl)piperidin-4-yl, 3-hydroxythietan-3-yl, 1-(2-hydroxyacetyl)azetidin-3-yl or 1-glycylazetidin-3-yl;
or a salt thereof.

2. A compound of formula I according to claim 1, wherein R¹ is the group M^{A};
or a salt thereof.

3. A compound of formula I according to claim 2, wherein
A represents a bond or C≡C;
R^{1A} is H or halogen;
R^{2A} is H; and
R^{3A} is (C₁-C₃)alkoxy, hydroxy(C₁-C₄)alkyl, 1-hydroxymethyl-cycloprop-1-yl, 3-hydroxyoxetan-3-yl, or 3-aminooxetan-3-yl;
or a salt thereof.

4. A compound of formula I according to claim 2, wherein A represents a bond;
or a salt thereof.

5. A compound of formula I according to claim 4, wherein
R^{1A} is H or halogen;
R^{2A} is H; and
R^{3A} is (C₁-C₃)alkoxy;
or a salt thereof.

6. A compound of formula I according to claim 2, wherein A represents C≡C;
or a salt thereof.

7. A compound of formula I according to claim 6, wherein
R^{1A} and R^{2A} are both H; and
R^{3A} is hydroxy(C₁-C₄)alkyl, 1-hydroxymethyl-cycloprop-1-yl, 3-hydroxyoxetan-3-yl, or 3-aminooxetan-3-yl;
or a salt thereof.

8. A compound of formula I according to claim 1, wherein R¹ is the group M^{B};
or a salt thereof.

9. A compound of formula I according to claim 8, wherein R^{1B} is hydroxy(C₁-C₄)alkyl, dihydroxy(C₂-C₄)alkyl, amino(C₁-C₄)alkyl, di(C₁-C₄)alkylamino(C₁-C₃)alkyl, 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 1-(hydroxymethyl)-cyclobutan-1-yl, *cis*-3-(hydroxymethyl)-1-hydroxy-cyclobutan-1-yl, 3-hydroxyoxetan-3-yl, 3-hydroxyoxetan-3-yl-(C₁-C₃)alkyl, 3-aminooxetan-3-yl, 3-hydroxymethyl-oxetan-3-yl, *trans*-(*cis*-3,4-dihydroxy)-cyclopent-1-yl, 4-hydroxytetrahydro-2*H*-pyran-4-yl, (3*R*,6*S*)-3-aminotetrahydro-2*H*-pyran-6-yl, piperidin-4-yl, or 1-(2-hydroxyacetyl)piperidin-4-yl;
or a salt thereof.

10. A compound of formula I according to claim 9, wherein R^{1B} is hydroxy(C₁-C₄)alkyl, dihydroxy(C₂-C₄)alkyl, amino(C₁-C₄)alkyl, 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 3-hydroxyoxetan-3-yl, 3-hydroxyoxetan-3-yl-(C₁-C₃)alkyl, 3-hydroxymethyl-oxetan-3-yl, or *trans*-(*cis*-3,4-dihydroxy)-cyclopent-1-yl;
or a salt thereof.

11. A compound of formula I according to claim 1, wherein
R¹ is the group M^{A}, A represents a bond,
R^{1A} is halogen,
R^{2A} is H, and
R^{3A} is (C₁-C₃)alkoxy;
or R¹ is the group M^{A}, A represents C≡C,
R^{1A} and R^{2A} are both H, and
R^{3A} is 1-hydroxymethyl-cycloprop-1-yl;
or R¹ is the group M^{B} and R^{1B} is di(C₁-C₄)alkylamino(C₁-C₃)alkyl, 1-(hydroxymethyl)-cyclobutan-1-yl, *cis*-3-(hydroxymethyl)-1-hydroxy-cyclobutan-1-yl, 3-aminooxetan-3-yl, 4-hydroxytetrahydro-2*H*-pyran-4-yl, (3*R*,6*S*)-3-aminotetrahydro-2*H*-pyran-6-yl, piperidin-4-yl, or 1-(2-hydroxyacetyl)piperidin-4-yl;
or a salt thereof.

12. A compound of formula I according to claim 1, wherein
A represents a bond or C≡C;
R^{1A} is H or halogen;
R^{2A} is H; and
R^{3A} is (C₁-C₃)alkoxy, hydroxy(C₁-C₄)alkyl, 1-hydroxymethyl-cycloprop-1-yl, 3-hydroxyoxetan-3-yl, or 3-aminooxetan-3-yl;
and wherein R^{1B} is hydroxy(C₁-C₄)alkyl, dihydroxy(C₂-C₄)alkyl, amino(C₁-C₄)alkyl, di(C₁-C₄)alkylamino(C₁-C₃)alkyl, 1-amino-cycloprop-1-yl, 1-hydroxymethyl-cycloprop-1-yl, *trans*-2-hydroxymethyl-cycloprop-1-yl, *trans*-2-hydroxymethyl-1-methyl-cycloprop-1-yl, *cis*-1-fluoro-2-(hydroxymethyl)cycloprop-1-yl, *cis*-2-fluoro-2-(hydroxymethyl)cycloprop-1-yl, 1-(hydroxymethyl)-cyclobutan-1-yl, *cis*-3-(hydroxymethyl)-1-hydroxy-cyclobutan-1-yl, 3 -hydroxyoxetan-3 -yl, 3-hydroxyoxetan-3-yl-(C₁-C₃)alkyl, 3-aminooxetan-3-yl, 3-hydroxymethyl-oxetan-3-yl, *trans*-(*cis*-3,4-dihydroxy)-cyclopent-1-yl, 4-hydroxytetrahydro-2*H*-pyran-4-yl, (3*R*,6*S*)-3-aminotetrahydro-2*H*-pyran-6-yl, piperidin-4-yl, or 1-(2-hydroxyacetyl)piperidin-4-yl;
or a salt thereof.

13. A compound of formula I according to claim 1, which is selected from the group consisting of:
(*R*)-*N*-hydroxy-4-(6-((3-hydroxyoxetan-3-yl)buta-1,3-diyn-1-yl)benzo[*d*]thiazol-2-yl)-2-methyl-2-(methylsulfonyl)butanamide,
(*R*)-4-(6-(2-fluoro-4-methoxyphenyl)benzo[*d*]thiazol-2-yl)-*N*-hydroxy-2-methyl-2-(methylsulfonyl)butanamide,
(*R*)-N-hydroxy-4-(6-((4-(3-hydroxyoxetan-3-yl)phenyl)ethynyl)benzo[d]thiazol-2-yl)-2-methyl-2-(methylsulfonyl)butanamide,
(*R*)-N-hydroxy-4-(6-(5-hydroxy-5-methylhexa-1,3-diyn-1-yl)benzo[*d*]thiazol-2-yl)-2-methyl-2-(methylsulfonyl)butanamide,
(*R*)-4-(6-((*S*)-5,6-dihydroxyhexa-1,3-diyn-1-yl)benzo[*d*]thiazol-2-yl)-*N*-hydroxy-2-methyl-2-(methylsulfonyl)butanamide,
(*R*)-4-(6-(5-amino-5-methylhexa-1,3-diyn-1-yl)benzo[*d*]thiazol-2-yl)-*N*-hydroxy-2-methyl-2-(methylsulfonyl)butanamide,
(*R*)-*N*-hydroxy-4-(6-(((1*S*,2*S*)-2-(hydroxymethyl)cyclopropyl)buta-1,3-diyn-1-yl)benzo[*d*]thiazol-2-yl)-2-methyl-2-(methylsulfonyl)butanamide, (*R*)-*N*-hydroxy-4-(6-((1-(hydroxymethyl)cyclopropyl)buta-1,3-diyn-1-yl)benzo[*d*]thiazol-2-yl)-2-methyl-2-(methylsulfonyl)butanamide,
(*R*)-4-(6-((3-aminooxetan-3-yl)buta-1,3-diyn-1-yl)benzo[*d*]thiazol-2-yl)-*N*-hydroxy-2-methyl-2-(methylsulfonyl)butanamide,
(*R*)-4-(6-((1-aminocyclopropyl)buta-1,3-diyn-1-yl)benzo[*d*]thiazol-2-yl)-*N*-hydroxy-2-methyl-2-(methylsulfonyl)butanamide,
(*R*)-*N*-hydroxy-4-(6-((4-(1-(hydroxymethyl)cyclopropyl)phenyl)ethynyl)benzo[*d*]thiazol-2-yl)-2-methyl-2-(methylsulfonyl)butanamide,
(*R*)-*N*-hydroxy-4-(6-((1-(hydroxymethyl)cyclobutyl)buta-1,3-diyn-1-yl)benzo[*d*]thiazol-2-yl)-2-methyl-2-(methylsulfonyl)butanamide,
(*R*)-4-(6-(((1*s*,3*R*,4*S*)-3,4-dihydroxycyclopentyl)buta-1,3-diyn-1-yl)benzo[*d*]thiazol-2-yl)-*N*-hydroxy-2-methyl-2-(methylsulfonyl)butanamide,
(*R*)-*N*-hydroxy-4-(6-(5-(3-hydroxyoxetan-3-yl)penta-1,3-diyn-1-yl)benzo[*d*]thiazol-2-yl)-2-methyl-2-(methylsulfonyl)butanamide,
(*R*)-*N*hydroxy-4-(6-(((1*R*,2*R*)-2-(hydroxymethyl)-1-methylcyclopropyl)buta-1,3-diyn-1-yl)benzo[d]thiazol-2-yl)-2-methyl-2-(methylsulfonyl)butanamide,
(*R*)-4-(6-(((2*S*,5*R*)-5-aminotetrahydro-2*H*-pyran-2-yl)buta-1,3-diyn-1-yl)benzo[*d*]thiazol-2-yl)-*N*-hydroxy-2-methyl-2-(methylsulfonyl)butanamide,
(*R*)-4-(6-(5-(dimethylamino)penta-1,3-diyn-1-yl)benzo[*d*]thiazol-2-yl)-*N*-hydroxy-2-methyl-2-(methylsulfonyl)butanamide,
(*R*)-*N*-hydroxy-4-(6-((4-hydroxytetrahydro-2*H*-pyran-4-yl)buta-1,3-diyn-1-yl)benzo[*d*]thiazol-2-yl)-2-methyl-2-(methylsulfonyl)butanamide, (*R*)-*N*-hydroxy-2-methyl-2-(methylsulfonyl)-4-(6-(piperidin-4-ylbuta-1,3-diyn-1-yl)benzo[*d*]thiazol-2-yl)butanamide,
(*R*)-*N*-hydroxy-4-(6-((3-(hydroxymethyl)oxetan-3-yl)buta-1,3-diyn-1-yl)benzo[*d*]thiazol-2-yl)-2-methyl-2-(methylsulfonyl)butanamide,
(*R*)-*N*-hydroxy-4-(6-((*cis*-1-hydroxy-3-(hydroxymethyl)cyclobutyl)buta-1,3 -diyn-1-yl)benzo[*d*]thiazol-2-yl)-2-methyl-2-(methylsulfonyl)butanamide,
(*R*)-*N*-hydroxy-4-(6-((1-(2-hydroxyacetyl)piperidin-4-yl)buta-1,3-diyn-1-yl)benzo[*d*]thiazol-2-yl)-2-methyl-2-(methylsulfonyl)butanamide,
(*R*)-4-(6-(((*1R**,*2R**)-1-fluoro-2-(hydroxymethyl)cyclopropyl)buta-1,3 -diyn-1-yl)benzo[*d*]thiazol-2-yl)-*N*-hydroxy-2-methyl-2-(methylsulfonyl)butanamide, and
(*R*)-4-(6-(((*1R**,*2R**)-2-fluoro-2-(hydroxymethyl)cyclopropyl)buta-1,3 -diyn-1-yl)benzo[*d*]thiazol-2-yl)-*N*-hydroxy-2-methyl-2-(methylsulfonyl)butanamide,
or a salt of such compound.

14. A compound of formula I according to claim 1, which is selected from the group consisting of:
(*R*)-*N*-hydroxy-4-(6-(4-methoxyphenyl)benzo[*d*]thiazol-2-yl)-2-methyl-2-(methylsulfonyl)butanamide,
(*R*)-*N*-hydroxy-4-(6-((4-(hydroxymethyl)phenyl)ethynyl)benzo[*d*]thiazol-2-yl)-2-methyl-2-(methylsulfonyl)butanamide,
(*R*)-4-(6-((4-(3-aminooxetan-3-yl)phenyl)ethynyl)-benzo[*d*]thiazol-2-yl)-*N*-hydroxy-2-methyl-2-(methylsulfonyl)butanamide, and
(*R*)-*N*-hydroxy-4-(6-(5-hydroxypenta-1,3-diyn-1-yl)benzo[*d*]thiazol-2-yl)-2-methyl-2-(methylsulfonyl)butanamide,
or a salt of such compound.

15. A compound of formula I according to claim 1, which is selected from the group consisting of:
(*R*)-*N*-hydroxy-4-(6-((1-(2-hydroxyacetyl)azetidin-3-yl)buta-1,3-diyn-1-yl)benzo[*d*]thiazol-2-yl)-2-methyl-2-(methylsulfonyl)butanamide,
(*R*)-*N*-hydroxy-4-(6-(((*1R,2S*)-2-(hydroxymethyl)-2-methylcyclopropyl)buta-1,3-diyn-1-yl)benzo[d]thiazol-2-yl)-2-methyl-2-(methylsulfonyl)butanamide, and
(*R*)-4-(6-(((*1R*,*2R*)-2-fluoro-2-(hydroxymethyl)cyclopropyl)buta-1,3-diyn-1-yl)benzo[*d*]thiazol-2-yl)-*N*-hydroxy-2-methyl-2-(methylsulfonyl)butanamide,
or a salt of such compound.

16. A compound of formula I as defined in one of claims 1 to 15, or a pharmaceutically acceptable salt thereof for use as a medicament.

17. A pharmaceutical composition containing, as active principle, a compound of formula I as defined in one of claims 1 to 15, or a pharmaceutically acceptable salt thereof, and at least one therapeutically inert excipient.

18. A compound of formula I as defined in one of claims 1 to 15, or a pharmaceutically acceptable salt thereof, for use in the prevention or treatment of a bacterial infection.

19. A compound or pharmaceutically acceptable salt according to claim 18 for use in the prevention or treatment of a Gram-negative bacterial infection.

## Patentansprüche

1. Verbindung von Formel I 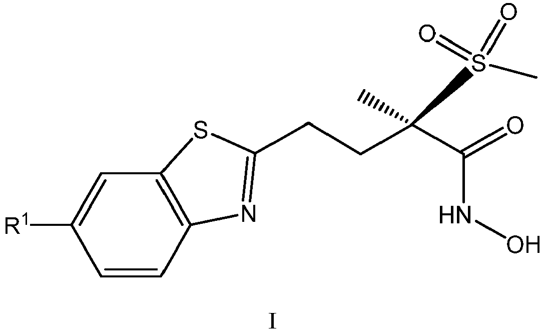 wobei
R¹ die Gruppe M ist, wobei M eine der nachfolgend repräsentierten Gruppen M^{A} und M^{B} ist 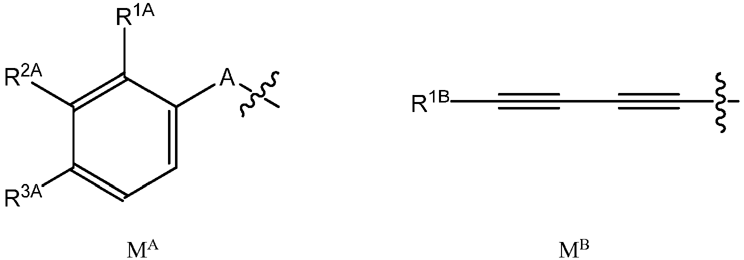
wobei A eine Bindung oder C≡C repräsentiert;
R^{1A} H oder Halogen ist;
R^{2A} H oder Halogen ist; und
R^{3A} Folgendes ist: H, (C₁-C₃)Alkoxy, Hydroxy(C₂-C₄)alkoxy, Hydroxy(C₁-C₄)alkyl, Dihydroxy(C₂-C₄)alkyl, 2-Hydroxyacetamido, 1-Hydroxymethyl-cycloprop-1-yl, *trans*-2-Hydroxymethyl-cycloprop-1-yl, 3-Hydroxyoxetan-3-yl, 3-(Hydroxy(C₁-C₃)alkyl)oxetan-3-yl, 3-Aminooxetan-3-yl oder 1-Aminocycloprop-1-yl; und wobei R^{1B} Folgendes ist: Hydroxy(C₁-C₄)alkyl, Dihydroxy(C₂-C₄)alkyl, Amino(C₁-C₄)alkyl, Di(C₁-C₄)alkylamino(C₁-C₃)alkyl, 1-Amino-cycloprop-1-yl, 1-Hydroxymethyl-cycloprop-1-yl, *trans-*2-Hydroxymethyl-cycloprop-1-yl, *trans*-2-Aminomethyl-cycloprop-1-yl, *trans*-2-Hydroxymethyl-1-methyl-cycloprop-1-yl, *trans*-2-Hydroxymethyl-2-methyl-cycloprop-1-yl, *cis*-1-Fluor-2-(hydroxymethyl)cycloprop-1-yl, *cis*-2-Fluor-2-(hydroxymethyl)cycloprop-1-yl, 2-(1,2-Dihydroxyethyl)-cycloprop-1-yl, 1-(Hydroxymethyl)-cyclobutan-1-yl, *cis*-3-(Hydroxymethyl)-1-hydroxy-cyclobutan-1-yl, 3-Hydroxyoxetan-3-yl, 3-Hydroxyoxetan-3-yl-(C₁-C₃)alkyl, 3-Aminooxetan-3-yl, 3-Hydroxymethyl-oxetan-3-yl, *trans*-(*cis*-3,4-Dihydroxy)-cyclopent-1-yl, 3-Hydroxymethylbicyclo[1,1,1]pentan-1-yl, 4-Hydroxytetrahydro-2*H*-pyran-4-yl, (3*R*,6*S*)-3-Aminotetrahydro-2*H*-pyran-6-yl, Piperidin-4-yl, 1-(2-Hydroxyacetyl)piperidin-4-yl, 3 -Hydroxythietan-3 -yl, 1-(2-Hydroxyacetyl)azetidin-3-yl oder 1-Glycylazetidin-3-yl;
oder ein Salz davon.

2. Verbindung von Formel I nach Anspruch 1, wobei R¹ die Gruppe M^{A} ist;
oder ein Salz davon.

3. Verbindung von Formel I nach Anspruch 2, wobei
A eine Bindung oder C≡C repräsentiert;
R^{1A} H oder Halogen ist;
R^{2A} H ist; und
R^{3A} (C₁-C₃)Alkoxy, Hydroxy(C₁-C₄)alkyl, 1-Hydroxymethyl-cycloprop-1-yl, 3-Hydroxyoxetan-3-yl oder 3-Aminooxetan-3-yl ist;
oder ein Salz davon.

4. Verbindung von Formel I nach Anspruch 2, wobei A eine Bindung repräsentiert;
oder ein Salz davon.

5. Verbindung von Formel I nach Anspruch 4, wobei
R^{1A} H oder Halogen ist;
R^{2A} H ist; und
R^{3A} (C₁-C₃)Alkoxy ist;
oder ein Salz davon.

6. Verbindung von Formel I nach Anspruch 2, wobei A C≡C repräsentiert;
oder ein Salz davon.

7. Verbindung von Formel I nach Anspruch 6, wobei
R^{1A} und R^{2A} beide H sind; und
R^{3A} Hydroxy(C₁-C₄)alkyl, 1-Hydroxymethyl-cycloprop-1-yl, 3-Hydroxyoxetan-3-yl oder 3-Aminooxetan-3-yl ist;
oder ein Salz davon.

8. Verbindung von Formel I nach Anspruch 1, wobei R¹ die Gruppe M^{B} ist;
oder ein Salz davon.

9. Verbindung von Formel I nach Anspruch 8, wobei R^{1B} Folgendes ist: Hydroxy(C₁-C₄)alkyl, Dihydroxy(C₂-C₄)alkyl, Amino(C₁-C₄)alkyl, Di(C₁-C₄)alkylamino(C₁-C₃)alkyl, 1-Amino-cycloprop-1-yl, 1-Hydroxymethyl-cycloprop-1-yl, *trans*-2-Hydroxymethyl-cycloprop-1-yl, *trans*-2-Hydroxymethyl-1-methyl-cycloprop-1-yl, *cis*-1-Fluor-2-(hydroxymethyl)cycloprop-1-yl, *cis*-2-Fluor-2-(hydroxymethyl)cycloprop-1-yl, 1-(Hydroxymethyl)-cyclobutan-1-yl, *cis*-3-(Hydroxymethyl)-1-hydroxy-cyclobutan-1-yl, 3-Hydroxyoxetan-3-yl, 3 -Hydroxyoxetan-3-yl-(C₁-C₃)alkyl, 3-Aminooxetan-3-yl, 3-Hydroxymethyl-oxetan-3-yl, *trans-*(*cis*-3,4-Dihydroxy)-cyclopent-1-yl, 4-Hydroxytetrahydro-2*H*-pyran-4-yl, (*3R*,*6S*)-3-Aminotetrahydro-2*H*-pyran-6-yl, Piperidin-4-yl oder 1-(2-Hydroxyacetyl)piperidin-4-yl; oder ein Salz davon.

10. Verbindung von Formel I nach Anspruch 9, wobei R^{1B} Folgendes ist: Hydroxy(C₁-C₄)alkyl, Dihydroxy(C₂-C₄)alkyl, Amino(C₁-C₄)alkyl, 1-Amino-cycloprop-1-yl, 1-Hydroxymethyl-cycloprop-1-yl, *trans-*2-Hydroxymethyl-cycloprop-1-yl, *trans*-2-Hydroxymethyl-1-methyl-cycloprop-1-yl, *cis*-1-Fluor-2-(hydroxymethyl)cycloprop-1-yl, *cis*-2-Fluor-2-(hydroxymethyl)cycloprop-1-yl, 3-Hydroxyoxetan-3-yl, 3-Hydroxyoxetan-3-yl-(C₁-C₃)alkyl, 3-Hydroxymethyl-oxetan-3-yl oder *trans*-(*cis*-3,4-Dihydroxy)-cyclopent-1-yl;
oder ein Salz davon.

11. Verbindung von Formel I nach Anspruch 1, wobei
R¹ die Gruppe M^{A} ist, A eine Bindung repräsentiert,
R^{1A} Halogen ist,
R^{2A} H ist, und
R^{3A} (C₁-C₃)Alkoxy ist;
oder R¹ die Gruppe M^{A} ist, A C≡C repräsentiert,
R^{1A} und R^{2A} beide H sind, und
R^{3A} 1-Hydroxymethyl-cycloprop-1-yl ist;
oder R¹ die Gruppe M^{B} ist und R^{1B} Di(C₁-C₄)alkylamino(C₁-C₃)alkyl, 1-(Hydroxymethyl)-cyclobutan-1-yl, *cis*-3-(Hydroxymethyl)-1-hydroxy-cyclobutan-1-yl, 3-Aminooxetan-3-yl, 4-Hydroxytetrahydro-2*H*-pyran-4-yl, (*3R*,*6S*)-3-Aminotetrahydro-2*H*-pyran-6-yl, Piperidin-4-yl oder 1-(2-Hydroxyacetyl)piperidin-4-yl ist;
oder ein Salz davon.

12. Verbindung von Formel I nach Anspruch 1, wobei
A eine Bindung oder C≡C repräsentiert;
R^{1A} H oder Halogen ist;
R^{2A} H ist; und
R^{3A} (C₁-C₃)Alkoxy, Hydroxy(C₁-C₄)alkyl, 1-Hydroxymethyl-cycloprop-1-yl, 3-Hydroxyoxetan-3-yl oder 3-Aminooxetan-3-yl ist;
und wobei R^{1B} Folgendes ist: Hydroxy(C₁-C₄)alkyl, Dihydroxy(C₂-C₄)alkyl, Amino(C₁-C₄)alkyl, Di(C₁-C₄)alkylamino(C₁-C₃)alkyl, 1-Amino-cycloprop-1-yl, 1-Hydroxymethyl-cycloprop-1-yl, *trans-*2-Hydroxymethyl-cycloprop-1-yl, *trans*-2-Hydroxymethyl-1-methyl-cycloprop-1-yl, *cis*-1-Fluor-2-(hydroxymethyl)cycloprop-1-yl, *cis*-2-Fluor-2-(hydroxymethyl)cycloprop-1-yl, 1-(Hydroxymethyl)-cyclobutan-1-yl, *cis*-3-(Hydroxymethyl)-1-hydroxy-cyclobutan-1-yl, 3-Hydroxyoxetan-3-yl, 3-Hydroxyoxetan-3-yl-(C₁-C₃)alkyl, 3-Aminooxetan-3-yl, 3-Hydroxymethyl-oxetan-3-yl, *trans*-(*cis*-3,4-Dihydroxy)-cyclopent-1-yl, 4-Hydroxytetrahydro-2*H*-pyran-4-yl, (*3R*,*6S*)-3-Aminotetrahydro-2*H*-pyran-6-yl, Piperidin-4-yl oder 1-(2-Hydroxyacetyl)piperidin-4-yl,
oder ein Salz davon.

13. Verbindung von Formel I nach Anspruch 1, die ausgewählt ist aus der Gruppe bestehend aus:
(*R*)-*N*-Hydroxy-4-(6-((3-hydroxyoxetan-3-yl)buta-1,3-diyn-1-yl)benzo[*d*]thiazol-2-yl)-2-methyl-2-(methylsulfonyl)butanamid,
(*R*)-4-(6-(2-Fluor-4-methoxyphenyl)benzo[*d*]thiazol-2-yl)-*N*-hydroxy-2-methyl-2-(methylsulfonyl)butanamid,
(*R*)-N-Hydroxy-4-(6-((4-(3-hydroxyoxetan-3-yl)phenyl)ethynyl)benzo[d]thiazol-2-yl)-2-methyl-2-(methylsulfonyl)butanamid,
(*R*)-N-Hydroxy-4-(6-(5-hydroxy-5-methylhexa-1,3-diyn-1-yl)benzo[*d*]thiazol-2-yl)-2-methyl-2-(methylsulfonyl)butanamid,
(*R*)-4-(6-((*S*)-5,6-Dihydroxyhexa-1,3-diyn-1-yl)benzo[*d*]thiazol-2-yl)-*N*-hydroxy-2-methyl-2-(methylsulfonyl)butanamid,
(*R*)-4-(6-(5-Amino-5-methylhexa-1,3-diyn-1-yl)benzo[*d*]thiazol-2-yl)-*N*-hydroxy-2-methyl-2-(methylsulfonyl)butanamid,
(*R*)-*N*-Hydroxy-4-(6-(((*1S,2S*)-2-(hydroxymethyl)cyclopropyl)buta-1,3-diyn-1-yl)benzo[*d*]thiazol-2-yl)-2-methyl-2-(methylsulfonyl)butanamid,
(*R*)-*N*-Hydroxy-4-(6-((1-(hydroxymethyl)cyclopropyl)buta-1,3-diyn-1-yl)benzo[*d*]thiazol-2-yl)-2-methyl-2-(methylsulfonyl)butanamid,
(*R*)-4-(6-((3-Aminooxetan-3-yl)buta-1,3-diyn-1-yl)benzo[*d*]thiazol-2-yl)-*N*-hydroxy-2-methyl-2-(methylsulfonyl)butanamid,
(*R*)-4-(6-((1-Aminocyclopropyl)buta-1,3-diyn-1-yl)benzo[*d*]thiazol-2-yl)-*N*-hydroxy-2-methyl-2-(methylsulfonyl)butanamid,
(*R*)-*N*-Hydroxy-4-(6-((4-(1-(hydroxymethyl)cyclopropyl)phenyl)ethynyl)benzo[*d*]thiazol-2-yl)-2-methyl-2-(methylsulfonyl)butanamid,
(*R*)-*N*-Hydroxy-4-(6-((1-(hydroxymethyl)cyclobutyl)buta-1,3-diyn-1-yl)benzo[*d*]thiazol-2-yl)-2-methyl-2-(methylsulfonyl)butanamid,
(*R*)-4-(6-(((*1s,3R,4S*)-3,4-Dihydroxycyclopentyl)buta-1,3-diyn-1-yl)benzo[*d*]thiazol-2-yl)-*N*-hydroxy-2-methyl-2-(methylsulfonyl)butanamid,
(*R*)-*N*-Hydroxy-4-(6-(5-(3-hydroxyoxetan-3-yl)penta-1,3-diyn-1-yl)benzo[*d*]thiazol-2-yl)-2-methyl-2-(methylsulfonyl)butanamid,
(*R*)-*N*-Hydroxy-4-(6-(((*1R,2R*)-2-(hydroxymethyl)-1-methylcyclopropyl)buta-1,3-diyn-1-yl)benzo[*d*]thiazol-2-yl)-2-methyl-2-(methylsulfonyl)butanamid, (*R*)-4-(6-(((*2S,5R*)-5-Aminotetrahydro-2*H*-pyran-2-yl)buta-1,3-diyn-1-yl)benzo[*d*]thiazol-2-yl)-*N*-hydroxy-2-methyl-2-(methylsulfonyl)butanamid,
(*R*)-4-(6-(5-(Dimethylamino)penta-1,3-diyn-1-yl)benzo[*d*]thiazol-2-yl)-*N*-hydroxy-2-methyl-2-(methylsulfonyl)butanamid,
(*R*)-*N*-Hydroxy-4-(6-((4-hydroxytetrahydro-2*H*-pyran-4-yl)buta-1,3-diyn-1-yl)benzo[*d*]thiazol-2-yl)-2-methyl-2-(methylsulfonyl)butanamid,
(*R*)-*N*-Hydroxy-2-methyl-2-(methylsulfonyl)-4-(6-(piperidin-4-ylbuta-1,3-diyn-1-yl)benzo[*d*]thiazol-2-yl)butanamid,
(*R*)-*N*-Hydroxy-4-(6-((3-(hydroxymethyl)oxetan-3-yl)buta-1,3-diyn-1-yl)benzo[*d*]thiazol-2-yl)-2-methyl-2-(methylsulfonyl)butanamid,
(*R*)-*N*-Hydroxy-4-(6-((*cis*-1-hydroxy-3-(hydroxymethyl)cyclobutyl)buta-1,3-diyn-1-yl)benzo[*d*]thiazol-2-yl)-2-methyl-2-(methylsulfonyl)butanamid,
(*R*)-*N*-Hydroxy-4-(6-((1-(2-hydroxyacetyl)piperidin-4-yl)buta-1,3-diyn-1-yl)benzo[*d*]thiazol-2-yl)-2-methyl-2-(methylsulfonyl)butanamid,
(*R*)-4-(6-(((*1R**,*2R**)-1-Fluor-2-(hydroxymethyl)cyclopropyl)buta-1,3-diyn-1-yl)benzo[*d*]thiazol-2-yl)-*N*-hydroxy-2-methyl-2-(methylsulfonyl)butanamid und
(*R*)-4-(6-(((*1R**,*2R**)-2-Fluor-2-(hydroxymethyl)cyclopropyl)buta-1,3-diyn-1-yl)benzo[*d*]thiazol-2-yl)-*N*-hydroxy-2-methyl-2-(methylsulfonyl)butanamid,
oder ein Salz einer solchen Verbindung.

14. Verbindung von Formel I nach Anspruch 1, die ausgewählt ist aus der Gruppe bestehend aus:
(*R*)-*N*-Hydroxy-4-(6-(4-methoxyphenyl)benzo[*d*]thiazol-2-yl)-2-methyl-2-(methylsulfonyl)butanamid,
(*R*)-*N*-Hydroxy-4-(6-((4-(hydroxymethyl)phenyl)ethynyl)benzo[*d*]thiazol-2-yl)-2-methyl-2-(methylsulfonyl)butanamid,
(*R*)-4-(6-((4-(3-Aminooxetan-3-yl)phenyl)ethynyl)-benzo[*d*]thiazol-2-yl)-*N*-hydroxy-2-methyl-2-(methylsulfonyl)butanamid und
(*R*)-*N*-Hydroxy-4-(6-(5-hydroxypenta-1,3-diyn-1-yl)benzo[*d*]thiazol-2-yl)-2-methyl-2-(methylsulfonyl)butanamid,
oder ein Salz einer solchen Verbindung.

15. Verbindung von Formel I nach Anspruch 1, die ausgewählt ist aus der Gruppe bestehend aus:
(*R*)-*N*-Hydroxy-4-(6-((1-(2-hydroxyacetyl)azetidin-3-yl)buta-1,3-diyn-1-yl)benzo[*d*]thiazol-2-yl)-2-methyl-2-(methylsulfonyl)butanamid,
(*R*)-*N*-Hydroxy-4-(6-(((*1R,2S*)-2-(hydroxymethyl)-2-methylcyclopropyl)buta-1,3-diyn-1-yl)benzo[d]thiazol-2-yl)-2-methyl-2-(methylsulfonyl)butanamid und
(*R*)-4-(6-(((*1R,2R*)-2-Fluor-2-(hydroxymethyl)cyclopropyl)buta-1,3-diyn-1-yl)benzo[d]thiazol-2-yl)-*N*-hydroxy-2-methyl-2-(methylsulfonyl)butanamid,
oder ein Salz einer solchen Verbindung.

16. Verbindung von Formel I nach einem der Ansprüche 1 bis 15 oder ein pharmazeutisch akzeptables Salz davon zur Verwendung als Medikament.

17. Pharmazeutische Zusammensetzung, die als Wirkstoff eine Verbindung von Formel I nach einem der Ansprüche 1 bis 15 oder ein pharmazeutisch akzeptables Salz davon und wenigstens einen therapeutisch inerten Hilfsstoff enthält.

18. Verbindung von Formel I nach einem der Ansprüche 1 bis 15 oder ein pharmazeutisch akzeptables Salz davon zur Verwendung bei der Verhütung oder Behandlung einer bakteriellen Infektion.

19. Verbindung oder pharmazeutisch akzeptables Salz nach Anspruch 18 zur Verwendung bei der Verhütung oder Behandlung einer gramnegativen bakteriellen Infektion.

## Revendications

1. Composé de formule I 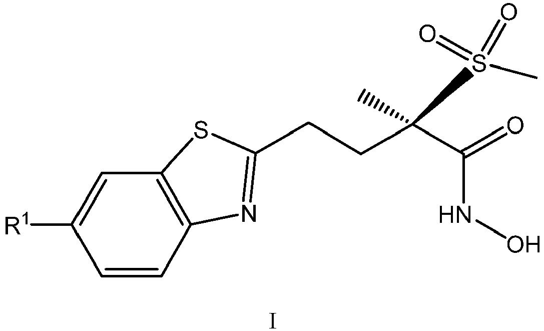 où
R¹ représente le groupement M, M étant l'un des groupements M^{A} et M^{B} illustrés ci-dessous 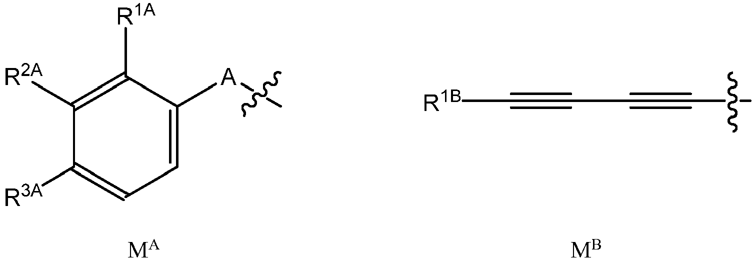
où A représente une liaison ou un C≡C ;
R^{1A} représente un H ou un halogène ;
R^{2A} représente un H ou un halogène ; et
R^{3A} représente un H, (C₁-C₃)alkoxy, hydroxy(C₂-C₄)alkoxy, hydroxy(C₁-C₄)alkyle, dihydroxy(C₂-C₄)alkyle, 2-hydroxyacétamido, 1-hydroxyméthyl-cycloprop-1-yle, *trans*-2-hydroxyméthyl-cycloprop-1-yle, 3-hydroxyoxétan-3-yle, 3-(hydroxy(C₁-C₃)alkyl)oxétan-3-yle, 3-aminooxétan-3-yle ou 1-aminocycloprop-1-yle ;
et où R^{1B} représente un hydroxy(C₁-C₄)alkyle, dihydroxy(C₂-C₄)alkyle, amino(C₁-C₄)alkyle, di(C₁-C₄)alkylamino(C₁-C₃)alkyle, 1-amino-cycloprop-1-yle, 1-hydroxyméthyl-cycloprop-1-yle, *trans*-2-hydroxyméthyl-cycloprop-1-yle, *trans*-2-aminométhyl-cycloprop-1-yle, *trans*-2-hydroxyméthyl-1-méthyl-cycloprop-1-yle, *trans*-2-hydroxyméthyl-2-méthyl-cycloprop-1-yle, *cis*-1-fluoro-2-(hydroxyméthyl)cycloprop-1-yle, *cis*-2-fluoro-2-(hydroxyméthyl)cycloprop-1-yle, 2-(1,2-dihydroxyéthyl)-cycloprop-1-yle, 1-(hydroxyméthyl)-cyclobutan-1-yle, *cis*-3-(hydroxyméthyl)-1-hydroxy-cyclobutan-1-yle, 3-hydroxyoxétan-3 -yle, 3-hydroxyoxétan-3-yl-(C₁-C₃)alkyle, 3-aminooxétan-3-yle, 3-hydroxyméthyl-oxétan-3-yle, *trans*-(*cis*-3,4-dihydroxy)-cyclopent-1-yle, 3-hydroxyméthylbicyclo[1,1,1]pentan-1-yle, 4-hydroxytétrahydro-2*H*-pyran-4-yle, (*3R*,*6S*)-3-aminotétrahydro-2*H*-pyran-6-yle, pipéridin-4-yle, 1-(2-hydroxyacétyl)pipéridin-4-yle, 3-hydroxythiétan-3-yle, 1-(2-hydroxyacétyl)azétidin-3-yle ou 1-glycylazétidin-3-yle ;
ou sel de celui-ci.

2. Composé de formule I selon la revendication 1, où R¹ représente le groupement M^{A} ;
ou sel de celui-ci.

3. Composé de formule I selon la revendication 2, où
A représente une liaison ou un C≡C ;
R^{1A} représente un H ou un halogène ;
R^{2A} représente un H ; et
R^{3A} représente un (C₁-C₃)alkoxy, hydroxy(C₁-C₄)alkyle, 1-hydroxyméthyl-cycloprop-1-yle, 3-hydroxyoxétan-3-yle ou 3-aminooxétan-3-yle ;
ou sel de celui-ci.

4. Composé de formule I selon la revendication 2, où A représente une liaison ;
ou sel de celui-ci.

5. Composé de formule I selon la revendication 4, où
R^{1A} représente un H ou un halogène ;
R^{2A} représente un H ; et
R^{3A} représente un (C₁-C₃)alkoxy ;
ou sel de celui-ci.

6. Composé de formule I selon la revendication 2, où A représente un C≡C ;
ou sel de celui-ci.

7. Composé de formule I selon la revendication 6, où
R^{1A} et R^{2A} représentent tous deux un H ; et
R^{3A} représente un hydroxy(C₁-C₄)alkyle, 1-hydroxyméthyl-cycloprop-1-yle, 3-hydroxyoxétan-3-yle ou 3-aminooxétan-3-yle ;
ou sel de celui-ci.

8. Composé de formule I selon la revendication 1, où R¹ représente le groupement M^{B} ;
ou sel de celui-ci.

9. Composé de formule I selon la revendication 8, où R^{1B} représente un hydroxy(C₁-C₄)alkyle, dihydroxy(C₂-C₄)alkyle, amino(C₁-C₄)alkyle, di(C₁-C₄)alkylamino(C₁-C₃)alkyle, 1-amino-cycloprop-1-yle, 1-hydroxyméthyl-cycloprop-1-yle, *trans*-2-hydroxyméthyl-cycloprop-1-yle, *trans*-2-hydroxyméthyl-1-méthyl-cycloprop-1-yle, *cis*-1-fluoro-2-(hydroxyméthyl)cycloprop-1-yle, *cis*-2-fluoro-2-(hydroxyméthyl)cycloprop-1-yle, 1-(hydroxyméthyl)-cyclobutan-1-yle, *cis*-3-(hydroxyméthyl)-1-hydroxy-cyclobutan-1-yle, 3-hydroxyoxétan-3-yle, 3-hydroxyoxétan-3-yl-(C₁-C₃)alkyle, 3-aminooxétan-3-yle, 3-hydroxyméthyl-oxétan-3-yle, *trans*-(*cis*-3,4-dihydroxy)-cyclopent-1-yle, 4-hydroxytétrahydro-2*H*-pyran-4-yle, (*3R,6S*)-3-aminotétrahydro-2*H*-pyran-6-yle, pipéridin-4-yle ou 1-(2-hydroxyacétyl)pipéridin-4-yle ; ou sel de celui-ci.

10. Composé de formule I selon la revendication 9, où R^{1B} représente un hydroxy(C₁-C₄)alkyle, dihydroxy(C₂-C₄)alkyle, amino(C₁-C₄)alkyle, 1-amino-cycloprop-1-yle, 1-hydroxyméthyl-cycloprop-1-yle, *trans-*2-hydroxyméthyl-cycloprop-1-yle, *trans*-2-hydroxyméthyl-1-méthyl-cycloprop-1-yle, *cis*-1-fluoro-2-(hydroxyméthyl)cycloprop-1-yle, *cis*-2-fluoro-2-(hydroxyméthyl)cycloprop-1-yle, 3-hydroxyoxétan-3-yle, 3-hydroxyoxétan-3-yl-(C₁-C₃)alkyle, 3-hydroxyméthyl-oxétan-3-yle ou *trans*-(*cis*-3,4-dihydroxy)-cyclopent-1-yle ;
ou sel de celui-ci.

11. Composé de formule I selon la revendication 1, où
R¹ représente le groupement M^{A}, A représente une liaison,
R^{1A} représente un halogène,
R^{2A} représente un H, et
R^{3A} représente un (C₁-C₃)alkoxy ;
ou R¹ représente le groupement M^{A}, A représente un C≡C,
R^{1A} et R^{2A} représentent tous deux un H, et
R^{3A} représente un 1-hydroxyméthyl-cycloprop-l-yle ;
ou R¹ représente le groupement M^{B} et R^{1B} représente un di(C₁-C₄)alkylamino(C₁-C₃)alkyle, 1-(hydroxyméthyl)-cyclobutan-1-yle, *cis*-3-(hydroxyméthyl)-1-hydroxy-cyclobutan-1-yle, 3-aminooxétan-3-yle, 4-hydroxytétrahydro-2*H*-pyran-4-yle, (*3R,6S*)-3-aminotétrahydro-2*H-*pyran-6-yle, pipéridin-4-yle ou 1-(2-hydroxyacétyl)pipéridin-4-yle ;
ou sel de celui-ci.

12. Composé de formule I selon la revendication 1, où
A représente une liaison ou un C≡C ;
R^{1A} représente un H ou un halogène ;
R^{2A} représente un H ; et
R^{3A} représente un (C₁-C₃)alkoxy, hydroxy(C₁-C₄)alkyle, 1-hydroxyméthyl-cycloprop-1-yle, 3-hydroxyoxétan-3-yle ou 3-aminooxétan-3-yle ;
et où R^{1B} représente un hydroxy(C₁-C₄)alkyle, dihydroxy(C₂-C₄)alkyle, amino(C₁-C₄)alkyle, di(C₁-C₄)alkylamino(C₁-C₃)alkyle, 1-amino-cycloprop-1-yle, 1-hydroxyméthyl-cycloprop-1-yle, *trans*-2-hydroxyméthyl-cycloprop-1-yle, *trans*-2-hydroxyméthyl-1-méthyl-cycloprop-1-yle, *cis*-1-fluoro-2-(hydroxyméthyl)cycloprop-1-yle, *cis*-2-fluoro-2-(hydroxyméthyl)cycloprop-1-yle, 1-(hydroxyméthyl)-cyclobutan-1-yle, *cis*-3-(hydroxyméthyl)-1-hydroxy-cyclobutan-1-yle, 3-hydroxyoxétan-3-yle, 3-hydroxyoxétan-3-yl-(C₁-C₃)alkyle, 3-aminooxétan-3-yle, 3-hydroxyméthyl-oxétan-3-yle, *trans*-(*cis*-3,4-dihydroxy)-cyclopent-1-yle, 4-hydroxytétrahydro-2*H*-pyran-4-yle, (*3R*,*6S*)-3-aminotétrahydro-2*H*-pyran-6-yle, pipéridin-4-yle ou 1-(2-hydroxyacétyl)pipéridin-4-yle ;
ou sel de celui-ci.

13. Composé de formule I selon la revendication 1, qui est sélectionné dans le groupe consistant en les suivants :
(*R*)-*N*-hydroxy-4-(6-((3-hydroxyoxétan-3-yl)buta-1,3-diyn-1-yl)benzo[*d*]thiazol-2-yl)-2-méthyl-2-(méthylsulfonyl)butanamide,
(*R*)-4-(6-(2-fluoro-4-méthoxyphényl)benzo[*d*]thiazol-2-yl)-*N*-hydroxy-2-méthyl-2-(méthylsulfonyl)butanamide,
(*R*)-*N*-hydroxy-4-(6-((4-(3-hydroxyoxétan-3-yl)phényl)éthynyl)benzo[*d*]thiazol-2-yl)-2-méthyl-2-(méthylsulfonyl)butanamide,
(*R*)-*N*-hydroxy-4-(6-(5-hydroxy-5-méthylhexa-1,3-diyn-1-yl)benzo[*d*]thiazol-2-yl)-2-méthyl-2-(méthylsulfonyl)butanamide,
(*R*)-4-(6-((*S*)-5,6-dihydroxyhexa-1,3-diyn-1-yl)benzo[*d*]thiazol-2-yl)-*N*-hydroxy-2-méthyl-2-(méthylsulfonyl)butanamide,
(*R*)-4-(6-(5-amino-5-méthylhexa-1,3-diyn-1-yl)benzo[*d*]thiazol-2-yl)-*N*-hydroxy-2-méthyl-2-(méthylsulfonyl)butanamide,
(*R*)-*N*-hydroxy-4-(6-(((*1S*,*2S*)-2-(hydroxyméthyl)cyclopropyl)buta-1,3-diyn-1-yl)benzo[*d*]thiazol-2-yl)-2-méthyl-2-(méthylsulfonyl)butanamide,
(*R*)-*N*-hydroxy-4-(6-((1-(hydroxyméthyl)cyclopropyl)buta-1,3-diyn-1-yl)benzo[*d*]thiazol-2-yl)-2-méthyl-2-(méthylsulfonyl)butanamide,
(*R*)-4-(6-((3-aminooxétan-3-yl)buta-1,3-diyn-1-yl)benzo[*d*]thiazol-2-yl)-*N*-hydroxy-2-méthyl-2-(méthylsulfonyl)butanamide,
(*R*)-4-(6-((1-aminocyclopropyl)buta-1,3-diyn-1-yl)benzo[*d*]thiazol-2-yl)-*N*-hydroxy-2-méthyl-2-(méthylsulfonyl)butanamide,
(*R*)-*N*-hydroxy-4-(6-((4-(1-(hydroxyméthyl)cyclopropyl)phényl)éthynyl)benzo[*d*]thiazol-2-yl)-2-méthyl-2-(méthylsulfonyl)butanamide,
(*R*)-*N*-hydroxy-4-(6-((1-(hydroxyméthyl)cyclobutyl)buta-1,3-diyn-1-yl)benzo[*d*]thiazol-2-yl)-2-méthyl-2-(méthylsulfonyl)butanamide,
(*R*)-4-(6-(((*1s*,*3R*,*4S*)-3,4-dihydroxycyclopentyl)buta-1,3-diyn-1-yl)benzo[*d*]thiazol-2-yl)-*N-*hydroxy-2-méthyl-2-(méthylsulfonyl)butanamide,
(*R*)-*N*-hydroxy-4-(6-(5-(3-hydroxyoxétan-3-yl)penta-1,3-diyn-1-yl)benzo[*d*]thiazol-2-yl)-2-méthyl-2-(méthylsulfonyl)butanamide,
(*R*)-*N*-hydroxy-4-(6-(((*1R*,*2R*)-2-(hydroxyméthyl)-1-méthylcyclopropyl)buta-1,3-diyn-1-yl)benzo[d]thiazol-2-yl)-2-méthyl-2-(méthylsulfonyl)butanamide,
(*R*)-4-(6-(((*2S*,*5R*)-5-aminotétrahydro-2*H*-pyran-2-yl)buta-1,3-diyn-1-yl)benzo[*d*]thiazol-2-yl)-*N*-hydroxy-2-méthyl-2-(méthylsulfonyl)butanamide,
(*R*)-4-(6-(5-(diméthylamino)penta-1,3-diyn-1-yl)benzo[*d*]thiazol-2-yl)-*N*-hydroxy-2-méthyl-2-(méthylsulfonyl)butanamide,
(*R*)-*N*-hydroxy-4-(6-((4-hydroxytétrahydro-2*H*-pyran-4-yl)b-uta-1,3-diyn-1-yl)benzo[*d*]thiazol-2-yl)-2-méthyl-2-(méthylsulfonyl)butanamide, (*R*)-*N*-hydroxy-2-méthyl-2-(méthylsulfonyl)-4-(6-(pipéridin-4-ylbuta-1,3-diyn-1-yl)benzo[*d*]thiazol-2-yl)butanamide,
(*R*)-*N*-hydroxy-4-(6-((3-(hydroxyméthyl)oxétan-3-yl)buta-1,3-diyn-1-yl)benzo[*d*]thiazol-2-yl)-2-méthyl-2-(méthylsulfonyl)butanamide,
(*R*)-*N*-hydroxy-4-(6-((*cis*-1-hydroxy-3-(hydroxyméthyl)cyclobutyl)buta-1,3-diyn-1-yl)benzo[*d*]thiazol-2-yl)-2-méthyl-2-(méthylsulfonyl)butanamide,
(*R*)-*N*-hydroxy-4-(6-((1-(2-hydroxyacetyl)pipéridin-4-yl)buta-1,3-diyn-1-yl)benzo[*d*]thiazol-2-yl)-2-méthyl-2-(méthylsulfonyl)butanamide,
(*R*)-4-(6-(((*1R**,*2R**)-1-fluoro-2-(hydroxyméthyl)cyclopropyl)buta-1,3-diyn-1-yl)benzo[*d*]thiazol-2-yl)-*N*-hydroxy-2-méthyl-2-(méthylsulfonyl)butanamide et
(*R*)-4-(6-(((*1R**,*2R**)-2-fluoro-2-(hydroxymethyl)cyclopropyl)buta-1,3-diyn-1-yl)benzo[*d*]thiazol-2-yl)-*N*-hydroxy-2-méthyl-2-(méthylsulfonyl)butanamide,
ou sel de celui-ci.

14. Composé de formule I selon la revendication 1, qui est sélectionné dans le groupe consistant en les suivants :
(*R*)-*N*-hydroxy-4-(6-(4-méthoxyphényl)benzo[*d*]thiazol-2-yl)-2-méthyl-2-(méthylsulfonyl)butanamide,
(*R*)-*N*-hydroxy-4-(6-((4-(hydroxyméthyl)phényl)éthynyl)benzo[*d*]thiazol-2-yl)-2-méthyl-2-(méthylsulfonyl)butanamide,
(*R*)-4-(6-((4-(3-aminooxétan-3-yl)phényl)éthynyl)-benzo[*d*]thiazol-2-y1)-*N*-hydroxy-2-méthyl-2-(méthylsulfonyl)butanamide et
(*R*)-*N*-hydroxy-4-(6-(5-hydroxypenta-1,3-diyn-1-yl)benzo[*d*]thiazol-2-yl)-2-méthyl-2-(méthylsulfonyl)butanamide,
ou sel de celui-ci.

15. Composé de formule I selon la revendication 1, qui est sélectionné dans le groupe consistant en les suivants :
(*R*)-*N*-hydroxy-4-(6-((1-(2-hydroxyacetyl)azétidin-3-yl)buta-1,3-diyn-1-yl)benzo[*d*]thiazol-2-yl)-2-méthyl-2-(méthylsulfonyl)butanamide,
(*R*)-*N*-hydroxy-4-(6-(((*1R,2S*)-2-(hydroxyméthyl)-2-méthylcyclopropyl)buta-1,3-diyn-1-yl)benzo[d]thiazol-2-yl)-2-méthyl-2-(méthylsulfonyl)butanamide et
(*R*)-4-(6-(((*1R*,*2R*)-2-fluoro-2-(hydroxyméthyl)cyclopropyl)buta-1,3-diyn-1-yl)benzo[d]thiazol-2-yl)-*N*-hydroxy-2-méthyl-2-(méthylsulfonyl)butanamide,
ou sel de celui-ci.

16. Composé de formule I tel que défini à l'une des revendications 1 à 15 ou sel pharmaceutiquement acceptable de celui-ci pour une utilisation en tant que médicament.

17. Composition pharmaceutique contenant, comme principe actif, un composé de formule I tel que défini à l'une des revendications 1 à 15 ou un sel pharmaceutiquement acceptable de celui-ci et au moins un excipient thérapeutiquement inerte.

18. Composé de formule I tel que défini à l'une des revendications 1 à 15 ou sel pharmaceutiquement acceptable de celui-ci pour une utilisation dans la prévention ou le traitement d'une infection bactérienne.

19. Composé ou sel pharmaceutiquement acceptable selon la revendication 18 pour une utilisation dans la prévention ou le traitement d'une infection à bactérie Gram négatif.
